# EUROPEAN PATENT APPLICATION

(11) **EP 4 039 265 A1**
(43) Date of publication of application: **10.08.2022**
(21) Application number: 20872581.2
(22) Date of filing: 30.09.2020
(51) Int. Cl.: A61K 38/16, A61K 9/14, A61K 9/70, A61K 47/10, A61K 47/20, A61P 17/02, A61P 21/00, C12N 15/12

(54) **MUSCLE TISSUE-REGENERATING AGENT**

(30) Priority: 30.09.2019 JP 2019180709
(71) Applicant: Spiber Inc., Tsuruoka-shi, Yamagata 997-0052 (JP); National University Corporation Tokai National Higher Education and Research System, Nagoya-shi, Aichi 464-8601 (JP)
(72) Inventor: UCHIDA Hiroo, Nagoya-shi, Aichi 464-8601 (JP); NAGATA Hizuru, Nagoya-shi, Aichi 464-8601 (JP); HINOKI Akinari, Nagoya-shi, Aichi 464-8601 (JP); FUJII Masataka, Tsuruoka-shi, Yamagata 997-0052 (JP); CHANG Yun-Hsiang, Tsuruoka-shi, Yamagata 997-0052 (JP); HARADA Satoshi, Tsuruoka-shi, Yamagata 997-0052 (JP); SATO Takehiro, Tsuruoka-shi, Yamagata 997-0052 (JP)
(74) Representative: Handley, Matthew Edward
(86) International application number: PCT/JP2020/037354
(87) International publication number: WO 2021/066078

(57) **Abstract**

To provide a muscle tissue-regenerating agent containing a fibroin protein.

A muscle tissue-regenerating agent containing a modified fibroin protein.

SEQUENCE LISTING

<110>  Spiber Inc.

<120>  Muscle Tissue Regenerating Agent

<130>  19PS064WO1

<160>  44

<170>  PatentIn version 3.5

<210>  1
<211>  50
<212>  PRT
<213>  Araneus diadematus

<400>  1

Ser Gly Cys Asp Val Leu Val Gln Ala Leu Leu Glu Val Val Ser Ala
1               5                   10                  15

Leu Val Ser Ile Leu Gly Ser Ser Ser Ile Gly Gln Ile Asn Tyr Gly
            20                  25                  30

Ala Ser Ala Gln Tyr Thr Gln Met Val Gly Gln Ser Val Ala Gln Ala
        35                  40                  45

Leu Ala
    50

<210>  2
<211>  30
<212>  PRT
<213>  Araneus diadematus

<400>  2

Ser Gly Cys Asp Val Leu Val Gln Ala Leu Leu Glu Val Val Ser Ala
1               5                   10                  15

Leu Val Ser Ile Leu Gly Ser Ser Ser Ile Gly Gln Ile Asn
            20                  25                  30

<210>  3
<211>  21
<212>  PRT
<213>  Araneus diadematus

<400>  3

Ser Gly Cys Asp Val Leu Val Gln Ala Leu Leu Glu Val Val Ser Ala
1               5                   10                  15

Leu Val Ser Ile Leu
            20

<210>  4
<211>  1154
<212>  PRT
<213>  Artificial Sequence

<220>
<223>  recombinant spider silk protein ADF3KaiLargeNRSH1

<400>  4

Met His His His His His His His His His His Ser Ser Gly Ser Ser
1               5                   10                  15

Leu Glu Val Leu Phe Gln Gly Pro Ala Arg Ala Gly Ser Gly Gln Gln
            20                  25                  30

Gly Pro Gly Gln Gln Gly Pro Gly Gln Gln Gly Pro Gly Gln Gln Gly
        35                  40                  45

Pro Tyr Gly Pro Gly Ala Ser Ala Ala Ala Ala Ala Ala Gly Gly Tyr
    50                  55                  60

Gly Pro Gly Ser Gly Gln Gln Gly Pro Ser Gln Gln Gly Pro Gly Gln
65                  70                  75                  80

Gln Gly Pro Gly Gly Gln Gly Pro Tyr Gly Pro Gly Ala Ser Ala Ala
                85                  90                  95

Ala Ala Ala Ala Gly Gly Tyr Gly Pro Gly Ser Gly Gln Gln Gly Pro
            100                 105                 110

Gly Gly Gln Gly Pro Tyr Gly Pro Gly Ser Ser Ala Ala Ala Ala Ala
        115                 120                 125

Ala Gly Gly Asn Gly Pro Gly Ser Gly Gln Gln Gly Ala Gly Gln Gln
    130                 135                 140

Gly Pro Gly Gln Gln Gly Pro Gly Ala Ser Ala Ala Ala Ala Ala Ala
145                 150                 155                 160

Gly Gly Tyr Gly Pro Gly Ser Gly Gln Gln Gly Pro Gly Gln Gln Gly
                165                 170                 175

Pro Gly Gly Gln Gly Pro Tyr Gly Pro Gly Ala Ser Ala Ala Ala Ala
            180                 185                 190

Ala Ala Gly Gly Tyr Gly Pro Gly Ser Gly Gln Gly Pro Gly Gln Gln
        195                 200                 205

Gly Pro Gly Gly Gln Gly Pro Tyr Gly Pro Gly Ala Ser Ala Ala Ala
    210                 215                 220

Ala Ala Ala Gly Gly Tyr Gly Pro Gly Ser Gly Gln Gln Gly Pro Gly
225                 230                 235                 240

Gln Gln Gly Pro Gly Gln Gln Gly Pro Gly Gly Gln Gly Pro Tyr Gly
                245                 250                 255

Pro Gly Ala Ser Ala Ala Ala Ala Ala Ala Gly Gly Tyr Gly Pro Gly
            260                 265                 270

Tyr Gly Gln Gln Gly Pro Gly Gln Gln Gly Pro Gly Gly Gln Gly Pro
        275                 280                 285

Tyr Gly Pro Gly Ala Ser Ala Ala Ser Ala Ala Ser Gly Gly Tyr Gly
    290                 295                 300

Pro Gly Ser Gly Gln Gln Gly Pro Gly Gln Gln Gly Pro Gly Gly Gln
305                 310                 315                 320

Gly Pro Tyr Gly Pro Gly Ala Ser Ala Ala Ala Ala Ala Ala Gly Gly
                325                 330                 335

Tyr Gly Pro Gly Ser Gly Gln Gln Gly Pro Gly Gln Gln Gly Pro Gly
            340                 345                 350

Gln Gln Gly Pro Gly Gln Gln Gly Pro Gly Gly Gln Gly Pro Tyr Gly
        355                 360                 365

Pro Gly Ala Ser Ala Ala Ala Ala Ala Ala Gly Gly Tyr Gly Pro Gly
    370                 375                 380

Ser Gly Gln Gln Gly Pro Gly Gln Gln Gly Pro Gly Gln Gln Gly Pro
385                 390                 395                 400

Gly Gln Gln Gly Pro Gly Gln Gln Gly Pro Gly Gln Gln Gly Pro Gly
                405                 410                 415

Gln Gln Gly Pro Gly Gln Gln Gly Pro Gly Gln Gln Gly Pro Gly Gly
            420                 425                 430

Gln Gly Ala Tyr Gly Pro Gly Ala Ser Ala Ala Ala Gly Ala Ala Gly
        435                 440                 445

Gly Tyr Gly Pro Gly Ser Gly Gln Gln Gly Pro Gly Gln Gln Gly Pro
    450                 455                 460

Gly Gln Gln Gly Pro Gly Gln Gln Gly Pro Gly Gln Gln Gly Pro Gly
465                 470                 475                 480

Gln Gln Gly Pro Gly Gln Gln Gly Pro Gly Gln Gln Gly Pro Tyr Gly
                485                 490                 495

Pro Gly Ala Ser Ala Ala Ala Ala Ala Ala Gly Gly Tyr Gly Pro Gly
            500                 505                 510

Ser Gly Gln Gln Gly Pro Gly Gln Gln Gly Pro Gly Gln Gln Gly Pro
        515                 520                 525

Gly Gly Gln Gly Pro Tyr Gly Pro Gly Ala Ala Ser Ala Ala Val Ser
    530                 535                 540

Val Ser Arg Ala Arg Ala Gly Ser Gly Gln Gln Gly Pro Gly Gln Gln
545                 550                 555                 560

Gly Pro Gly Gln Gln Gly Pro Gly Gln Gln Gly Pro Tyr Gly Pro Gly
                565                 570                 575

Ala Ser Ala Ala Ala Ala Ala Ala Gly Gly Tyr Gly Pro Gly Ser Gly
            580                 585                 590

Gln Gln Gly Pro Ser Gln Gln Gly Pro Gly Gln Gln Gly Pro Gly Gly
        595                 600                 605

Gln Gly Pro Tyr Gly Pro Gly Ala Ser Ala Ala Ala Ala Ala Ala Gly
    610                 615                 620

Gly Tyr Gly Pro Gly Ser Gly Gln Gln Gly Pro Gly Gly Gln Gly Pro
625                 630                 635                 640

Tyr Gly Pro Gly Ser Ser Ala Ala Ala Ala Ala Ala Gly Gly Asn Gly
                645                 650                 655

Pro Gly Ser Gly Gln Gln Gly Ala Gly Gln Gln Gly Pro Gly Gln Gln
            660                 665                 670

Gly Pro Gly Ala Ser Ala Ala Ala Ala Ala Ala Gly Gly Tyr Gly Pro
        675                 680                 685

Gly Ser Gly Gln Gln Gly Pro Gly Gln Gln Gly Pro Gly Gly Gln Gly
    690                 695                 700

Pro Tyr Gly Pro Gly Ala Ser Ala Ala Ala Ala Ala Ala Gly Gly Tyr

705                 710                 715                 720

Gly Pro Gly Ser Gly Gln Gly Pro Gly Gln Gln Gly Pro Gly Gly Gln
                725                 730                 735

Gly Pro Tyr Gly Pro Gly Ala Ser Ala Ala Ala Ala Ala Ala Gly Gly
            740                 745                 750

Tyr Gly Pro Gly Ser Gly Gln Gln Gly Pro Gly Gln Gln Gly Pro Gly
        755                 760                 765

Gln Gln Gly Pro Gly Gly Gln Gly Pro Tyr Gly Pro Gly Ala Ser Ala
    770                 775                 780

Ala Ala Ala Ala Ala Gly Gly Tyr Gly Pro Gly Tyr Gly Gln Gln Gly
785                 790                 795                 800

Pro Gly Gln Gln Gly Pro Gly Gly Gln Gly Pro Tyr Gly Pro Gly Ala
                805                 810                 815

Ser Ala Ala Ser Ala Ala Ser Gly Gly Tyr Gly Pro Gly Ser Gly Gln
            820                 825                 830

Gln Gly Pro Gly Gln Gln Gly Pro Gly Gly Gln Gly Pro Tyr Gly Pro
        835                 840                 845

Gly Ala Ser Ala Ala Ala Ala Ala Ala Gly Gly Tyr Gly Pro Gly Ser
    850                 855                 860

Gly Gln Gln Gly Pro Gly Gln Gln Gly Pro Gly Gln Gln Gly Pro Gly
865                 870                 875                 880

Gln Gln Gly Pro Gly Gly Gln Gly Pro Tyr Gly Pro Gly Ala Ser Ala
                885                 890                 895

Ala Ala Ala Ala Ala Gly Gly Tyr Gly Pro Gly Ser Gly Gln Gln Gly
            900                 905                 910

Pro Gly Gln Gln Gly Pro Gly Gln Gln Gly Pro Gly Gln Gln Gly Pro
        915                 920                 925

Gly Gln Gln Gly Pro Gly Gln Gln Gly Pro Gly Gln Gln Gly Pro Gly
    930                 935                 940

Gln Gln Gly Pro Gly Gln Gln Gly Pro Gly Gly Gln Gly Ala Tyr Gly
945                 950                 955                 960

Pro Gly Ala Ser Ala Ala Ala Gly Ala Ala Gly Gly Tyr Gly Pro Gly
                965                 970                 975

Ser Gly Gln Gln Gly Pro Gly Gln Gln Gly Pro Gly Gln Gln Gly Pro
            980                 985                 990

Gly Gln Gln Gly Pro Gly Gln Gln  Gly Pro Gly Gln Gln  Gly Pro Gly
        995                 1000                 1005

Gln Gln  Gly Pro Gly Gln Gln  Gly Pro Tyr Gly Pro  Gly Ala Ser
    1010                 1015                 1020

Ala Ala  Ala Ala Ala Ala Gly  Gly Tyr Gly Pro Gly  Ser Gly Gln
    1025                 1030                 1035

Gln Gly  Pro Gly Gln Gln Gly  Pro Gly Gln Gln Gly  Pro Gly Gly
    1040                 1045                 1050

Gln Gly  Pro Tyr Gly Pro Gly  Ala Ala Ser Ala Ala  Val Ser Val
    1055                 1060                 1065

Gly Gly  Tyr Gly Pro Gln Ser  Ser Ser Val Pro Val  Ala Ser Ala
    1070                 1075                 1080

Val Ala  Ser Arg Leu Ser Ser  Pro Ala Ala Ser Ser  Arg Val Ser
    1085                 1090                 1095

Ser Ala  Val Ser Ser Leu Val  Ser Ser Gly Pro Thr  Lys His Ala
    1100                 1105                 1110

Ala Leu  Ser Asn Thr Ile Ser  Ser Val Val Ser Gln  Val Ser Ala
    1115                 1120                 1125

Ser Asn  Pro Gly Leu Ser Gly  Cys Asp Val Leu Val  Gln Ala Leu
    1130                 1135                 1140

Leu Glu  Val Val Ser Ala Leu  Val Ser Ile Leu
    1145                 1150

<210>  5
<211>  24
<212>  PRT
<213>  Artificial Sequence

<220>
<223>  His tag and start codon

<400>  5

Met His His His His His His His His His His Ser Ser Gly Ser Ser

1               5                   10                  15

Leu Glu Val Leu Phe Gln Gly Pro
            20

<210>  6
<211>  597
<212>  PRT
<213>  Artificial Sequence

<220>
<223>  Met-PRT380

<400>  6

Met Gly Pro Gly Gln Gln Gly Pro Tyr Gly Pro Gly Ala Ser Ala Ala
1               5                   10                  15

Ala Ala Ala Gly Gln Asn Gly Pro Gly Ser Gly Gln Gln Gly Pro Gly
            20                  25                  30

Gln Ser Ala Ala Ala Ala Ala Gly Gln Tyr Gly Pro Gly Gln Gln Gly
        35                  40                  45

Pro Gly Gln Gln Gly Pro Gly Ser Ser Ala Ala Ala Ala Ala Gly Pro
    50                  55                  60

Gly Gln Tyr Gly Pro Gly Gln Gln Gly Pro Ser Ala Ser Ala Ala Ala
65                  70                  75                  80

Ala Ala Gly Pro Gly Ser Gly Gln Gln Gly Pro Gly Ala Ser Ala Ala
                85                  90                  95

Ala Ala Ala Gly Gln Tyr Gly Pro Gly Gln Gln Gly Pro Gly Gln Gln
            100                 105                 110

Gly Pro Gly Ser Ser Ala Ala Ala Ala Ala Gly Gln Tyr Gly Ser Gly
        115                 120                 125

Pro Gly Gln Gln Gly Pro Tyr Gly Ser Ala Ala Ala Ala Ala Gly Pro
    130                 135                 140

Gly Ser Gly Gln Tyr Gly Gln Gly Pro Tyr Gly Pro Gly Ala Ser Ala
145                 150                 155                 160

Ala Ala Ala Ala Gly Pro Gly Gln Tyr Gly Pro Gly Gln Gln Gly Pro
                165                 170                 175

Ser Ala Ser Ala Ala Ala Ala Ala Gly Ser Gly Gln Gln Gly Pro Gly
            180                 185                 190

Gln Tyr Gly Pro Tyr Ala Ser Ala Ala Ala Ala Ala Gly Gln Tyr Gly
        195                 200                 205

Ser Gly Pro Gly Gln Gln Gly Pro Tyr Gly Pro Gly Gln Ser Ala Ala
    210                 215                 220

Ala Ala Ala Gly Ser Gly Gln Gln Gly Pro Gly Gln Gln Gly Pro Tyr
225                 230                 235                 240

Ala Ser Ala Ala Ala Ala Ala Gly Pro Gly Gln Gln Gly Pro Tyr Gly
                245                 250                 255

Pro Gly Ser Ser Ala Ala Ala Ala Ala Gly Gln Tyr Gly Tyr Gly Pro
            260                 265                 270

Gly Gln Gln Gly Pro Tyr Gly Pro Gly Ala Ser Ala Ala Ala Ala Ala
        275                 280                 285

Gly Gln Asn Gly Pro Gly Ser Gly Gln Tyr Gly Pro Gly Gln Gln Gly
    290                 295                 300

Pro Gly Gln Ser Ala Ala Ala Ala Ala Gly Pro Gly Gln Gln Gly Pro
305                 310                 315                 320

Tyr Gly Pro Gly Ala Ser Ala Ala Ala Ala Ala Gly Gln Tyr Gly Pro
                325                 330                 335

Gly Gln Gln Gly Pro Gly Gln Tyr Gly Pro Gly Ser Ser Ala Ala Ala
            340                 345                 350

Ala Ala Gly Pro Gly Gln Gln Gly Pro Tyr Gly Pro Gly Ser Ser Ala
        355                 360                 365

Ala Ala Ala Ala Gly Gln Tyr Gly Pro Gly Gln Gln Gly Pro Tyr Gly
    370                 375                 380

Pro Gly Gln Ser Ala Ala Ala Ala Ala Gly Gln Tyr Gln Gln Gly Pro
385                 390                 395                 400

Gly Gln Gln Gly Pro Tyr Gly Pro Gly Ala Ser Ala Ala Ala Ala Ala
                405                 410                 415

Gly Pro Gly Gln Gln Gly Pro Tyr Gly Pro Gly Ala Ser Ala Ala Ala
            420                 425                 430

Ala Ala Gly Pro Gly Gln Tyr Gly Pro Gly Gln Gln Gly Pro Ser Ala

435                 440                 445

Ser Ala Ala Ala Ala Ala Gly Gln Tyr Gly Ser Gly Pro Gly Gln Tyr
    450                 455                 460

Gly Pro Tyr Gly Pro Gly Gln Ser Ala Ala Ala Ala Ala Gly Pro Gly
465                 470                 475                 480

Ser Gly Gln Gln Gly Gln Gly Pro Tyr Gly Pro Gly Ala Ser Ala Ala
                485                 490                 495

Ala Ala Ala Gly Gln Tyr Gly Pro Gly Gln Gln Gly Pro Tyr Gly Pro
            500                 505                 510

Gly Gln Ser Ala Ala Ala Ala Ala Gly Pro Gly Ser Gly Gln Tyr Gly
        515                 520                 525

Pro Gly Ala Ser Ala Ala Ala Ala Ala Gly Gln Asn Gly Pro Gly Ser
    530                 535                 540

Gly Gln Tyr Gly Pro Gly Gln Gln Gly Pro Gly Gln Ser Ala Ala Ala
545                 550                 555                 560

Ala Ala Gly Gln Tyr Gln Gln Gly Pro Gly Gln Gln Gly Pro Tyr Gly
                565                 570                 575

Pro Gly Ala Ser Ala Ala Ala Ala Ala Gly Pro Gly Ser Gly Gln Gln
            580                 585                 590

Gly Pro Gly Ala Ser
        595

<210>  7
<211>  590
<212>  PRT
<213>  Artificial Sequence

<220>
<223>  Met-PRT410

<400>  7

Met Gly Pro Gly Gln Gln Gly Pro Tyr Gly Pro Gly Ala Ser Ala Ala
1               5                   10                  15

Ala Ala Ala Gly Gln Asn Gly Pro Gly Ser Gly Gln Gln Gly Pro Gly
            20                  25                  30

Gln Ser Gly Gln Tyr Gly Pro Gly Gln Gln Gly Pro Gly Gln Gln Gly
        35                  40                  45

Pro Gly Ser Ser Ala Ala Ala Ala Ala Gly Pro Gly Gln Tyr Gly Pro
    50                  55                  60

Gly Gln Gln Gly Pro Ser Ala Ser Ala Ala Ala Ala Ala Gly Pro Gly
65                  70                  75                  80

Ser Gly Gln Gln Gly Pro Gly Ala Ser Gly Gln Tyr Gly Pro Gly Gln
                85                  90                  95

Gln Gly Pro Gly Gln Gln Gly Pro Gly Ser Ser Ala Ala Ala Ala Ala
            100                 105                 110

Gly Gln Tyr Gly Ser Gly Pro Gly Gln Gln Gly Pro Tyr Gly Ser Ala
        115                 120                 125

Ala Ala Ala Ala Gly Pro Gly Ser Gly Gln Tyr Gly Gln Gly Pro Tyr
    130                 135                 140

Gly Pro Gly Ala Ser Gly Pro Gly Gln Tyr Gly Pro Gly Gln Gln Gly
145                 150                 155                 160

Pro Ser Ala Ser Ala Ala Ala Ala Ala Gly Ser Gly Gln Gln Gly Pro
                165                 170                 175

Gly Gln Tyr Gly Pro Tyr Ala Ser Ala Ala Ala Ala Ala Gly Gln Tyr
            180                 185                 190

Gly Ser Gly Pro Gly Gln Gln Gly Pro Tyr Gly Pro Gly Gln Ser Gly
        195                 200                 205

Ser Gly Gln Gln Gly Pro Gly Gln Gln Gly Pro Tyr Ala Ser Ala Ala
    210                 215                 220

Ala Ala Ala Gly Pro Gly Gln Gln Gly Pro Tyr Gly Pro Gly Ser Ser
225                 230                 235                 240

Ala Ala Ala Ala Ala Gly Gln Tyr Gly Tyr Gly Pro Gly Gln Gln Gly
                245                 250                 255

Pro Tyr Gly Pro Gly Ala Ser Gly Gln Asn Gly Pro Gly Ser Gly Gln
            260                 265                 270

Tyr Gly Pro Gly Gln Gln Gly Pro Gly Gln Ser Ala Ala Ala Ala Ala
        275                 280                 285

Gly Pro Gly Gln Gln Gly Pro Tyr Gly Pro Gly Ala Ser Ala Ala Ala

290                 295                 300

Ala Ala Gly Gln Tyr Gly Pro Gly Gln Gln Gly Pro Gly Gln Tyr Gly
305                 310                 315                 320

Pro Gly Ser Ser Gly Pro Gly Gln Gln Gly Pro Tyr Gly Pro Gly Ser
                325                 330                 335

Ser Ala Ala Ala Ala Ala Gly Gln Tyr Gly Pro Gly Gln Gln Gly Pro
            340                 345                 350

Tyr Gly Pro Gly Gln Ser Ala Ala Ala Ala Ala Gly Gln Tyr Gln Gln
        355                 360                 365

Gly Pro Gly Gln Gln Gly Pro Tyr Gly Pro Gly Ala Ser Gly Pro Gly
    370                 375                 380

Gln Gln Gly Pro Tyr Gly Pro Gly Ala Ser Ala Ala Ala Ala Ala Gly
385                 390                 395                 400

Pro Gly Gln Tyr Gly Pro Gly Gln Gln Gly Pro Ser Ala Ser Ala Ala
                405                 410                 415

Ala Ala Ala Gly Gln Tyr Gly Ser Gly Pro Gly Gln Tyr Gly Pro Tyr
            420                 425                 430

Gly Pro Gly Gln Ser Gly Pro Gly Ser Gly Gln Gln Gly Gln Gly Pro
        435                 440                 445

Tyr Gly Pro Gly Ala Ser Ala Ala Ala Ala Ala Gly Gln Tyr Gly Pro
    450                 455                 460

Gly Gln Gln Gly Pro Tyr Gly Pro Gly Gln Ser Ala Ala Ala Ala Ala
465                 470                 475                 480

Gly Pro Gly Ser Gly Gln Tyr Gly Pro Gly Ala Ser Gly Gln Asn Gly
                485                 490                 495

Pro Gly Ser Gly Gln Tyr Gly Pro Gly Gln Gln Gly Pro Gly Gln Ser
            500                 505                 510

Ala Ala Ala Ala Ala Gly Gln Tyr Gln Gln Gly Pro Gly Gln Gln Gly
        515                 520                 525

Pro Tyr Gly Pro Gly Ala Ser Ala Ala Ala Ala Ala Gly Gln Tyr Gly
    530                 535                 540

Ser Gly Pro Gly Gln Gln Gly Pro Tyr Gly Pro Gly Gln Ser Gly Ser
545                 550                 555                 560

Gly Gln Gln Gly Pro Gly Gln Gln Gly Pro Tyr Ala Ser Ala Ala Ala
                565                 570                 575

Ala Ala Gly Pro Gly Ser Gly Gln Gln Gly Pro Gly Ala Ser
            580                 585                 590

<210>  8
<211>  565
<212>  PRT
<213>  Artificial Sequence

<220>
<223>  Met-PRT468

<400>  8

Met Gly Pro Gly Gln Gln Gly Pro Tyr Gly Pro Gly Ala Ser Ala Ala
1               5                   10                  15

Ala Ala Ala Ala Ala Gly Ser Asn Gly Pro Gly Ser Gly Gln Gln Gly
            20                  25                  30

Pro Gly Gln Ser Gly Gln Tyr Gly Pro Gly Gln Gln Gly Pro Gly Gln
        35                  40                  45

Gln Gly Pro Gly Ser Ser Ala Ala Ala Ala Ala Ala Ala Gly Pro Gly
    50                  55                  60

Gln Tyr Gly Pro Gly Gln Gln Gly Pro Ser Ala Ser Ala Ala Ala Ala
65                  70                  75                  80

Ala Ala Ala Gly Pro Gly Ser Gly Gln Gln Gly Pro Gly Ala Ser Gly
                85                  90                  95

Gln Tyr Gly Pro Gly Gln Gln Gly Pro Gly Gln Gln Gly Pro Gly Ser
            100                 105                 110

Ser Ala Ala Ala Ala Ala Ala Ala Gly Ser Tyr Gly Ser Gly Pro Gly
        115                 120                 125

Gln Gln Gly Pro Tyr Gly Ser Ala Ala Ala Ala Ala Ala Ala Gly Pro
    130                 135                 140

Gly Ser Gly Gln Tyr Gly Gln Gly Pro Tyr Gly Pro Gly Ala Ser Gly
145                 150                 155                 160

Pro Gly Gln Tyr Gly Pro Gly Gln Gln Gly Pro Ser Ala Ser Ala Ala

                165                 170                 175

Ala Ala Ala Ala Ala Gly Ser Gly Gln Gln Gly Pro Gly Gln Tyr Gly
            180                 185                 190

Pro Tyr Ala Ser Ala Ala Ala Ala Ala Ala Ala Gly Ser Tyr Gly Ser
        195                 200                 205

Gly Pro Gly Gln Gln Gly Pro Tyr Gly Pro Gly Gln Ser Gly Ser Gly
    210                 215                 220

Gln Gln Gly Pro Gly Gln Gln Gly Pro Tyr Ala Ser Ala Ala Ala Ala
225                 230                 235                 240

Ala Ala Ala Gly Pro Gly Gln Gln Gly Pro Tyr Gly Pro Gly Ser Ser
                245                 250                 255

Ala Ala Ala Ala Ala Ala Ala Gly Ser Tyr Gly Tyr Gly Pro Gly Gln
            260                 265                 270

Gln Gly Pro Tyr Gly Pro Gly Ala Ser Gly Gln Asn Gly Pro Gly Ser
        275                 280                 285

Gly Gln Tyr Gly Pro Gly Gln Gln Gly Pro Gly Pro Ser Ala Ala Ala
    290                 295                 300

Ala Ala Ala Ala Gly Pro Gly Gln Gln Gly Pro Tyr Gly Pro Gly Ala
305                 310                 315                 320

Ser Ala Ala Ala Ala Ala Ala Ala Gly Ser Tyr Gly Pro Gly Gln Gln
                325                 330                 335

Gly Pro Gly Gln Tyr Gly Pro Gly Ser Ser Gly Pro Gly Gln Gln Gly
            340                 345                 350

Pro Tyr Gly Pro Gly Ser Ser Ala Ala Ala Ala Ala Ala Ala Gly Ser
        355                 360                 365

Tyr Gly Pro Gly Gln Gln Gly Pro Tyr Gly Pro Gly Pro Ser Ala Ala
    370                 375                 380

Ala Ala Ala Ala Ala Gly Ser Tyr Gln Gln Gly Pro Gly Gln Gln Gly
385                 390                 395                 400

Pro Tyr Gly Pro Gly Ala Ser Gly Pro Gly Gln Gln Gly Pro Tyr Gly
                405                 410                 415

Pro Gly Ala Ser Ala Ala Ala Ala Ala Ala Ala Gly Pro Gly Gln Tyr
                420                 425                 430

Gly Pro Gly Gln Gln Gly Pro Ser Ala Ser Ala Ala Ala Ala Ala Ala
        435                 440                 445

Ala Gly Ser Tyr Gly Ser Gly Pro Gly Gln Tyr Gly Pro Tyr Gly Pro
    450                 455                 460

Gly Gln Ser Gly Pro Gly Ser Gly Gln Gln Gly Gln Gly Pro Tyr Gly
465                 470                 475                 480

Pro Gly Ala Ser Ala Ala Ala Ala Ala Ala Ala Gly Ser Tyr Gly Pro
                485                 490                 495

Gly Gln Gln Gly Pro Tyr Gly Pro Gly Pro Ser Ala Ala Ala Ala Ala
            500                 505                 510

Ala Ala Gly Pro Gly Ser Gly Gln Tyr Gly Pro Gly Ala Ser Gly Gln
        515                 520                 525

Asn Gly Pro Gly Ser Gly Gln Tyr Gly Pro Gly Gln Gln Gly Pro Gly
    530                 535                 540

Pro Ser Ala Ala Ala Ala Ala Ala Ala Gly Pro Gly Ser Gly Gln Gln
545                 550                 555                 560

Gly Pro Gly Ala Ser
                565

<210>  9
<211>  2364
<212>  PRT
<213>  Artificial Sequence

<220>
<223>  Met-PRT799

<400>  9

Met Gly Pro Gly Gln Gln Gly Pro Tyr Gly Pro Gly Ala Ser Ala Ala
1               5                   10                  15

Ala Ala Ala Gly Gln Asn Gly Pro Gly Ser Gly Gln Gln Gly Pro Gly
            20                  25                  30

Gln Ser Gly Gln Tyr Gly Pro Gly Gln Gln Gly Pro Gly Gln Gln Gly
        35                  40                  45

Pro Gly Ser Ser Ala Ala Ala Ala Ala Gly Pro Gly Gln Tyr Gly Pro

50                  55                  60

Gly Gln Gln Gly Pro Ser Ala Ser Ala Ala Ala Ala Ala Gly Pro Gly
65                  70                  75                  80

Ser Gly Gln Gln Gly Pro Gly Ala Ser Gly Gln Tyr Gly Pro Gly Gln
                85                  90                  95

Gln Gly Pro Gly Gln Gln Gly Pro Gly Ser Ser Ala Ala Ala Ala Ala
            100                 105                 110

Gly Gln Tyr Gly Ser Gly Pro Gly Gln Gln Gly Pro Tyr Gly Ser Ala
        115                 120                 125

Ala Ala Ala Ala Gly Pro Gly Ser Gly Gln Tyr Gly Gln Gly Pro Tyr
    130                 135                 140

Gly Pro Gly Ala Ser Gly Pro Gly Gln Tyr Gly Pro Gly Gln Gln Gly
145                 150                 155                 160

Pro Ser Ala Ser Ala Ala Ala Ala Ala Gly Ser Gly Gln Gln Gly Pro
                165                 170                 175

Gly Gln Tyr Gly Pro Tyr Ala Ser Ala Ala Ala Ala Ala Gly Gln Tyr
            180                 185                 190

Gly Ser Gly Pro Gly Gln Gln Gly Pro Tyr Gly Pro Gly Gln Ser Gly
        195                 200                 205

Ser Gly Gln Gln Gly Pro Gly Gln Gln Gly Pro Tyr Ala Ser Ala Ala
    210                 215                 220

Ala Ala Ala Gly Pro Gly Gln Gln Gly Pro Tyr Gly Pro Gly Ser Ser
225                 230                 235                 240

Ala Ala Ala Ala Ala Gly Gln Tyr Gly Tyr Gly Pro Gly Gln Gln Gly
                245                 250                 255

Pro Tyr Gly Pro Gly Ala Ser Gly Gln Asn Gly Pro Gly Ser Gly Gln
            260                 265                 270

Tyr Gly Pro Gly Gln Gln Gly Pro Gly Gln Ser Ala Ala Ala Ala Ala
        275                 280                 285

Gly Pro Gly Gln Gln Gly Pro Tyr Gly Pro Gly Ala Ser Ala Ala Ala
    290                 295                 300

Ala Ala Gly Gln Tyr Gly Pro Gly Gln Gln Gly Pro Gly Gln Tyr Gly
305                 310                 315                 320

Pro Gly Ser Ser Gly Pro Gly Gln Gln Gly Pro Tyr Gly Pro Gly Ser
                325                 330                 335

Ser Ala Ala Ala Ala Ala Gly Gln Tyr Gly Pro Gly Gln Gln Gly Pro
            340                 345                 350

Tyr Gly Pro Gly Gln Ser Ala Ala Ala Ala Ala Gly Gln Tyr Gln Gln
        355                 360                 365

Gly Pro Gly Gln Gln Gly Pro Tyr Gly Pro Gly Ala Ser Gly Pro Gly
    370                 375                 380

Gln Gln Gly Pro Tyr Gly Pro Gly Ala Ser Ala Ala Ala Ala Ala Gly
385                 390                 395                 400

Pro Gly Gln Tyr Gly Pro Gly Gln Gln Gly Pro Ser Ala Ser Ala Ala
                405                 410                 415

Ala Ala Ala Gly Gln Tyr Gly Ser Gly Pro Gly Gln Tyr Gly Pro Tyr
            420                 425                 430

Gly Pro Gly Gln Ser Gly Pro Gly Ser Gly Gln Gln Gly Gln Gly Pro
        435                 440                 445

Tyr Gly Pro Gly Ala Ser Ala Ala Ala Ala Ala Gly Gln Tyr Gly Pro
    450                 455                 460

Gly Gln Gln Gly Pro Tyr Gly Pro Gly Gln Ser Ala Ala Ala Ala Ala
465                 470                 475                 480

Gly Pro Gly Ser Gly Gln Tyr Gly Pro Gly Ala Ser Gly Gln Asn Gly
                485                 490                 495

Pro Gly Ser Gly Gln Tyr Gly Pro Gly Gln Gln Gly Pro Gly Gln Ser
            500                 505                 510

Ala Ala Ala Ala Ala Gly Gln Tyr Gln Gln Gly Pro Gly Gln Gln Gly
        515                 520                 525

Pro Tyr Gly Pro Gly Ala Ser Ala Ala Ala Ala Ala Gly Gln Tyr Gly
    530                 535                 540

Ser Gly Pro Gly Gln Gln Gly Pro Tyr Gly Pro Gly Gln Ser Gly Ser
545                 550                 555                 560

Gly Gln Gln Gly Pro Gly Gln Gln Gly Pro Tyr Ala Ser Ala Ala Ala
                565                 570                 575

Ala Ala Gly Pro Gly Ser Gly Gln Gln Gly Pro Gly Ala Ser Gly Gln
            580                 585                 590

Gln Gly Pro Tyr Gly Pro Gly Ala Ser Ala Ala Ala Ala Ala Gly Gln
        595                 600                 605

Asn Gly Pro Gly Ser Gly Gln Gln Gly Pro Gly Gln Ser Gly Gln Tyr
    610                 615                 620

Gly Pro Gly Gln Gln Gly Pro Gly Gln Gln Gly Pro Gly Ser Ser Ala
625                 630                 635                 640

Ala Ala Ala Ala Gly Pro Gly Gln Tyr Gly Pro Gly Gln Gln Gly Pro
                645                 650                 655

Ser Ala Ser Ala Ala Ala Ala Ala Gly Pro Gly Ser Gly Gln Gln Gly
            660                 665                 670

Pro Gly Ala Ser Gly Gln Tyr Gly Pro Gly Gln Gln Gly Pro Gly Gln
        675                 680                 685

Gln Gly Pro Gly Ser Ser Ala Ala Ala Ala Ala Gly Gln Tyr Gly Ser
    690                 695                 700

Gly Pro Gly Gln Gln Gly Pro Tyr Gly Ser Ala Ala Ala Ala Ala Gly
705                 710                 715                 720

Pro Gly Ser Gly Gln Tyr Gly Gln Gly Pro Tyr Gly Pro Gly Ala Ser
                725                 730                 735

Gly Pro Gly Gln Tyr Gly Pro Gly Gln Gln Gly Pro Ser Ala Ser Ala
            740                 745                 750

Ala Ala Ala Ala Gly Ser Gly Gln Gln Gly Pro Gly Gln Tyr Gly Pro
        755                 760                 765

Tyr Ala Ser Ala Ala Ala Ala Ala Gly Gln Tyr Gly Ser Gly Pro Gly
    770                 775                 780

Gln Gln Gly Pro Tyr Gly Pro Gly Gln Ser Gly Ser Gly Gln Gln Gly
785                 790                 795                 800

Pro Gly Gln Gln Gly Pro Tyr Ala Ser Ala Ala Ala Ala Ala Gly Pro
                805                 810                 815

Gly Gln Gln Gly Pro Tyr Gly Pro Gly Ser Ser Ala Ala Ala Ala Ala
            820                 825                 830

Gly Gln Tyr Gly Tyr Gly Pro Gly Gln Gln Gly Pro Tyr Gly Pro Gly
        835                 840                 845

Ala Ser Gly Gln Asn Gly Pro Gly Ser Gly Gln Tyr Gly Pro Gly Gln
    850                 855                 860

Gln Gly Pro Gly Gln Ser Ala Ala Ala Ala Ala Gly Pro Gly Gln Gln
865                 870                 875                 880

Gly Pro Tyr Gly Pro Gly Ala Ser Ala Ala Ala Ala Ala Gly Gln Tyr
                885                 890                 895

Gly Pro Gly Gln Gln Gly Pro Gly Gln Tyr Gly Pro Gly Ser Ser Gly
            900                 905                 910

Pro Gly Gln Gln Gly Pro Tyr Gly Pro Gly Ser Ser Ala Ala Ala Ala
        915                 920                 925

Ala Gly Gln Tyr Gly Pro Gly Gln Gln Gly Pro Tyr Gly Pro Gly Gln
    930                 935                 940

Ser Ala Ala Ala Ala Ala Gly Gln Tyr Gln Gln Gly Pro Gly Gln Gln
945                 950                 955                 960

Gly Pro Tyr Gly Pro Gly Ala Ser Gly Pro Gly Gln Gln Gly Pro Tyr
                965                 970                 975

Gly Pro Gly Ala Ser Ala Ala Ala Ala Ala Gly Pro Gly Gln Tyr Gly
            980                 985                 990

Pro Gly Gln Gln Gly Pro Ser Ala  Ser Ala Ala Ala Ala  Ala Gly Gln
        995                 1000                1005

Tyr Gly  Ser Gly Pro Gly Gln  Tyr Gly Pro Tyr Gly  Pro Gly Gln
    1010                1015                1020

Ser Gly  Pro Gly Ser Gly Gln  Gln Gly Gln Gly Pro  Tyr Gly Pro
    1025                1030                1035

Gly Ala  Ser Ala Ala Ala Ala  Ala Gly Gln Tyr Gly  Pro Gly Gln
    1040                1045                1050

Gln Gly  Pro Tyr Gly Pro Gly  Gln Ser Ala Ala Ala  Ala Ala Gly

1055                1060                1065

Pro Gly  Ser Gly Gln Tyr Gly  Pro Gly Ala Ser Gly  Gln Asn Gly
    1070                1075                1080

Pro Gly  Ser Gly Gln Tyr Gly  Pro Gly Gln Gln Gly  Pro Gly Gln
    1085                1090                1095

Ser Ala  Ala Ala Ala Ala Gly  Gln Tyr Gln Gln Gly  Pro Gly Gln
    1100                1105                1110

Gln Gly  Pro Tyr Gly Pro Gly  Ala Ser Ala Ala Ala  Ala Ala Gly
    1115                1120                1125

Gln Tyr  Gly Ser Gly Pro Gly  Gln Gln Gly Pro Tyr  Gly Pro Gly
    1130                1135                1140

Gln Ser  Gly Ser Gly Gln Gln  Gly Pro Gly Gln Gln  Gly Pro Tyr
    1145                1150                1155

Ala Ser  Ala Ala Ala Ala Ala  Gly Pro Gly Ser Gly  Gln Gln Gly
    1160                1165                1170

Pro Gly  Ala Ser Gly Gln Gln  Gly Pro Tyr Gly Pro  Gly Ala Ser
    1175                1180                1185

Ala Ala  Ala Ala Ala Gly Gln  Asn Gly Pro Gly Ser  Gly Gln Gln
    1190                1195                1200

Gly Pro  Gly Gln Ser Gly Gln  Tyr Gly Pro Gly Gln  Gln Gly Pro
    1205                1210                1215

Gly Gln  Gln Gly Pro Gly Ser  Ser Ala Ala Ala Ala  Ala Gly Pro
    1220                1225                1230

Gly Gln  Tyr Gly Pro Gly Gln  Gln Gly Pro Ser Ala  Ser Ala Ala
    1235                1240                1245

Ala Ala  Ala Gly Pro Gly Ser  Gly Gln Gln Gly Pro  Gly Ala Ser
    1250                1255                1260

Gly Gln  Tyr Gly Pro Gly Gln  Gln Gly Pro Gly Gln  Gln Gly Pro
    1265                1270                1275

Gly Ser  Ser Ala Ala Ala Ala  Ala Gly Gln Tyr Gly  Ser Gly Pro
    1280                1285                1290

Gly Gln Gln Gly Pro Tyr Gly Ser Ala Ala Ala Ala Ala Gly Pro
    1295                1300                1305

Gly Ser Gly Gln Tyr Gly Gln Gly Pro Tyr Gly Pro Gly Ala Ser
    1310                1315                1320

Gly Pro Gly Gln Tyr Gly Pro Gly Gln Gln Gly Pro Ser Ala Ser
    1325                1330                1335

Ala Ala Ala Ala Ala Gly Ser Gly Gln Gln Gly Pro Gly Gln Tyr
    1340                1345                1350

Gly Pro Tyr Ala Ser Ala Ala Ala Ala Ala Gly Gln Tyr Gly Ser
    1355                1360                1365

Gly Pro Gly Gln Gln Gly Pro Tyr Gly Pro Gly Gln Ser Gly Ser
    1370                1375                1380

Gly Gln Gln Gly Pro Gly Gln Gln Gly Pro Tyr Ala Ser Ala Ala
    1385                1390                1395

Ala Ala Ala Gly Pro Gly Gln Gln Gly Pro Tyr Gly Pro Gly Ser
    1400                1405                1410

Ser Ala Ala Ala Ala Ala Gly Gln Tyr Gly Tyr Gly Pro Gly Gln
    1415                1420                1425

Gln Gly Pro Tyr Gly Pro Gly Ala Ser Gly Gln Asn Gly Pro Gly
    1430                1435                1440

Ser Gly Gln Tyr Gly Pro Gly Gln Gln Gly Pro Gly Gln Ser Ala
    1445                1450                1455

Ala Ala Ala Ala Gly Pro Gly Gln Gln Gly Pro Tyr Gly Pro Gly
    1460                1465                1470

Ala Ser Ala Ala Ala Ala Ala Gly Gln Tyr Gly Pro Gly Gln Gln
    1475                1480                1485

Gly Pro Gly Gln Tyr Gly Pro Gly Ser Ser Gly Pro Gly Gln Gln
    1490                1495                1500

Gly Pro Tyr Gly Pro Gly Ser Ser Ala Ala Ala Ala Ala Gly Gln
    1505                1510                1515

Tyr Gly Pro Gly Gln Gln Gly Pro Tyr Gly Pro Gly Gln Ser Ala
    1520                1525                1530

Ala Ala Ala Ala Gly Gln Tyr Gln Gln Gly Pro Gly Gln Gln Gly
    1535                1540                1545

Pro Tyr Gly Pro Gly Ala Ser Gly Pro Gly Gln Gln Gly Pro Tyr
    1550                1555                1560

Gly Pro Gly Ala Ser Ala Ala Ala Ala Ala Gly Pro Gly Gln Tyr
    1565                1570                1575

Gly Pro Gly Gln Gln Gly Pro Ser Ala Ser Ala Ala Ala Ala Ala
    1580                1585                1590

Gly Gln Tyr Gly Ser Gly Pro Gly Gln Tyr Gly Pro Tyr Gly Pro
    1595                1600                1605

Gly Gln Ser Gly Pro Gly Ser Gly Gln Gln Gly Gln Gly Pro Tyr
    1610                1615                1620

Gly Pro Gly Ala Ser Ala Ala Ala Ala Ala Gly Gln Tyr Gly Pro
    1625                1630                1635

Gly Gln Gln Gly Pro Tyr Gly Pro Gly Gln Ser Ala Ala Ala Ala
    1640                1645                1650

Ala Gly Pro Gly Ser Gly Gln Tyr Gly Pro Gly Ala Ser Gly Gln
    1655                1660                1665

Asn Gly Pro Gly Ser Gly Gln Tyr Gly Pro Gly Gln Gln Gly Pro
    1670                1675                1680

Gly Gln Ser Ala Ala Ala Ala Ala Gly Gln Tyr Gln Gln Gly Pro
    1685                1690                1695

Gly Gln Gln Gly Pro Tyr Gly Pro Gly Ala Ser Ala Ala Ala Ala
    1700                1705                1710

Ala Gly Gln Tyr Gly Ser Gly Pro Gly Gln Gln Gly Pro Tyr Gly
    1715                1720                1725

Pro Gly Gln Ser Gly Ser Gly Gln Gln Gly Pro Gly Gln Gln Gly
    1730                1735                1740

Pro Tyr Ala Ser Ala Ala Ala Ala Ala Gly Pro Gly Ser Gly Gln
    1745                1750                1755

Gln Gly Pro Gly Ala Ser Gly Gln Gln Gly Pro Tyr Gly Pro Gly
    1760                1765                1770

Ala Ser Ala Ala Ala Ala Ala Gly Gln Asn Gly Pro Gly Ser Gly
    1775                1780                1785

Gln Gln Gly Pro Gly Gln Ser Gly Gln Tyr Gly Pro Gly Gln Gln
    1790                1795                1800

Gly Pro Gly Gln Gln Gly Pro Gly Ser Ser Ala Ala Ala Ala Ala
    1805                1810                1815

Gly Pro Gly Gln Tyr Gly Pro Gly Gln Gln Gly Pro Ser Ala Ser
    1820                1825                1830

Ala Ala Ala Ala Ala Gly Pro Gly Ser Gly Gln Gln Gly Pro Gly
    1835                1840                1845

Ala Ser Gly Gln Tyr Gly Pro Gly Gln Gln Gly Pro Gly Gln Gln
    1850                1855                1860

Gly Pro Gly Ser Ser Ala Ala Ala Ala Ala Gly Gln Tyr Gly Ser
    1865                1870                1875

Gly Pro Gly Gln Gln Gly Pro Tyr Gly Ser Ala Ala Ala Ala Ala
    1880                1885                1890

Gly Pro Gly Ser Gly Gln Tyr Gly Gln Gly Pro Tyr Gly Pro Gly
    1895                1900                1905

Ala Ser Gly Pro Gly Gln Tyr Gly Pro Gly Gln Gln Gly Pro Ser
    1910                1915                1920

Ala Ser Ala Ala Ala Ala Ala Gly Ser Gly Gln Gln Gly Pro Gly
    1925                1930                1935

Gln Tyr Gly Pro Tyr Ala Ser Ala Ala Ala Ala Ala Gly Gln Tyr
    1940                1945                1950

Gly Ser Gly Pro Gly Gln Gln Gly Pro Tyr Gly Pro Gly Gln Ser
    1955                1960                1965

Gly Ser Gly Gln Gln Gly Pro Gly Gln Gln Gly Pro Tyr Ala Ser
    1970                1975                1980

Ala Ala Ala Ala Ala Gly Pro Gly Gln Gln Gly Pro Tyr Gly Pro
    1985                1990                1995

Gly Ser Ser Ala Ala Ala Ala Ala Gly Gln Tyr Gly Tyr Gly Pro

2000                2005                2010

Gly Gln Gln Gly Pro Tyr Gly Pro Gly Ala Ser Gly Gln Asn Gly
    2015                2020                2025

Pro Gly Ser Gly Gln Tyr Gly Pro Gly Gln Gln Gly Pro Gly Gln
    2030                2035                2040

Ser Ala Ala Ala Ala Ala Gly Pro Gly Gln Gln Gly Pro Tyr Gly
    2045                2050                2055

Pro Gly Ala Ser Ala Ala Ala Ala Ala Gly Gln Tyr Gly Pro Gly
    2060                2065                2070

Gln Gln Gly Pro Gly Gln Tyr Gly Pro Gly Ser Ser Gly Pro Gly
    2075                2080                2085

Gln Gln Gly Pro Tyr Gly Pro Gly Ser Ser Ala Ala Ala Ala Ala
    2090                2095                2100

Gly Gln Tyr Gly Pro Gly Gln Gln Gly Pro Tyr Gly Pro Gly Gln
    2105                2110                2115

Ser Ala Ala Ala Ala Ala Gly Gln Tyr Gln Gln Gly Pro Gly Gln
    2120                2125                2130

Gln Gly Pro Tyr Gly Pro Gly Ala Ser Gly Pro Gly Gln Gln Gly
    2135                2140                2145

Pro Tyr Gly Pro Gly Ala Ser Ala Ala Ala Ala Ala Gly Pro Gly
    2150                2155                2160

Gln Tyr Gly Pro Gly Gln Gln Gly Pro Ser Ala Ser Ala Ala Ala
    2165                2170                2175

Ala Ala Gly Gln Tyr Gly Ser Gly Pro Gly Gln Tyr Gly Pro Tyr
    2180                2185                2190

Gly Pro Gly Gln Ser Gly Pro Gly Ser Gly Gln Gln Gly Gln Gly
    2195                2200                2205

Pro Tyr Gly Pro Gly Ala Ser Ala Ala Ala Ala Ala Gly Gln Tyr
    2210                2215                2220

Gly Pro Gly Gln Gln Gly Pro Tyr Gly Pro Gly Gln Ser Ala Ala
    2225                2230                2235

Ala Ala Ala Gly Pro Gly Ser Gly Gln Tyr Gly Pro Gly Ala Ser
    2240                2245                2250

Gly Gln Asn Gly Pro Gly Ser Gly Gln Tyr Gly Pro Gly Gln Gln
    2255                2260                2265

Gly Pro Gly Gln Ser Ala Ala Ala Ala Ala Gly Gln Tyr Gln Gln
    2270                2275                2280

Gly Pro Gly Gln Gln Gly Pro Tyr Gly Pro Gly Ala Ser Ala Ala
    2285                2290                2295

Ala Ala Ala Gly Gln Tyr Gly Ser Gly Pro Gly Gln Gln Gly Pro
    2300                2305                2310

Tyr Gly Pro Gly Gln Ser Gly Ser Gly Gln Gln Gly Pro Gly Gln
    2315                2320                2325

Gln Gly Pro Tyr Ala Ser Ala Ala Ala Ala Ala Gly Pro Gly Ser
    2330                2335                2340

Gly Gln Gln Gly Ser Ser Val Asp Lys Leu Ala Ala Ala Leu Glu
    2345                2350                2355

His His His His His His
    2360

<210>  10
<211>  597
<212>  PRT
<213>  Artificial Sequence

<220>
<223>  Met-PRT313

<400>  10

Met Gly Pro Gly Gly Gln Gly Pro Tyr Gly Pro Gly Ala Ser Ala Ala
1               5                   10                  15

Ala Ala Ala Gly Gly Asn Gly Pro Gly Ser Gly Gln Gln Gly Pro Gly
            20                  25                  30

Gly Ser Ala Ala Ala Ala Ala Gly Gly Tyr Gly Pro Gly Gly Gln Gly
        35                  40                  45

Pro Gly Gln Gln Gly Pro Gly Ser Ser Ala Ala Ala Ala Ala Gly Pro
    50                  55                  60

Gly Gly Tyr Gly Pro Gly Gly Gln Gly Pro Ser Ala Ser Ala Ala Ala

65                  70                  75                  80

Ala Ala Gly Pro Gly Ser Gly Gln Gln Gly Pro Gly Ala Ser Ala Ala
                85                  90                  95

Ala Ala Ala Gly Gly Tyr Gly Pro Gly Gly Gln Gly Pro Gly Gln Gln
            100                 105                 110

Gly Pro Gly Ser Ser Ala Ala Ala Ala Ala Gly Gly Tyr Gly Ser Gly
        115                 120                 125

Pro Gly Gln Gln Gly Pro Tyr Gly Ser Ala Ala Ala Ala Ala Gly Pro
    130                 135                 140

Gly Ser Gly Gly Tyr Gly Gln Gly Pro Tyr Gly Pro Gly Ala Ser Ala
145                 150                 155                 160

Ala Ala Ala Ala Gly Pro Gly Gly Tyr Gly Pro Gly Gly Gln Gly Pro
                165                 170                 175

Ser Ala Ser Ala Ala Ala Ala Ala Gly Ser Gly Gln Gln Gly Pro Gly
            180                 185                 190

Gly Tyr Gly Pro Tyr Ala Ser Ala Ala Ala Ala Ala Gly Gly Tyr Gly
        195                 200                 205

Ser Gly Pro Gly Gln Gln Gly Pro Tyr Gly Pro Gly Gly Ser Ala Ala
    210                 215                 220

Ala Ala Ala Gly Ser Gly Gln Gln Gly Pro Gly Gln Gln Gly Pro Tyr
225                 230                 235                 240

Ala Ser Ala Ala Ala Ala Ala Gly Pro Gly Gly Gln Gly Pro Tyr Gly
                245                 250                 255

Pro Gly Ser Ser Ala Ala Ala Ala Ala Gly Gly Tyr Gly Tyr Gly Pro
            260                 265                 270

Gly Gly Gln Gly Pro Tyr Gly Pro Gly Ala Ser Ala Ala Ala Ala Ala
        275                 280                 285

Gly Gly Asn Gly Pro Gly Ser Gly Gly Tyr Gly Pro Gly Gln Gln Gly
    290                 295                 300

Pro Gly Gly Ser Ala Ala Ala Ala Ala Gly Pro Gly Gly Gln Gly Pro
305                 310                 315                 320

Tyr Gly Pro Gly Ala Ser Ala Ala Ala Ala Ala Gly Gly Tyr Gly Pro
                325                 330                 335

Gly Gly Gln Gly Pro Gly Gly Tyr Gly Pro Gly Ser Ser Ala Ala Ala
            340                 345                 350

Ala Ala Gly Pro Gly Gly Gln Gly Pro Tyr Gly Pro Gly Ser Ser Ala
        355                 360                 365

Ala Ala Ala Ala Gly Gly Tyr Gly Pro Gly Gln Gln Gly Pro Tyr Gly
    370                 375                 380

Pro Gly Gly Ser Ala Ala Ala Ala Ala Gly Gly Tyr Gln Gln Gly Pro
385                 390                 395                 400

Gly Gly Gln Gly Pro Tyr Gly Pro Gly Ala Ser Ala Ala Ala Ala Ala
                405                 410                 415

Gly Pro Gly Gly Gln Gly Pro Tyr Gly Pro Gly Ala Ser Ala Ala Ala
            420                 425                 430

Ala Ala Gly Pro Gly Gly Tyr Gly Pro Gly Gly Gln Gly Pro Ser Ala
        435                 440                 445

Ser Ala Ala Ala Ala Ala Gly Gly Tyr Gly Ser Gly Pro Gly Gly Tyr
    450                 455                 460

Gly Pro Tyr Gly Pro Gly Gly Ser Ala Ala Ala Ala Ala Gly Pro Gly
465                 470                 475                 480

Ser Gly Gln Gln Gly Gln Gly Pro Tyr Gly Pro Gly Ala Ser Ala Ala
                485                 490                 495

Ala Ala Ala Gly Gly Tyr Gly Pro Gly Gln Gln Gly Pro Tyr Gly Pro
            500                 505                 510

Gly Gly Ser Ala Ala Ala Ala Ala Gly Pro Gly Ser Gly Gly Tyr Gly
        515                 520                 525

Pro Gly Ala Ser Ala Ala Ala Ala Ala Gly Gly Asn Gly Pro Gly Ser
    530                 535                 540

Gly Gly Tyr Gly Pro Gly Gln Gln Gly Pro Gly Gly Ser Ala Ala Ala
545                 550                 555                 560

Ala Ala Gly Gly Tyr Gln Gln Gly Pro Gly Gly Gln Gly Pro Tyr Gly
                565                 570                 575

Pro Gly Ala Ser Ala Ala Ala Ala Ala Gly Pro Gly Ser Gly Gln Gln
            580                 585                 590

Gly Pro Gly Ala Ser
        595

<210>  11
<211>  601
<212>  PRT
<213>  Artificial Sequence

<220>
<223>  PRT410

<400>  11

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Gly Gln
            20                  25                  30

Asn Gly Pro Gly Ser Gly Gln Gln Gly Pro Gly Gln Ser Gly Gln Tyr
        35                  40                  45

Gly Pro Gly Gln Gln Gly Pro Gly Gln Gln Gly Pro Gly Ser Ser Ala
    50                  55                  60

Ala Ala Ala Ala Gly Pro Gly Gln Tyr Gly Pro Gly Gln Gln Gly Pro
65                  70                  75                  80

Ser Ala Ser Ala Ala Ala Ala Ala Gly Pro Gly Ser Gly Gln Gln Gly
                85                  90                  95

Pro Gly Ala Ser Gly Gln Tyr Gly Pro Gly Gln Gln Gly Pro Gly Gln
            100                 105                 110

Gln Gly Pro Gly Ser Ser Ala Ala Ala Ala Ala Gly Gln Tyr Gly Ser
        115                 120                 125

Gly Pro Gly Gln Gln Gly Pro Tyr Gly Ser Ala Ala Ala Ala Ala Gly
    130                 135                 140

Pro Gly Ser Gly Gln Tyr Gly Gln Gly Pro Tyr Gly Pro Gly Ala Ser
145                 150                 155                 160

Gly Pro Gly Gln Tyr Gly Pro Gly Gln Gln Gly Pro Ser Ala Ser Ala
                165                 170                 175

Ala Ala Ala Ala Gly Ser Gly Gln Gln Gly Pro Gly Gln Tyr Gly Pro
        180                 185                 190

Tyr Ala Ser Ala Ala Ala Ala Ala Gly Gln Tyr Gly Ser Gly Pro Gly
    195                 200                 205

Gln Gln Gly Pro Tyr Gly Pro Gly Gln Ser Gly Ser Gly Gln Gln Gly
    210                 215                 220

Pro Gly Gln Gln Gly Pro Tyr Ala Ser Ala Ala Ala Ala Ala Gly Pro
225                 230                 235                 240

Gly Gln Gln Gly Pro Tyr Gly Pro Gly Ser Ser Ala Ala Ala Ala Ala
                245                 250                 255

Gly Gln Tyr Gly Tyr Gly Pro Gly Gln Gln Gly Pro Tyr Gly Pro Gly
        260                 265                 270

Ala Ser Gly Gln Asn Gly Pro Gly Ser Gly Gln Tyr Gly Pro Gly Gln
    275                 280                 285

Gln Gly Pro Gly Gln Ser Ala Ala Ala Ala Ala Gly Pro Gly Gln Gln
    290                 295                 300

Gly Pro Tyr Gly Pro Gly Ala Ser Ala Ala Ala Ala Ala Gly Gln Tyr
305                 310                 315                 320

Gly Pro Gly Gln Gln Gly Pro Gly Gln Tyr Gly Pro Gly Ser Ser Gly
                325                 330                 335

Pro Gly Gln Gln Gly Pro Tyr Gly Pro Gly Ser Ser Ala Ala Ala Ala
        340                 345                 350

Ala Gly Gln Tyr Gly Pro Gly Gln Gln Gly Pro Tyr Gly Pro Gly Gln
    355                 360                 365

Ser Ala Ala Ala Ala Ala Gly Gln Tyr Gln Gln Gly Pro Gly Gln Gln
    370                 375                 380

Gly Pro Tyr Gly Pro Gly Ala Ser Gly Pro Gly Gln Gln Gly Pro Tyr
385                 390                 395                 400

Gly Pro Gly Ala Ser Ala Ala Ala Ala Ala Gly Pro Gly Gln Tyr Gly
                405                 410                 415

Pro Gly Gln Gln Gly Pro Ser Ala Ser Ala Ala Ala Ala Ala Gly Gln
        420                 425                 430

Tyr Gly Ser Gly Pro Gly Gln Tyr Gly Pro Tyr Gly Pro Gly Gln Ser
        435                 440                 445

Gly Pro Gly Ser Gly Gln Gln Gly Gln Gly Pro Tyr Gly Pro Gly Ala
    450                 455                 460

Ser Ala Ala Ala Ala Ala Gly Gln Tyr Gly Pro Gly Gln Gln Gly Pro
465                 470                 475                 480

Tyr Gly Pro Gly Gln Ser Ala Ala Ala Ala Ala Gly Pro Gly Ser Gly
                485                 490                 495

Gln Tyr Gly Pro Gly Ala Ser Gly Gln Asn Gly Pro Gly Ser Gly Gln
            500                 505                 510

Tyr Gly Pro Gly Gln Gln Gly Pro Gly Gln Ser Ala Ala Ala Ala Ala
        515                 520                 525

Gly Gln Tyr Gln Gln Gly Pro Gly Gln Gln Gly Pro Tyr Gly Pro Gly
    530                 535                 540

Ala Ser Ala Ala Ala Ala Ala Gly Gln Tyr Gly Ser Gly Pro Gly Gln
545                 550                 555                 560

Gln Gly Pro Tyr Gly Pro Gly Gln Ser Gly Ser Gly Gln Gln Gly Pro
                565                 570                 575

Gly Gln Gln Gly Pro Tyr Ala Ser Ala Ala Ala Ala Ala Gly Pro Gly
            580                 585                 590

Ser Gly Gln Gln Gly Pro Gly Ala Ser
        595                 600

<210>  12
<211>  12
<212>  PRT
<213>  Artificial Sequence

<220>
<223>  HisTag

<400>  12

Met His His His His His His Ser Ser Gly Ser Ser
1               5                   10

<210>  13
<211>  608
<212>  PRT
<213>  Artificial Sequence

<220>
<223>  PRT380

<400>  13
   Met His His His His His His Ser Ser Gly Ser Ser Gly Pro Gly Gln
   1               5                   10                  15
   Gln Gly Pro Tyr Gly Pro Gly Ala Ser Ala Ala Ala Ala Ala Gly Gln
               20                  25                  30
   Asn Gly Pro Gly Ser Gly Gln Gln Gly Pro Gly Gln Ser Ala Ala Ala
           35                  40                  45
   Ala Ala Gly Gln Tyr Gly Pro Gly Gln Gln Gly Pro Gly Gln Gln Gly
       50                  55                  60
   Pro Gly Ser Ser Ala Ala Ala Ala Ala Gly Pro Gly Gln Tyr Gly Pro
   65                  70                  75                  80
   Gly Gln Gln Gly Pro Ser Ala Ser Ala Ala Ala Ala Ala Gly Pro Gly
                   85                  90                  95
   Ser Gly Gln Gln Gly Pro Gly Ala Ser Ala Ala Ala Ala Ala Gly Gln
               100                 105                 110
   Tyr Gly Pro Gly Gln Gln Gly Pro Gly Gln Gln Gly Pro Gly Ser Ser
           115                 120                 125
   Ala Ala Ala Ala Ala Gly Gln Tyr Gly Ser Gly Pro Gly Gln Gln Gly
       130                 135                 140
   Pro Tyr Gly Ser Ala Ala Ala Ala Ala Gly Pro Gly Ser Gly Gln Tyr
   145                 150                 155                 160
   Gly Gln Gly Pro Tyr Gly Pro Gly Ala Ser Ala Ala Ala Ala Ala Gly
                   165                 170                 175
   Pro Gly Gln Tyr Gly Pro Gly Gln Gln Gly Pro Ser Ala Ser Ala Ala
               180                 185                 190
   Ala Ala Ala Gly Ser Gly Gln Gln Gly Pro Gly Gln Tyr Gly Pro Tyr
           195                 200                 205
   Ala Ser Ala Ala Ala Ala Ala Gly Gln Tyr Gly Ser Gly Pro Gly Gln
       210                 215                 220
   Gln Gly Pro Tyr Gly Pro Gly Gln Ser Ala Ala Ala Ala Ala Gly Ser
   225                 230                 235                 240
   Gly Gln Gln Gly Pro Gly Gln Gln Gly Pro Tyr Ala Ser Ala Ala Ala
                   245                 250                 255
   Ala Ala Gly Pro Gly Gln Gln Gly Pro Tyr Gly Pro Gly Ser Ser Ala
               260                 265                 270
   Ala Ala Ala Ala Gly Gln Tyr Gly Tyr Gly Pro Gly Gln Gln Gly Pro
           275                 280                 285
   Tyr Gly Pro Gly Ala Ser Ala Ala Ala Ala Ala Gly Gln Asn Gly Pro
       290                 295                 300
   Gly Ser Gly Gln Tyr Gly Pro Gly Gln Gln Gly Pro Gly Gln Ser Ala
   305                 310                 315                 320
   Ala Ala Ala Ala Gly Pro Gly Gln Gln Gly Pro Tyr Gly Pro Gly Ala
                   325                 330                 335
   Ser Ala Ala Ala Ala Ala Gly Gln Tyr Gly Pro Gly Gln Gln Gly Pro
               340                 345                 350
   Gly Gln Tyr Gly Pro Gly Ser Ser Ala Ala Ala Ala Ala Gly Pro Gly
           355                 360                 365
   Gln Gln Gly Pro Tyr Gly Pro Gly Ser Ser Ala Ala Ala Ala Ala Gly
       370                 375                 380
   Gln Tyr Gly Pro Gly Gln Gln Gly Pro Tyr Gly Pro Gly Gln Ser Ala
   385                 390                 395                 400
   Ala Ala Ala Ala Gly Gln Tyr Gln Gln Gly Pro Gly Gln Gln Gly Pro
                   405                 410                 415
   Tyr Gly Pro Gly Ala Ser Ala Ala Ala Ala Ala Gly Pro Gly Gln Gln
               420                 425                 430
   Gly Pro Tyr Gly Pro Gly Ala Ser Ala Ala Ala Ala Ala Gly Pro Gly
           435                 440                 445
   Gln Tyr Gly Pro Gly Gln Gln Gly Pro Ser Ala Ser Ala Ala Ala Ala
       450                 455                 460
   Ala Gly Gln Tyr Gly Ser Gly Pro Gly Gln Tyr Gly Pro Tyr Gly Pro
   465                 470                 475                 480
   Gly Gln Ser Ala Ala Ala Ala Ala Gly Pro Gly Ser Gly Gln Gln Gly
                   485                 490                 495
   Gln Gly Pro Tyr Gly Pro Gly Ala Ser Ala Ala Ala Ala Ala Gly Gln
               500                 505                 510
   Tyr Gly Pro Gly Gln Gln Gly Pro Tyr Gly Pro Gly Gln Ser Ala Ala
           515                 520                 525
   Ala Ala Ala Gly Pro Gly Ser Gly Gln Tyr Gly Pro Gly Ala Ser Ala
       530                 535                 540
   Ala Ala Ala Ala Gly Gln Asn Gly Pro Gly Ser Gly Gln Tyr Gly Pro
   545                 550                 555                 560
   Gly Gln Gln Gly Pro Gly Gln Ser Ala Ala Ala Ala Ala Gly Gln Tyr
                   565                 570                 575
   Gln Gln Gly Pro Gly Gln Gln Gly Pro Tyr Gly Pro Gly Ala Ser Ala
               580                 585                 590
   Ala Ala Ala Ala Gly Pro Gly Ser Gly Gln Gln Gly Pro Gly Ala Ser
           595                 600                 605

<210>  14
<211>  576
<212>  PRT
<213>  Artificial Sequence

<220>
<223>  PRT468

<400>  14

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Ala Ala
            20                  25                  30

Gly Ser Asn Gly Pro Gly Ser Gly Gln Gln Gly Pro Gly Gln Ser Gly
        35                  40                  45

Gln Tyr Gly Pro Gly Gln Gln Gly Pro Gly Gln Gln Gly Pro Gly Ser
    50                  55                  60

Ser Ala Ala Ala Ala Ala Ala Ala Gly Pro Gly Gln Tyr Gly Pro Gly
65                  70                  75                  80

Gln Gln Gly Pro Ser Ala Ser Ala Ala Ala Ala Ala Ala Ala Gly Pro

                85                  90                  95

Gly Ser Gly Gln Gln Gly Pro Gly Ala Ser Gly Gln Tyr Gly Pro Gly
            100                 105                 110

Gln Gln Gly Pro Gly Gln Gln Gly Pro Gly Ser Ser Ala Ala Ala Ala
        115                 120                 125

Ala Ala Ala Gly Ser Tyr Gly Ser Gly Pro Gly Gln Gln Gly Pro Tyr
    130                 135                 140

Gly Ser Ala Ala Ala Ala Ala Ala Ala Gly Pro Gly Ser Gly Gln Tyr
145                 150                 155                 160

Gly Gln Gly Pro Tyr Gly Pro Gly Ala Ser Gly Pro Gly Gln Tyr Gly
                165                 170                 175

Pro Gly Gln Gln Gly Pro Ser Ala Ser Ala Ala Ala Ala Ala Ala Ala
            180                 185                 190

Gly Ser Gly Gln Gln Gly Pro Gly Gln Tyr Gly Pro Tyr Ala Ser Ala
        195                 200                 205

Ala Ala Ala Ala Ala Ala Gly Ser Tyr Gly Ser Gly Pro Gly Gln Gln
    210                 215                 220

Gly Pro Tyr Gly Pro Gly Gln Ser Gly Ser Gly Gln Gln Gly Pro Gly
225                 230                 235                 240

Gln Gln Gly Pro Tyr Ala Ser Ala Ala Ala Ala Ala Ala Ala Gly Pro
                245                 250                 255

Gly Gln Gln Gly Pro Tyr Gly Pro Gly Ser Ser Ala Ala Ala Ala Ala
            260                 265                 270

Ala Ala Gly Ser Tyr Gly Tyr Gly Pro Gly Gln Gln Gly Pro Tyr Gly
        275                 280                 285

Pro Gly Ala Ser Gly Gln Asn Gly Pro Gly Ser Gly Gln Tyr Gly Pro
    290                 295                 300

Gly Gln Gln Gly Pro Gly Pro Ser Ala Ala Ala Ala Ala Ala Ala Gly
305                 310                 315                 320

Pro Gly Gln Gln Gly Pro Tyr Gly Pro Gly Ala Ser Ala Ala Ala Ala
                325                 330                 335

Ala Ala Ala Gly Ser Tyr Gly Pro Gly Gln Gln Gly Pro Gly Gln Tyr
            340                 345                 350

Gly Pro Gly Ser Ser Gly Pro Gly Gln Gln Gly Pro Tyr Gly Pro Gly
        355                 360                 365

Ser Ser Ala Ala Ala Ala Ala Ala Ala Gly Ser Tyr Gly Pro Gly Gln
    370                 375                 380

Gln Gly Pro Tyr Gly Pro Gly Pro Ser Ala Ala Ala Ala Ala Ala Ala
385                 390                 395                 400

Gly Ser Tyr Gln Gln Gly Pro Gly Gln Gln Gly Pro Tyr Gly Pro Gly
                405                 410                 415

Ala Ser Gly Pro Gly Gln Gln Gly Pro Tyr Gly Pro Gly Ala Ser Ala
            420                 425                 430

Ala Ala Ala Ala Ala Ala Gly Pro Gly Gln Tyr Gly Pro Gly Gln Gln
        435                 440                 445

Gly Pro Ser Ala Ser Ala Ala Ala Ala Ala Ala Ala Gly Ser Tyr Gly
    450                 455                 460

Ser Gly Pro Gly Gln Tyr Gly Pro Tyr Gly Pro Gly Gln Ser Gly Pro
465                 470                 475                 480

Gly Ser Gly Gln Gln Gly Gln Gly Pro Tyr Gly Pro Gly Ala Ser Ala
                485                 490                 495

Ala Ala Ala Ala Ala Ala Gly Ser Tyr Gly Pro Gly Gln Gln Gly Pro
            500                 505                 510

Tyr Gly Pro Gly Pro Ser Ala Ala Ala Ala Ala Ala Ala Gly Pro Gly
        515                 520                 525

Ser Gly Gln Tyr Gly Pro Gly Ala Ser Gly Gln Asn Gly Pro Gly Ser
    530                 535                 540

Gly Gln Tyr Gly Pro Gly Gln Gln Gly Pro Gly Pro Ser Ala Ala Ala
545                 550                 555                 560

Ala Ala Ala Ala Gly Pro Gly Ser Gly Gln Gln Gly Pro Gly Ala Ser
                565                 570                 575

<210>  15
<211>  2375
<212>  PRT

<213>  Artificial Sequence

<220>
<223>  PRT799

<400>  15

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Gly Gln
            20                  25                  30

Asn Gly Pro Gly Ser Gly Gln Gln Gly Pro Gly Gln Ser Gly Gln Tyr
        35                  40                  45

Gly Pro Gly Gln Gln Gly Pro Gly Gln Gln Gly Pro Gly Ser Ser Ala
    50                  55                  60

Ala Ala Ala Ala Gly Pro Gly Gln Tyr Gly Pro Gly Gln Gln Gly Pro
65                  70                  75                  80

Ser Ala Ser Ala Ala Ala Ala Ala Gly Pro Gly Ser Gly Gln Gln Gly
                85                  90                  95

Pro Gly Ala Ser Gly Gln Tyr Gly Pro Gly Gln Gln Gly Pro Gly Gln
            100                 105                 110

Gln Gly Pro Gly Ser Ser Ala Ala Ala Ala Ala Gly Gln Tyr Gly Ser
        115                 120                 125

Gly Pro Gly Gln Gln Gly Pro Tyr Gly Ser Ala Ala Ala Ala Ala Gly
    130                 135                 140

Pro Gly Ser Gly Gln Tyr Gly Gln Gly Pro Tyr Gly Pro Gly Ala Ser
145                 150                 155                 160

Gly Pro Gly Gln Tyr Gly Pro Gly Gln Gln Gly Pro Ser Ala Ser Ala
                165                 170                 175

Ala Ala Ala Ala Gly Ser Gly Gln Gln Gly Pro Gly Gln Tyr Gly Pro
            180                 185                 190

Tyr Ala Ser Ala Ala Ala Ala Ala Gly Gln Tyr Gly Ser Gly Pro Gly
        195                 200                 205

Gln Gln Gly Pro Tyr Gly Pro Gly Gln Ser Gly Ser Gly Gln Gln Gly
    210                 215                 220

Pro Gly Gln Gln Gly Pro Tyr Ala Ser Ala Ala Ala Ala Ala Gly Pro
225                 230                 235                 240

Gly Gln Gln Gly Pro Tyr Gly Pro Gly Ser Ser Ala Ala Ala Ala Ala
                245                 250                 255

Gly Gln Tyr Gly Tyr Gly Pro Gly Gln Gln Gly Pro Tyr Gly Pro Gly
            260                 265                 270

Ala Ser Gly Gln Asn Gly Pro Gly Ser Gly Gln Tyr Gly Pro Gly Gln
        275                 280                 285

Gln Gly Pro Gly Gln Ser Ala Ala Ala Ala Ala Gly Pro Gly Gln Gln
    290                 295                 300

Gly Pro Tyr Gly Pro Gly Ala Ser Ala Ala Ala Ala Ala Gly Gln Tyr
305                 310                 315                 320

Gly Pro Gly Gln Gln Gly Pro Gly Gln Tyr Gly Pro Gly Ser Ser Gly
                325                 330                 335

Pro Gly Gln Gln Gly Pro Tyr Gly Pro Gly Ser Ser Ala Ala Ala Ala
            340                 345                 350

Ala Gly Gln Tyr Gly Pro Gly Gln Gln Gly Pro Tyr Gly Pro Gly Gln
        355                 360                 365

Ser Ala Ala Ala Ala Ala Gly Gln Tyr Gln Gln Gly Pro Gly Gln Gln
    370                 375                 380

Gly Pro Tyr Gly Pro Gly Ala Ser Gly Pro Gly Gln Gln Gly Pro Tyr
385                 390                 395                 400

Gly Pro Gly Ala Ser Ala Ala Ala Ala Ala Gly Pro Gly Gln Tyr Gly
                405                 410                 415

Pro Gly Gln Gln Gly Pro Ser Ala Ser Ala Ala Ala Ala Ala Gly Gln
            420                 425                 430

Tyr Gly Ser Gly Pro Gly Gln Tyr Gly Pro Tyr Gly Pro Gly Gln Ser
        435                 440                 445

Gly Pro Gly Ser Gly Gln Gln Gly Gln Gly Pro Tyr Gly Pro Gly Ala
    450                 455                 460

Ser Ala Ala Ala Ala Ala Gly Gln Tyr Gly Pro Gly Gln Gln Gly Pro
465                 470                 475                 480

Tyr Gly Pro Gly Gln Ser Ala Ala Ala Ala Ala Gly Pro Gly Ser Gly
                    485                 490                 495

Gln Tyr Gly Pro Gly Ala Ser Gly Gln Asn Gly Pro Gly Ser Gly Gln
                500                 505                 510

Tyr Gly Pro Gly Gln Gln Gly Pro Gly Gln Ser Ala Ala Ala Ala Ala
            515                 520                 525

Gly Gln Tyr Gln Gln Gly Pro Gly Gln Gln Gly Pro Tyr Gly Pro Gly
    530                 535                 540

Ala Ser Ala Ala Ala Ala Ala Gly Gln Tyr Gly Ser Gly Pro Gly Gln
545                 550                 555                 560

Gln Gly Pro Tyr Gly Pro Gly Gln Ser Gly Ser Gly Gln Gln Gly Pro
                565                 570                 575

Gly Gln Gln Gly Pro Tyr Ala Ser Ala Ala Ala Ala Ala Gly Pro Gly
                580                 585                 590

Ser Gly Gln Gln Gly Pro Gly Ala Ser Gly Gln Gln Gly Pro Tyr Gly
            595                 600                 605

Pro Gly Ala Ser Ala Ala Ala Ala Ala Gly Gln Asn Gly Pro Gly Ser
    610                 615                 620

Gly Gln Gln Gly Pro Gly Gln Ser Gly Gln Tyr Gly Pro Gly Gln Gln
625                 630                 635                 640

Gly Pro Gly Gln Gln Gly Pro Gly Ser Ser Ala Ala Ala Ala Ala Gly
                645                 650                 655

Pro Gly Gln Tyr Gly Pro Gly Gln Gln Gly Pro Ser Ala Ser Ala Ala
                660                 665                 670

Ala Ala Ala Gly Pro Gly Ser Gly Gln Gln Gly Pro Gly Ala Ser Gly
            675                 680                 685

Gln Tyr Gly Pro Gly Gln Gln Gly Pro Gly Gln Gln Gly Pro Gly Ser
    690                 695                 700

Ser Ala Ala Ala Ala Ala Gly Gln Tyr Gly Ser Gly Pro Gly Gln Gln
705                 710                 715                 720

Gly Pro Tyr Gly Ser Ala Ala Ala Ala Ala Gly Pro Gly Ser Gly Gln
                725                 730                 735

Tyr Gly Gln Gly Pro Tyr Gly Pro Gly Ala Ser Gly Pro Gly Gln Tyr
            740                 745                 750

Gly Pro Gly Gln Gln Gly Pro Ser Ala Ser Ala Ala Ala Ala Ala Gly
        755                 760                 765

Ser Gly Gln Gln Gly Pro Gly Gln Tyr Gly Pro Tyr Ala Ser Ala Ala
    770                 775                 780

Ala Ala Ala Gly Gln Tyr Gly Ser Gly Pro Gly Gln Gln Gly Pro Tyr
785                 790                 795                 800

Gly Pro Gly Gln Ser Gly Ser Gly Gln Gln Gly Pro Gly Gln Gln Gly
                805                 810                 815

Pro Tyr Ala Ser Ala Ala Ala Ala Ala Gly Pro Gly Gln Gln Gly Pro
            820                 825                 830

Tyr Gly Pro Gly Ser Ser Ala Ala Ala Ala Ala Gly Gln Tyr Gly Tyr
        835                 840                 845

Gly Pro Gly Gln Gln Gly Pro Tyr Gly Pro Gly Ala Ser Gly Gln Asn
    850                 855                 860

Gly Pro Gly Ser Gly Gln Tyr Gly Pro Gly Gln Gln Gly Pro Gly Gln
865                 870                 875                 880

Ser Ala Ala Ala Ala Ala Gly Pro Gly Gln Gln Gly Pro Tyr Gly Pro
                885                 890                 895

Gly Ala Ser Ala Ala Ala Ala Ala Gly Gln Tyr Gly Pro Gly Gln Gln
            900                 905                 910

Gly Pro Gly Gln Tyr Gly Pro Gly Ser Ser Gly Pro Gly Gln Gln Gly
        915                 920                 925

Pro Tyr Gly Pro Gly Ser Ser Ala Ala Ala Ala Ala Gly Gln Tyr Gly
    930                 935                 940

Pro Gly Gln Gln Gly Pro Tyr Gly Pro Gly Gln Ser Ala Ala Ala Ala
945                 950                 955                 960

Ala Gly Gln Tyr Gln Gln Gly Pro Gly Gln Gln Gly Pro Tyr Gly Pro
                965                 970                 975

Gly Ala Ser Gly Pro Gly Gln Gln Gly Pro Tyr Gly Pro Gly Ala Ser

            980                 985                 990

Ala Ala Ala Ala Ala Gly Pro Gly Gln Tyr Gly Pro Gly Gln Gln Gly
        995                 1000                1005

Pro Ser Ala Ser Ala Ala Ala Ala Ala Gly Gln Tyr Gly Ser Gly
    1010                1015                1020

Pro Gly Gln Tyr Gly Pro Tyr Gly Pro Gly Gln Ser Gly Pro Gly
    1025                1030                1035

Ser Gly Gln Gln Gly Gln Gly Pro Tyr Gly Pro Gly Ala Ser Ala
    1040                1045                1050

Ala Ala Ala Ala Gly Gln Tyr Gly Pro Gly Gln Gln Gly Pro Tyr
    1055                1060                1065

Gly Pro Gly Gln Ser Ala Ala Ala Ala Ala Gly Pro Gly Ser Gly
    1070                1075                1080

Gln Tyr Gly Pro Gly Ala Ser Gly Gln Asn Gly Pro Gly Ser Gly
    1085                1090                1095

Gln Tyr Gly Pro Gly Gln Gln Gly Pro Gly Gln Ser Ala Ala Ala
    1100                1105                1110

Ala Ala Gly Gln Tyr Gln Gln Gly Pro Gly Gln Gln Gly Pro Tyr
    1115                1120                1125

Gly Pro Gly Ala Ser Ala Ala Ala Ala Ala Gly Gln Tyr Gly Ser
    1130                1135                1140

Gly Pro Gly Gln Gln Gly Pro Tyr Gly Pro Gly Gln Ser Gly Ser
    1145                1150                1155

Gly Gln Gln Gly Pro Gly Gln Gln Gly Pro Tyr Ala Ser Ala Ala
    1160                1165                1170

Ala Ala Ala Gly Pro Gly Ser Gly Gln Gln Gly Pro Gly Ala Ser
    1175                1180                1185

Gly Gln Gln Gly Pro Tyr Gly Pro Gly Ala Ser Ala Ala Ala Ala
    1190                1195                1200

Ala Gly Gln Asn Gly Pro Gly Ser Gly Gln Gln Gly Pro Gly Gln
    1205                1210                1215

Ser Gly Gln Tyr Gly Pro Gly Gln Gln Gly Pro Gly Gln Gln Gly
    1220                1225                1230

Pro Gly Ser Ser Ala Ala Ala Ala Ala Gly Pro Gly Gln Tyr Gly
    1235                1240                1245

Pro Gly Gln Gln Gly Pro Ser Ala Ser Ala Ala Ala Ala Ala Gly
    1250                1255                1260

Pro Gly Ser Gly Gln Gln Gly Pro Gly Ala Ser Gly Gln Tyr Gly
    1265                1270                1275

Pro Gly Gln Gln Gly Pro Gly Gln Gln Gly Pro Gly Ser Ser Ala
    1280                1285                1290

Ala Ala Ala Ala Gly Gln Tyr Gly Ser Gly Pro Gly Gln Gln Gly
    1295                1300                1305

Pro Tyr Gly Ser Ala Ala Ala Ala Ala Gly Pro Gly Ser Gly Gln
    1310                1315                1320

Tyr Gly Gln Gly Pro Tyr Gly Pro Gly Ala Ser Gly Pro Gly Gln
    1325                1330                1335

Tyr Gly Pro Gly Gln Gln Gly Pro Ser Ala Ser Ala Ala Ala Ala
    1340                1345                1350

Ala Gly Ser Gly Gln Gln Gly Pro Gly Gln Tyr Gly Pro Tyr Ala
    1355                1360                1365

Ser Ala Ala Ala Ala Ala Gly Gln Tyr Gly Ser Gly Pro Gly Gln
    1370                1375                1380

Gln Gly Pro Tyr Gly Pro Gly Gln Ser Gly Ser Gly Gln Gln Gly
    1385                1390                1395

Pro Gly Gln Gln Gly Pro Tyr Ala Ser Ala Ala Ala Ala Ala Gly
    1400                1405                1410

Pro Gly Gln Gln Gly Pro Tyr Gly Pro Gly Ser Ser Ala Ala Ala
    1415                1420                1425

Ala Ala Gly Gln Tyr Gly Tyr Gly Pro Gly Gln Gln Gly Pro Tyr
    1430                1435                1440

Gly Pro Gly Ala Ser Gly Gln Asn Gly Pro Gly Ser Gly Gln Tyr
    1445                1450                1455

Gly Pro Gly Gln Gln Gly Pro Gly Gln Ser Ala Ala Ala Ala Ala
    1460                1465                1470

Gly Pro Gly Gln Gln Gly Pro Tyr Gly Pro Gly Ala Ser Ala Ala
    1475                1480                1485

Ala Ala Ala Gly Gln Tyr Gly Pro Gly Gln Gln Gly Pro Gly Gln
    1490                1495                1500

Tyr Gly Pro Gly Ser Ser Gly Pro Gly Gln Gln Gly Pro Tyr Gly
    1505                1510                1515

Pro Gly Ser Ser Ala Ala Ala Ala Ala Gly Gln Tyr Gly Pro Gly
    1520                1525                1530

Gln Gln Gly Pro Tyr Gly Pro Gly Gln Ser Ala Ala Ala Ala Ala
    1535                1540                1545

Gly Gln Tyr Gln Gln Gly Pro Gly Gln Gln Gly Pro Tyr Gly Pro
    1550                1555                1560

Gly Ala Ser Gly Pro Gly Gln Gln Gly Pro Tyr Gly Pro Gly Ala
    1565                1570                1575

Ser Ala Ala Ala Ala Ala Gly Pro Gly Gln Tyr Gly Pro Gly Gln
    1580                1585                1590

Gln Gly Pro Ser Ala Ser Ala Ala Ala Ala Ala Gly Gln Tyr Gly
    1595                1600                1605

Ser Gly Pro Gly Gln Tyr Gly Pro Tyr Gly Pro Gly Gln Ser Gly
    1610                1615                1620

Pro Gly Ser Gly Gln Gln Gly Gln Gly Pro Tyr Gly Pro Gly Ala
    1625                1630                1635

Ser Ala Ala Ala Ala Ala Gly Gln Tyr Gly Pro Gly Gln Gln Gly
    1640                1645                1650

Pro Tyr Gly Pro Gly Gln Ser Ala Ala Ala Ala Ala Gly Pro Gly
    1655                1660                1665

Ser Gly Gln Tyr Gly Pro Gly Ala Ser Gly Gln Asn Gly Pro Gly
    1670                1675                1680

Ser Gly Gln Tyr Gly Pro Gly Gln Gln Gly Pro Gly Gln Ser Ala
    1685                1690                1695

Ala Ala Ala Ala Gly Gln Tyr Gln Gln Gly Pro Gly Gln Gln Gly
    1700                1705                1710

Pro Tyr Gly Pro Gly Ala Ser Ala Ala Ala Ala Ala Gly Gln Tyr
    1715                1720                1725

Gly Ser Gly Pro Gly Gln Gln Gly Pro Tyr Gly Pro Gly Gln Ser
    1730                1735                1740

Gly Ser Gly Gln Gln Gly Pro Gly Gln Gln Gly Pro Tyr Ala Ser
    1745                1750                1755

Ala Ala Ala Ala Ala Gly Pro Gly Ser Gly Gln Gln Gly Pro Gly
    1760                1765                1770

Ala Ser Gly Gln Gln Gly Pro Tyr Gly Pro Gly Ala Ser Ala Ala
    1775                1780                1785

Ala Ala Ala Gly Gln Asn Gly Pro Gly Ser Gly Gln Gln Gly Pro
    1790                1795                1800

Gly Gln Ser Gly Gln Tyr Gly Pro Gly Gln Gln Gly Pro Gly Gln
    1805                1810                1815

Gln Gly Pro Gly Ser Ser Ala Ala Ala Ala Ala Gly Pro Gly Gln
    1820                1825                1830

Tyr Gly Pro Gly Gln Gln Gly Pro Ser Ala Ser Ala Ala Ala Ala
    1835                1840                1845

Ala Gly Pro Gly Ser Gly Gln Gln Gly Pro Gly Ala Ser Gly Gln
    1850                1855                1860

Tyr Gly Pro Gly Gln Gln Gly Pro Gly Gln Gln Gly Pro Gly Ser
    1865                1870                1875

Ser Ala Ala Ala Ala Ala Gly Gln Tyr Gly Ser Gly Pro Gly Gln
    1880                1885                1890

Gln Gly Pro Tyr Gly Ser Ala Ala Ala Ala Ala Gly Pro Gly Ser
    1895                1900                1905

Gly Gln Tyr Gly Gln Gly Pro Tyr Gly Pro Gly Ala Ser Gly Pro
    1910                1915                1920

Gly Gln Tyr Gly Pro Gly Gln Gln Gly Pro Ser Ala Ser Ala Ala

1925                1930                1935

Ala Ala Ala Gly Ser Gly Gln Gln Gly Pro Gly Gln Tyr Gly Pro
    1940                1945                1950

Tyr Ala Ser Ala Ala Ala Ala Ala Gly Gln Tyr Gly Ser Gly Pro
    1955                1960                1965

Gly Gln Gln Gly Pro Tyr Gly Pro Gly Gln Ser Gly Ser Gly Gln
    1970                1975                1980

Gln Gly Pro Gly Gln Gln Gly Pro Tyr Ala Ser Ala Ala Ala Ala
    1985                1990                1995

Ala Gly Pro Gly Gln Gln Gly Pro Tyr Gly Pro Gly Ser Ser Ala
    2000                2005                2010

Ala Ala Ala Ala Gly Gln Tyr Gly Tyr Gly Pro Gly Gln Gln Gly
    2015                2020                2025

Pro Tyr Gly Pro Gly Ala Ser Gly Gln Asn Gly Pro Gly Ser Gly
    2030                2035                2040

Gln Tyr Gly Pro Gly Gln Gln Gly Pro Gly Gln Ser Ala Ala Ala
    2045                2050                2055

Ala Ala Gly Pro Gly Gln Gln Gly Pro Tyr Gly Pro Gly Ala Ser
    2060                2065                2070

Ala Ala Ala Ala Ala Gly Gln Tyr Gly Pro Gly Gln Gln Gly Pro
    2075                2080                2085

Gly Gln Tyr Gly Pro Gly Ser Ser Gly Pro Gly Gln Gln Gly Pro
    2090                2095                2100

Tyr Gly Pro Gly Ser Ser Ala Ala Ala Ala Ala Gly Gln Tyr Gly
    2105                2110                2115

Pro Gly Gln Gln Gly Pro Tyr Gly Pro Gly Gln Ser Ala Ala Ala
    2120                2125                2130

Ala Ala Gly Gln Tyr Gln Gln Gly Pro Gly Gln Gln Gly Pro Tyr
    2135                2140                2145

Gly Pro Gly Ala Ser Gly Pro Gly Gln Gln Gly Pro Tyr Gly Pro
    2150                2155                2160

Gly Ala Ser Ala Ala Ala Ala Ala Gly Pro Gly Gln Tyr Gly Pro
    2165                2170                2175

Gly Gln Gln Gly Pro Ser Ala Ser Ala Ala Ala Ala Ala Gly Gln
    2180                2185                2190

Tyr Gly Ser Gly Pro Gly Gln Tyr Gly Pro Tyr Gly Pro Gly Gln
    2195                2200                2205

Ser Gly Pro Gly Ser Gly Gln Gln Gly Gln Gly Pro Tyr Gly Pro
    2210                2215                2220

Gly Ala Ser Ala Ala Ala Ala Ala Gly Gln Tyr Gly Pro Gly Gln
    2225                2230                2235

Gln Gly Pro Tyr Gly Pro Gly Gln Ser Ala Ala Ala Ala Ala Gly
    2240                2245                2250

Pro Gly Ser Gly Gln Tyr Gly Pro Gly Ala Ser Gly Gln Asn Gly
    2255                2260                2265

Pro Gly Ser Gly Gln Tyr Gly Pro Gly Gln Gln Gly Pro Gly Gln
    2270                2275                2280

Ser Ala Ala Ala Ala Ala Gly Gln Tyr Gln Gln Gly Pro Gly Gln
    2285                2290                2295

Gln Gly Pro Tyr Gly Pro Gly Ala Ser Ala Ala Ala Ala Ala Gly
    2300                2305                2310

Gln Tyr Gly Ser Gly Pro Gly Gln Gln Gly Pro Tyr Gly Pro Gly
    2315                2320                2325

Gln Ser Gly Ser Gly Gln Gln Gly Pro Gly Gln Gln Gly Pro Tyr
    2330                2335                2340

Ala Ser Ala Ala Ala Ala Ala Gly Pro Gly Ser Gly Gln Gln Gly
    2345                2350                2355

Ser Ser Val Asp Lys Leu Ala Ala Ala Leu Glu His His His His
    2360                2365                2370

His His
    2375

<210>  16
<211>  608
<212>  PRT

<213>  Artificial Sequence

<220>
<223>  PRT313

<400>  16
   Met His His His His His His Ser Ser Gly Ser Ser Gly Pro Gly Gly
   1               5                   10                  15
   Gln Gly Pro Tyr Gly Pro Gly Ala Ser Ala Ala Ala Ala Ala Gly Gly
               20                  25                  30
   Asn Gly Pro Gly Ser Gly Gln Gln Gly Pro Gly Gly Ser Ala Ala Ala
           35                  40                  45
   Ala Ala Gly Gly Tyr Gly Pro Gly Gly Gln Gly Pro Gly Gln Gln Gly
       50                  55                  60
   Pro Gly Ser Ser Ala Ala Ala Ala Ala Gly Pro Gly Gly Tyr Gly Pro
   65                  70                  75                  80
   Gly Gly Gln Gly Pro Ser Ala Ser Ala Ala Ala Ala Ala Gly Pro Gly
                   85                  90                  95
   Ser Gly Gln Gln Gly Pro Gly Ala Ser Ala Ala Ala Ala Ala Gly Gly
               100                 105                 110
   Tyr Gly Pro Gly Gly Gln Gly Pro Gly Gln Gln Gly Pro Gly Ser Ser
           115                 120                 125
   Ala Ala Ala Ala Ala Gly Gly Tyr Gly Ser Gly Pro Gly Gln Gln Gly
       130                 135                 140
   Pro Tyr Gly Ser Ala Ala Ala Ala Ala Gly Pro Gly Ser Gly Gly Tyr
   145                 150                 155                 160
   Gly Gln Gly Pro Tyr Gly Pro Gly Ala Ser Ala Ala Ala Ala Ala Gly
                   165                 170                 175
   Pro Gly Gly Tyr Gly Pro Gly Gly Gln Gly Pro Ser Ala Ser Ala Ala
               180                 185                 190
   Ala Ala Ala Gly Ser Gly Gln Gln Gly Pro Gly Gly Tyr Gly Pro Tyr
           195                 200                 205
   Ala Ser Ala Ala Ala Ala Ala Gly Gly Tyr Gly Ser Gly Pro Gly Gln
       210                 215                 220
   Gln Gly Pro Tyr Gly Pro Gly Gly Ser Ala Ala Ala Ala Ala Gly Ser
   225                 230                 235                 240
   Gly Gln Gln Gly Pro Gly Gln Gln Gly Pro Tyr Ala Ser Ala Ala Ala
                   245                 250                 255
   Ala Ala Gly Pro Gly Gly Gln Gly Pro Tyr Gly Pro Gly Ser Ser Ala
               260                 265                 270
   Ala Ala Ala Ala Gly Gly Tyr Gly Tyr Gly Pro Gly Gly Gln Gly Pro
           275                 280                 285
   Tyr Gly Pro Gly Ala Ser Ala Ala Ala Ala Ala Gly Gly Asn Gly Pro
       290                 295                 300
   Gly Ser Gly Gly Tyr Gly Pro Gly Gln Gln Gly Pro Gly Gly Ser Ala
   305                 310                 315                 320
   Ala Ala Ala Ala Gly Pro Gly Gly Gln Gly Pro Tyr Gly Pro Gly Ala
                   325                 330                 335
   Ser Ala Ala Ala Ala Ala Gly Gly Tyr Gly Pro Gly Gly Gln Gly Pro
               340                 345                 350
   Gly Gly Tyr Gly Pro Gly Ser Ser Ala Ala Ala Ala Ala Gly Pro Gly
           355                 360                 365
   Gly Gln Gly Pro Tyr Gly Pro Gly Ser Ser Ala Ala Ala Ala Ala Gly
       370                 375                 380
   Gly Tyr Gly Pro Gly Gln Gln Gly Pro Tyr Gly Pro Gly Gly Ser Ala
   385                 390                 395                 400
   Ala Ala Ala Ala Gly Gly Tyr Gln Gln Gly Pro Gly Gly Gln Gly Pro
                   405                 410                 415
   Tyr Gly Pro Gly Ala Ser Ala Ala Ala Ala Ala Gly Pro Gly Gly Gln
               420                 425                 430
   Gly Pro Tyr Gly Pro Gly Ala Ser Ala Ala Ala Ala Ala Gly Pro Gly
           435                 440                 445
   Gly Tyr Gly Pro Gly Gly Gln Gly Pro Ser Ala Ser Ala Ala Ala Ala
       450                 455                 460
   Ala Gly Gly Tyr Gly Ser Gly Pro Gly Gly Tyr Gly Pro Tyr Gly Pro
   465                 470                 475                 480
   Gly Gly Ser Ala Ala Ala Ala Ala Gly Pro Gly Ser Gly Gln Gln Gly
                   485                 490                 495
   Gln Gly Pro Tyr Gly Pro Gly Ala Ser Ala Ala Ala Ala Ala Gly Gly
               500                 505                 510
   Tyr Gly Pro Gly Gln Gln Gly Pro Tyr Gly Pro Gly Gly Ser Ala Ala
           515                 520                 525
   Ala Ala Ala Gly Pro Gly Ser Gly Gly Tyr Gly Pro Gly Ala Ser Ala
       530                 535                 540
   Ala Ala Ala Ala Gly Gly Asn Gly Pro Gly Ser Gly Gly Tyr Gly Pro
   545                 550                 555                 560
   Gly Gln Gln Gly Pro Gly Gly Ser Ala Ala Ala Ala Ala Gly Gly Tyr
                   565                 570                 575
   Gln Gln Gly Pro Gly Gly Gln Gly Pro Tyr Gly Pro Gly Ala Ser Ala
               580                 585                 590
   Ala Ala Ala Ala Gly Pro Gly Ser Gly Gln Gln Gly Pro Gly Ala Ser
           595                 600                 605

<210>  17
<211>  601
<212>  PRT
<213>  Artificial Sequence

<220>
<223>  PRT399

<400>  17
   Met His His His His His His Ser Ser Gly Ser Ser Gly Pro Gly Gly
   1               5                   10                  15
   Gln Gly Pro Tyr Gly Pro Gly Ala Ser Ala Ala Ala Ala Ala Gly Gly
               20                  25                  30
   Asn Gly Pro Gly Ser Gly Gln Gln Gly Pro Gly Gly Ser Gly Gly Tyr
           35                  40                  45
   Gly Pro Gly Gly Gln Gly Pro Gly Gln Gln Gly Pro Gly Ser Ser Ala
       50                  55                  60
   Ala Ala Ala Ala Gly Pro Gly Gly Tyr Gly Pro Gly Gly Gln Gly Pro
   65                  70                  75                  80
   Ser Ala Ser Ala Ala Ala Ala Ala Gly Pro Gly Ser Gly Gln Gln Gly
                   85                  90                  95
   Pro Gly Ala Ser Gly Gly Tyr Gly Pro Gly Gly Gln Gly Pro Gly Gln
               100                 105                 110
   Gln Gly Pro Gly Ser Ser Ala Ala Ala Ala Ala Gly Gly Tyr Gly Ser
           115                 120                 125
   Gly Pro Gly Gln Gln Gly Pro Tyr Gly Ser Ala Ala Ala Ala Ala Gly
       130                 135                 140
   Pro Gly Ser Gly Gly Tyr Gly Gln Gly Pro Tyr Gly Pro Gly Ala Ser
   145                 150                 155                 160
   Gly Pro Gly Gly Tyr Gly Pro Gly Gly Gln Gly Pro Ser Ala Ser Ala
                   165                 170                 175
   Ala Ala Ala Ala Gly Ser Gly Gln Gln Gly Pro Gly Gly Tyr Gly Pro
               180                 185                 190
   Tyr Ala Ser Ala Ala Ala Ala Ala Gly Gly Tyr Gly Ser Gly Pro Gly
           195                 200                 205
   Gln Gln Gly Pro Tyr Gly Pro Gly Gly Ser Gly Ser Gly Gln Gln Gly
       210                 215                 220
   Pro Gly Gln Gln Gly Pro Tyr Ala Ser Ala Ala Ala Ala Ala Gly Pro
   225                 230                 235                 240
   Gly Gly Gln Gly Pro Tyr Gly Pro Gly Ser Ser Ala Ala Ala Ala Ala
                   245                 250                 255
   Gly Gly Tyr Gly Tyr Gly Pro Gly Gly Gln Gly Pro Tyr Gly Pro Gly
               260                 265                 270
   Ala Ser Gly Gly Asn Gly Pro Gly Ser Gly Gly Tyr Gly Pro Gly Gln
           275                 280                 285
   Gln Gly Pro Gly Gly Ser Ala Ala Ala Ala Ala Gly Pro Gly Gly Gln
       290                 295                 300
   Gly Pro Tyr Gly Pro Gly Ala Ser Ala Ala Ala Ala Ala Gly Gly Tyr
   305                 310                 315                 320
   Gly Pro Gly Gly Gln Gly Pro Gly Gly Tyr Gly Pro Gly Ser Ser Gly
                   325                 330                 335
   Pro Gly Gly Gln Gly Pro Tyr Gly Pro Gly Ser Ser Ala Ala Ala Ala
   340 345 350
   Ala Gly Gly Tyr Gly Pro Gly Gln Gln Gly Pro Tyr Gly Pro Gly Gly
   355 360 365
   Ser Ala Ala Ala Ala Ala Gly Gly Tyr Gln Gln Gly Pro Gly Gly Gln
   370 375 380
   Gly Pro Tyr Gly Pro Gly Ala Ser Gly Pro Gly Gly Gln Gly Pro Tyr
   385 390 395 400
   Gly Pro Gly Ala Ser Ala Ala Ala Ala Ala Gly Pro Gly Gly Tyr Gly
   405 410 415
   Pro Gly Gly Gln Gly Pro Ser Ala Ser Ala Ala Ala Ala Ala Gly Gly
   420 425 430
   Tyr Gly Ser Gly Pro Gly Gly Tyr Gly Pro Tyr Gly Pro Gly Gly Ser
   435 440 445
   Gly Pro Gly Ser Gly Gln Gln Gly Gln Gly Pro Tyr Gly Pro Gly Ala
   450 455 460
   Ser Ala Ala Ala Ala Ala Gly Gly Tyr Gly Pro Gly Gln Gln Gly Pro
   465 470 475 480
   Tyr Gly Pro Gly Gly Ser Ala Ala Ala Ala Ala Gly Pro Gly Ser Gly
   485 490 495
   Gly Tyr Gly Pro Gly Ala Ser Gly Gly Asn Gly Pro Gly Ser Gly Gly
   500 505 510
   Tyr Gly Pro Gly Gln Gln Gly Pro Gly Gly Ser Ala Ala Ala Ala Ala
   515 520 525
   Gly Gly Tyr Gln Gln Gly Pro Gly Gly Gln Gly Pro Tyr Gly Pro Gly
   530 535 540
   Ala Ser Ala Ala Ala Ala Ala Gly Gly Tyr Gly Ser Gly Pro Gly Gln
   545 550 555 560
   Gln Gly Pro Tyr Gly Pro Gly Gly Ser Gly Ser Gly Gln Gln Gly Pro
   565 570 575
   Gly Gln Gln Gly Pro Tyr Ala Ser Ala Ala Ala Ala Ala Gly Pro Gly
   580 585 590
   Ser Gly Gln Gln Gly Pro Gly Ala Ser
           595                 600

<210>  18
<211>  590
<212>  PRT
<213>  Artificial Sequence

<220>
<223>  Met-PRT399

<400>  18
   Met Gly Pro Gly Gly Gln Gly Pro Tyr Gly Pro Gly Ala Ser Ala Ala
   1               5                   10                  15
   Ala Ala Ala Gly Gly Asn Gly Pro Gly Ser Gly Gln Gln Gly Pro Gly
               20                  25                  30
   Gly Ser Gly Gly Tyr Gly Pro Gly Gly Gln Gly Pro Gly Gln Gln Gly
           35                  40                  45
   Pro Gly Ser Ser Ala Ala Ala Ala Ala Gly Pro Gly Gly Tyr Gly Pro
       50                  55                  60
   Gly Gly Gln Gly Pro Ser Ala Ser Ala Ala Ala Ala Ala Gly Pro Gly
   65                  70                  75                  80
   Ser Gly Gln Gln Gly Pro Gly Ala Ser Gly Gly Tyr Gly Pro Gly Gly
                   85                  90                  95
   Gln Gly Pro Gly Gln Gln Gly Pro Gly Ser Ser Ala Ala Ala Ala Ala
               100                 105                 110
   Gly Gly Tyr Gly Ser Gly Pro Gly Gln Gln Gly Pro Tyr Gly Ser Ala
           115                 120                 125
   Ala Ala Ala Ala Gly Pro Gly Ser Gly Gly Tyr Gly Gln Gly Pro Tyr
       130                 135                 140
   Gly Pro Gly Ala Ser Gly Pro Gly Gly Tyr Gly Pro Gly Gly Gln Gly
   145                 150                 155                 160
   Pro Ser Ala Ser Ala Ala Ala Ala Ala Gly Ser Gly Gln Gln Gly Pro
                   165                 170                 175
   Gly Gly Tyr Gly Pro Tyr Ala Ser Ala Ala Ala Ala Ala Gly Gly Tyr
               180                 185                 190
   Gly Ser Gly Pro Gly Gln Gln Gly Pro Tyr Gly Pro Gly Gly Ser Gly
           195                 200                 205
   Ser Gly Gln Gln Gly Pro Gly Gln Gln Gly Pro Tyr Ala Ser Ala Ala
       210                 215                 220
   Ala Ala Ala Gly Pro Gly Gly Gln Gly Pro Tyr Gly Pro Gly Ser Ser
   225                 230                 235                 240
   Ala Ala Ala Ala Ala Gly Gly Tyr Gly Tyr Gly Pro Gly Gly Gln Gly
                   245                 250                 255
   Pro Tyr Gly Pro Gly Ala Ser Gly Gly Asn Gly Pro Gly Ser Gly Gly
               260                 265                 270
   Tyr Gly Pro Gly Gln Gln Gly Pro Gly Gly Ser Ala Ala Ala Ala Ala
           275                 280                 285
   Gly Pro Gly Gly Gln Gly Pro Tyr Gly Pro Gly Ala Ser Ala Ala Ala
       290                 295                 300
   Ala Ala Gly Gly Tyr Gly Pro Gly Gly Gln Gly Pro Gly Gly Tyr Gly
   305                 310                 315                 320
   Pro Gly Ser Ser Gly Pro Gly Gly Gln Gly Pro Tyr Gly Pro Gly Ser
                   325                 330                 335
   Ser Ala Ala Ala Ala Ala Gly Gly Tyr Gly Pro Gly Gln Gln Gly Pro
               340                 345                 350
   Tyr Gly Pro Gly Gly Ser Ala Ala Ala Ala Ala Gly Gly Tyr Gln Gln
           355                 360                 365
   Gly Pro Gly Gly Gln Gly Pro Tyr Gly Pro Gly Ala Ser Gly Pro Gly
       370                 375                 380
   Gly Gln Gly Pro Tyr Gly Pro Gly Ala Ser Ala Ala Ala Ala Ala Gly
   385                 390                 395                 400
   Pro Gly Gly Tyr Gly Pro Gly Gly Gln Gly Pro Ser Ala Ser Ala Ala
                   405                 410                 415
   Ala Ala Ala Gly Gly Tyr Gly Ser Gly Pro Gly Gly Tyr Gly Pro Tyr
               420                 425                 430
   Gly Pro Gly Gly Ser Gly Pro Gly Ser Gly Gln Gln Gly Gln Gly Pro
           435                 440                 445
   Tyr Gly Pro Gly Ala Ser Ala Ala Ala Ala Ala Gly Gly Tyr Gly Pro
       450                 455                 460
   Gly Gln Gln Gly Pro Tyr Gly Pro Gly Gly Ser Ala Ala Ala Ala Ala
   465                 470                 475                 480
   Gly Pro Gly Ser Gly Gly Tyr Gly Pro Gly Ala Ser Gly Gly Asn Gly
                   485                 490                 495
   Pro Gly Ser Gly Gly Tyr Gly Pro Gly Gln Gln Gly Pro Gly Gly Ser
               500                 505                 510
   Ala Ala Ala Ala Ala Gly Gly Tyr Gln Gln Gly Pro Gly Gly Gln Gly
           515                 520                 525
   Pro Tyr Gly Pro Gly Ala Ser Ala Ala Ala Ala Ala Gly Gly Tyr Gly
       530                 535                 540
   Ser Gly Pro Gly Gln Gln Gly Pro Tyr Gly Pro Gly Gly Ser Gly Ser
   545                 550                 555                 560
   Gly Gln Gln Gly Pro Gly Gln Gln Gly Pro Tyr Ala Ser Ala Ala Ala
                   565                 570                 575
   Ala Ala Gly Pro Gly Ser Gly Gln Gln Gly Pro Gly Ala Ser
               580                 585                 590

<210>  19
<211>  612
<212>  PRT
<213>  Artificial Sequence

<220>
<223>  Met-PRT720

<400>  19

Met Gly Pro Gly Gln Gln Gly Pro Tyr Gly Pro Gly Ala Ser Ala Ala
1               5                   10                  15

Ala Ala Ala Gly Gln Asn Gly Pro Gly Ser Gly Gln Gln Gly Pro Gly
            20                  25                  30

Gln Ser Gly Gln Tyr Gly Pro Gly Gln Gln Gly Pro Gly Gln Gln Gly
        35                  40                  45

Pro Gly Ser Ser Ala Ala Ala Ala Ala Gly Pro Gly Gln Tyr Val Leu
    50                  55                  60

Ile Gly Pro Gly Gln Gln Val Leu Ile Gly Pro Ser Ala Ser Ala Ala
65                  70                  75                  80

Ala Ala Ala Gly Pro Gly Ser Gly Gln Gln Gly Pro Gly Ala Ser Gly
                85                  90                  95

Gln Tyr Gly Pro Gly Gln Gln Gly Pro Gly Gln Gln Gly Pro Gly Ser
            100                 105                 110

Ser Ala Ala Ala Ala Ala Gly Ser Tyr Gly Ser Val Leu Ile Gly Pro
        115                 120                 125

Gly Gln Gln Val Leu Ile Gly Pro Tyr Gly Ser Ala Ala Ala Ala Ala
    130                 135                 140

Gly Pro Gly Ser Gly Gln Tyr Gly Gln Gly Pro Tyr Gly Pro Gly Ala
145                 150                 155                 160

Ser Gly Pro Gly Gln Tyr Gly Pro Gly Gln Gln Gly Pro Ser Ala Ser
                165                 170                 175

Ala Ala Ala Ala Ala Gly Ser Gly Gln Gln Val Leu Ile Gly Pro Gly
            180                 185                 190

Gln Tyr Val Leu Ile Gly Pro Tyr Ala Ser Ala Ala Ala Ala Ala Gly
        195                 200                 205

Gln Tyr Gly Ser Gly Pro Gly Gln Gln Gly Pro Tyr Gly Pro Gly Gln
    210                 215                 220

Ser Gly Ser Gly Gln Gln Gly Pro Gly Gln Gln Gly Pro Tyr Ala Ser
225                 230                 235                 240

Ala Ala Ala Ala Ala Gly Pro Gly Gln Gln Val Leu Ile Gly Pro Tyr
                245                 250                 255

Val Leu Ile Gly Pro Gly Ser Ser Ala Ala Ala Ala Ala Gly Gln Tyr
            260                 265                 270

Gly Tyr Gly Pro Gly Gln Gln Gly Pro Tyr Gly Pro Gly Ala Ser Gly
        275                 280                 285

Gln Asn Gly Pro Gly Ser Gly Gln Tyr Gly Pro Gly Gln Gln Gly Pro
    290                 295                 300

Gly Gln Ser Ala Ala Ala Ala Ala Gly Pro Gly Gln Gln Val Leu Ile
305                 310                 315                 320

Gly Pro Tyr Val Leu Ile Gly Pro Gly Ala Ser Ala Ala Ala Ala Ala
                325                 330                 335

Gly Gln Tyr Gly Pro Gly Gln Gln Gly Pro Gly Gln Tyr Gly Pro Gly
            340                 345                 350

Ser Ser Gly Pro Gly Gln Gln Gly Pro Tyr Gly Pro Gly Ser Ser Ala
        355                 360                 365

Ala Ala Ala Ala Gly Ser Tyr Gly Pro Gly Gln Gln Val Leu Ile Gly
    370                 375                 380

Pro Tyr Val Leu Ile Gly Pro Gly Pro Ser Ala Ala Ala Ala Ala Gly
385                 390                 395                 400

Gln Tyr Gln Gln Gly Pro Gly Gln Gln Gly Pro Tyr Gly Pro Gly Ala
                405                 410                 415

Ser Gly Pro Gly Gln Gln Gly Pro Tyr Gly Pro Gly Ala Ser Ala Ala
            420                 425                 430

Ala Ala Ala Gly Pro Gly Gln Tyr Val Leu Ile Gly Pro Gly Gln Gln
        435                 440                 445

Val Leu Ile Gly Pro Ser Ala Ser Ala Ala Ala Ala Ala Gly Gln Tyr
    450                 455                 460

Gly Ser Gly Pro Gly Gln Tyr Gly Pro Tyr Gly Pro Gly Gln Ser Gly
465                 470                 475                 480

Pro Gly Ser Gly Gln Gln Gly Gln Gly Pro Tyr Gly Pro Gly Ala Ser
                485                 490                 495

Ala Ala Ala Ala Ala Gly Ser Tyr Gly Pro Gly Gln Gln Val Leu Ile
            500                 505                 510

Gly Pro Tyr Val Leu Ile Gly Pro Gly Pro Ser Ala Ala Ala Ala Ala
        515                 520                 525

Gly Pro Gly Ser Gly Gln Tyr Gly Pro Gly Ala Ser Gly Gln Asn Gly
    530                 535                 540

Pro Gly Ser Gly Gln Tyr Gly Pro Gly Gln Gln Gly Pro Gly Gln Ser
545                 550                 555                 560

Ala Ala Ala Ala Ala Gly Gln Tyr Gln Gln Val Leu Ile Gly Pro Gly

565                 570                 575

Gln Gln Gly Pro Tyr Val Leu Ile Gly Pro Gly Ala Ser Ala Ala Ala
            580                 585                 590

Ala Ala Gly Pro Gly Ser Gly Gln Gln Val Leu Ile Gly Pro Gly Ala
        595                 600                 605

Ser Val Leu Ile
    610

<210>  20
<211>  592
<212>  PRT
<213>  Artificial Sequence

<220>
<223>  Met-PRT665

<400>  20
   Met Gly Pro Gly Gln Gln Gly Pro Tyr Gly Pro Gly Ala Ser Ala Ala
   1               5                   10                  15
   Ala Ala Ala Ala Ala Gly Ser Asn Gly Pro Gly Ser Gly Gln Gln Gly
               20                  25                  30
   Pro Gly Gln Ser Gly Gln Tyr Gly Pro Gly Gln Gln Gly Pro Gly Gln
           35                  40                  45
   Gln Gly Pro Gly Ser Ser Ala Ala Ala Ala Ala Ala Ala Gly Pro Gly
       50                  55                  60
   Gln Tyr Val Leu Ile Gly Pro Gly Gln Gln Gly Pro Ser Ala Ser Ala
   65                  70                  75                  80
   Ala Ala Ala Ala Ala Ala Gly Pro Gly Ser Gly Gln Gln Gly Pro Gly
                   85                  90                  95
   Ala Ser Gly Gln Tyr Gly Pro Gly Gln Gln Gly Pro Gly Gln Gln Gly
               100                 105                 110
   Pro Gly Ser Ser Ala Ala Ala Ala Ala Ala Ala Gly Ser Tyr Gly Ser
           115                 120                 125
   Val Leu Ile Gly Pro Gly Gln Gln Gly Pro Tyr Gly Ser Ala Ala Ala
       130                 135                 140
   Ala Ala Ala Ala Gly Pro Gly Ser Gly Gln Tyr Gly Gln Gly Pro Tyr
   145                 150                 155                 160
   Gly Pro Gly Ala Ser Gly Pro Gly Gln Tyr Gly Pro Gly Gln Gln Gly
                   165                 170                 175
   Pro Ser Ala Ser Ala Ala Ala Ala Ala Ala Ala Gly Ser Gly Gln Gln
               180                 185                 190
   Val Leu Ile Gly Pro Gly Gln Tyr Gly Pro Tyr Ala Ser Ala Ala Ala
           195                 200                 205
   Ala Ala Ala Ala Gly Ser Tyr Gly Ser Gly Pro Gly Gln Gln Gly Pro
       210                 215                 220
   Tyr Gly Pro Gly Gln Ser Gly Ser Gly Gln Gln Gly Pro Gly Gln Gln
   225                 230                 235                 240
   Gly Pro Tyr Ala Ser Ala Ala Ala Ala Ala Ala Ala Gly Pro Gly Gln
                   245                 250                 255
   Gln Val Leu Ile Gly Pro Tyr Gly Pro Gly Ser Ser Ala Ala Ala Ala
               260                 265                 270
   Ala Ala Ala Gly Ser Tyr Gly Tyr Gly Pro Gly Gln Gln Gly Pro Tyr
           275                 280                 285
   Gly Pro Gly Ala Ser Gly Gln Asn Gly Pro Gly Ser Gly Gln Tyr Gly
       290                 295                 300
   Pro Gly Gln Gln Gly Pro Gly Pro Ser Ala Ala Ala Ala Ala Ala Ala
   305                 310                 315                 320
   Gly Pro Gly Gln Gln Val Leu Ile Gly Pro Tyr Gly Pro Gly Ala Ser
                   325                 330                 335
   Ala Ala Ala Ala Ala Ala Ala Gly Ser Tyr Gly Pro Gly Gln Gln Gly
               340                 345                 350
   Pro Gly Gln Tyr Gly Pro Gly Ser Ser Gly Pro Gly Gln Gln Gly Pro
           355                 360                 365
   Tyr Gly Pro Gly Ser Ser Ala Ala Ala Ala Ala Ala Ala Gly Ser Tyr
       370                 375                 380
   Gly Pro Gly Gln Gln Val Leu Ile Gly Pro Tyr Gly Pro Gly Pro Ser
   385                 390                 395                 400
   Ala Ala Ala Ala Ala Ala Ala Gly Ser Tyr Gln Gln Gly Pro Gly Gln
   405 410 415
   Gln Gly Pro Tyr Gly Pro Gly Ala Ser Gly Pro Gly Gln Gln Gly Pro
   420 425 430
   Tyr Gly Pro Gly Ala Ser Ala Ala Ala Ala Ala Ala Ala Gly Pro Gly
   435 440 445
   Gln Tyr Val Leu Ile Gly Pro Gly Gln Gln Gly Pro Ser Ala Ser Ala
   450 455 460
   Ala Ala Ala Ala Ala Ala Gly Ser Tyr Gly Ser Gly Pro Gly Gln Tyr
   465 470 475 480
   Gly Pro Tyr Gly Pro Gly Gln Ser Gly Pro Gly Ser Gly Gln Gln Gly
   485 490 495
   Gln Gly Pro Tyr Gly Pro Gly Ala Ser Ala Ala Ala Ala Ala Ala Ala
   500 505 510
   Gly Ser Tyr Gly Pro Gly Gln Gln Val Leu Ile Gly Pro Tyr Gly Pro
   515 520 525
   Gly Pro Ser Ala Ala Ala Ala Ala Ala Ala Gly Pro Gly Ser Gly Gln
   530 535 540
   Tyr Gly Pro Gly Ala Ser Gly Gln Asn Gly Pro Gly Ser Gly Gln Tyr
   545 550 555 560
   Gly Pro Gly Gln Gln Gly Pro Gly Pro Ser Ala Ala Ala Ala Ala Ala
   565 570 575
   Ala Gly Pro Gly Ser Gly Gln Gln Gly Pro Gly Ala Ser Val Leu Ile
   580 585 590

<210>  21
<211>  619
<212>  PRT
<213>  Artificial Sequence

<220>
<223>  Met-PRT666

<400>  21
   Met Gly Pro Gly Gln Gln Gly Pro Tyr Gly Pro Gly Ala Ser Ala Ala
   1 5 10 15
   Ala Ala Ala Ala Ala Gly Ser Asn Gly Pro Gly Ser Gly Gln Gln Gly
   20 25 30
   Pro Gly Gln Ser Gly Gln Tyr Gly Pro Gly Gln Gln Gly Pro Gly Gln
           35                  40                  45
   Gln Gly Pro Gly Ser Ser Ala Ala Ala Ala Ala Ala Ala Gly Pro Gly
       50                  55                  60
   Gln Tyr Val Leu Ile Gly Pro Gly Gln Gln Val Leu Ile Gly Pro Ser
   65                  70                  75                  80
   Ala Ser Ala Ala Ala Ala Ala Ala Ala Gly Pro Gly Ser Gly Gln Gln
                   85                  90                  95
   Gly Pro Gly Ala Ser Gly Gln Tyr Gly Pro Gly Gln Gln Gly Pro Gly
               100                 105                 110
   Gln Gln Gly Pro Gly Ser Ser Ala Ala Ala Ala Ala Ala Ala Gly Ser
           115                 120                 125
   Tyr Gly Ser Val Leu Ile Gly Pro Gly Gln Gln Val Leu Ile Gly Pro
       130                 135                 140
   Tyr Gly Ser Ala Ala Ala Ala Ala Ala Ala Gly Pro Gly Ser Gly Gln
   145                 150                 155                 160
   Tyr Gly Gln Gly Pro Tyr Gly Pro Gly Ala Ser Gly Pro Gly Gln Tyr
                   165                 170                 175
   Gly Pro Gly Gln Gln Gly Pro Ser Ala Ser Ala Ala Ala Ala Ala Ala
               180                 185                 190
   Ala Gly Ser Gly Gln Gln Val Leu Ile Gly Pro Gly Gln Tyr Val Leu
           195                 200                 205
   Ile Gly Pro Tyr Ala Ser Ala Ala Ala Ala Ala Ala Ala Gly Ser Tyr
       210                 215                 220
   Gly Ser Gly Pro Gly Gln Gln Gly Pro Tyr Gly Pro Gly Gln Ser Gly
   225                 230                 235                 240
   Ser Gly Gln Gln Gly Pro Gly Gln Gln Gly Pro Tyr Ala Ser Ala Ala
                   245                 250                 255
   Ala Ala Ala Ala Ala Gly Pro Gly Gln Gln Val Leu Ile Gly Pro Tyr
               260                 265                 270
   Val Leu Ile Gly Pro Gly Ser Ser Ala Ala Ala Ala Ala Ala Ala Gly
   275 280 285
   Ser Tyr Gly Tyr Gly Pro Gly Gln Gln Gly Pro Tyr Gly Pro Gly Ala
   290 295 300
   Ser Gly Gln Asn Gly Pro Gly Ser Gly Gln Tyr Gly Pro Gly Gln Gln
   305 310 315 320
   Gly Pro Gly Pro Ser Ala Ala Ala Ala Ala Ala Ala Gly Pro Gly Gln
   325 330 335
   Gln Val Leu Ile Gly Pro Tyr Val Leu Ile Gly Pro Gly Ala Ser Ala
   340 345 350
   Ala Ala Ala Ala Ala Ala Gly Ser Tyr Gly Pro Gly Gln Gln Gly Pro
   355 360 365
   Gly Gln Tyr Gly Pro Gly Ser Ser Gly Pro Gly Gln Gln Gly Pro Tyr
   370 375 380
   Gly Pro Gly Ser Ser Ala Ala Ala Ala Ala Ala Ala Gly Ser Tyr Gly
   385 390 395 400
   Pro Gly Gln Gln Val Leu Ile Gly Pro Tyr Val Leu Ile Gly Pro Gly
   405 410 415
   Pro Ser Ala Ala Ala Ala Ala Ala Ala Gly Ser Tyr Gln Gln Gly Pro
   420 425 430
   Gly Gln Gln Gly Pro Tyr Gly Pro Gly Ala Ser Gly Pro Gly Gln Gln
   435 440 445
   Gly Pro Tyr Gly Pro Gly Ala Ser Ala Ala Ala Ala Ala Ala Ala Gly
   450 455 460
   Pro Gly Gln Tyr Val Leu Ile Gly Pro Gly Gln Gln Val Leu Ile Gly
   465 470 475 480
   Pro Ser Ala Ser Ala Ala Ala Ala Ala Ala Ala Gly Ser Tyr Gly Ser
   485 490 495
   Gly Pro Gly Gln Tyr Gly Pro Tyr Gly Pro Gly Gln Ser Gly Pro Gly
   500 505 510
   Ser Gly Gln Gln Gly Gln Gly Pro Tyr Gly Pro Gly Ala Ser Ala Ala
   515 520 525
   Ala Ala Ala Ala Ala Gly Ser Tyr Gly Pro Gly Gln Gln Val Leu Ile
       530                 535                 540
   Gly Pro Tyr Val Leu Ile Gly Pro Gly Pro Ser Ala Ala Ala Ala Ala
   545                 550                 555                 560
   Ala Ala Gly Pro Gly Ser Gly Gln Tyr Gly Pro Gly Ala Ser Gly Gln
                   565                 570                 575
   Asn Gly Pro Gly Ser Gly Gln Tyr Gly Pro Gly Gln Gln Gly Pro Gly
               580                 585                 590
   Pro Ser Ala Ala Ala Ala Ala Ala Ala Gly Pro Gly Ser Gly Gln Gln
           595                 600                 605
   Val Leu Ile Gly Pro Gly Ala Ser Val Leu Ile
       610                 615

<210>  22
<211>  590
<212>  PRT
<213>  Artificial Sequence

<220>
<223>  Met-PRT410

<400>  22

Met Gly Pro Gly Gln Gln Gly Pro Tyr Gly Pro Gly Ala Ser Ala Ala
1               5                   10                  15

Ala Ala Ala Gly Gln Asn Gly Pro Gly Ser Gly Gln Gln Gly Pro Gly
            20                  25                  30

Gln Ser Gly Gln Tyr Gly Pro Gly Gln Gln Gly Pro Gly Gln Gln Gly
        35                  40                  45

Pro Gly Ser Ser Ala Ala Ala Ala Ala Gly Pro Gly Gln Tyr Gly Pro
    50                  55                  60

Gly Gln Gln Gly Pro Ser Ala Ser Ala Ala Ala Ala Ala Gly Pro Gly
65                  70                  75                  80

Ser Gly Gln Gln Gly Pro Gly Ala Ser Gly Gln Tyr Gly Pro Gly Gln
                85                  90                  95

Gln Gly Pro Gly Gln Gln Gly Pro Gly Ser Ser Ala Ala Ala Ala Ala
            100                 105                 110

Gly Gln Tyr Gly Ser Gly Pro Gly Gln Gln Gly Pro Tyr Gly Ser Ala

115                 120                 125

Ala Ala Ala Ala Gly Pro Gly Ser Gly Gln Tyr Gly Gln Gly Pro Tyr
    130                 135                 140

Gly Pro Gly Ala Ser Gly Pro Gly Gln Tyr Gly Pro Gly Gln Gln Gly
145                 150                 155                 160

Pro Ser Ala Ser Ala Ala Ala Ala Ala Gly Ser Gly Gln Gln Gly Pro
                165                 170                 175

Gly Gln Tyr Gly Pro Tyr Ala Ser Ala Ala Ala Ala Ala Gly Gln Tyr
            180                 185                 190

Gly Ser Gly Pro Gly Gln Gln Gly Pro Tyr Gly Pro Gly Gln Ser Gly
        195                 200                 205

Ser Gly Gln Gln Gly Pro Gly Gln Gln Gly Pro Tyr Ala Ser Ala Ala
    210                 215                 220

Ala Ala Ala Gly Pro Gly Gln Gln Gly Pro Tyr Gly Pro Gly Ser Ser
225                 230                 235                 240

Ala Ala Ala Ala Ala Gly Gln Tyr Gly Tyr Gly Pro Gly Gln Gln Gly
                245                 250                 255

Pro Tyr Gly Pro Gly Ala Ser Gly Gln Asn Gly Pro Gly Ser Gly Gln
            260                 265                 270

Tyr Gly Pro Gly Gln Gln Gly Pro Gly Gln Ser Ala Ala Ala Ala Ala
        275                 280                 285

Gly Pro Gly Gln Gln Gly Pro Tyr Gly Pro Gly Ala Ser Ala Ala Ala
    290                 295                 300

Ala Ala Gly Gln Tyr Gly Pro Gly Gln Gln Gly Pro Gly Gln Tyr Gly
305                 310                 315                 320

Pro Gly Ser Ser Gly Pro Gly Gln Gln Gly Pro Tyr Gly Pro Gly Ser
                325                 330                 335

Ser Ala Ala Ala Ala Ala Gly Gln Tyr Gly Pro Gly Gln Gln Gly Pro
            340                 345                 350

Tyr Gly Pro Gly Gln Ser Ala Ala Ala Ala Ala Gly Gln Tyr Gln Gln
        355                 360                 365

Gly Pro Gly Gln Gln Gly Pro Tyr Gly Pro Gly Ala Ser Gly Pro Gly
    370                 375                 380

Gln Gln Gly Pro Tyr Gly Pro Gly Ala Ser Ala Ala Ala Ala Ala Gly
385                 390                 395                 400

Pro Gly Gln Tyr Gly Pro Gly Gln Gln Gly Pro Ser Ala Ser Ala Ala
                405                 410                 415

Ala Ala Ala Gly Gln Tyr Gly Ser Gly Pro Gly Gln Tyr Gly Pro Tyr
            420                 425                 430

Gly Pro Gly Gln Ser Gly Pro Gly Ser Gly Gln Gln Gly Gln Gly Pro
        435                 440                 445

Tyr Gly Pro Gly Ala Ser Ala Ala Ala Ala Ala Gly Gln Tyr Gly Pro
    450                 455                 460

Gly Gln Gln Gly Pro Tyr Gly Pro Gly Gln Ser Ala Ala Ala Ala Ala
465                 470                 475                 480

Gly Pro Gly Ser Gly Gln Tyr Gly Pro Gly Ala Ser Gly Gln Asn Gly
                485                 490                 495

Pro Gly Ser Gly Gln Tyr Gly Pro Gly Gln Gln Gly Pro Gly Gln Ser
            500                 505                 510

Ala Ala Ala Ala Ala Gly Gln Tyr Gln Gln Gly Pro Gly Gln Gln Gly
        515                 520                 525

Pro Tyr Gly Pro Gly Ala Ser Ala Ala Ala Ala Ala Gly Gln Tyr Gly
    530                 535                 540

Ser Gly Pro Gly Gln Gln Gly Pro Tyr Gly Pro Gly Gln Ser Gly Ser
545                 550                 555                 560

Gly Gln Gln Gly Pro Gly Gln Gln Gly Pro Tyr Ala Ser Ala Ala Ala
                565                 570                 575

Ala Ala Gly Pro Gly Ser Gly Gln Gln Gly Pro Gly Ala Ser
            580                 585                 590

<210>  23
<211>  565
<212>  PRT
<213>  Artificial Sequence

<220>
<223>  Met-PRT468

<400>  23

Met Gly Pro Gly Gln Gln Gly Pro Tyr Gly Pro Gly Ala Ser Ala Ala
1               5                   10                  15

Ala Ala Ala Ala Ala Gly Ser Asn Gly Pro Gly Ser Gly Gln Gln Gly
            20                  25                  30

Pro Gly Gln Ser Gly Gln Tyr Gly Pro Gly Gln Gln Gly Pro Gly Gln
        35                  40                  45

Gln Gly Pro Gly Ser Ser Ala Ala Ala Ala Ala Ala Ala Gly Pro Gly
    50                  55                  60

Gln Tyr Gly Pro Gly Gln Gln Gly Pro Ser Ala Ser Ala Ala Ala Ala
65                  70                  75                  80

Ala Ala Ala Gly Pro Gly Ser Gly Gln Gln Gly Pro Gly Ala Ser Gly
                85                  90                  95

Gln Tyr Gly Pro Gly Gln Gln Gly Pro Gly Gln Gln Gly Pro Gly Ser
            100                 105                 110

Ser Ala Ala Ala Ala Ala Ala Ala Gly Ser Tyr Gly Ser Gly Pro Gly
        115                 120                 125

Gln Gln Gly Pro Tyr Gly Ser Ala Ala Ala Ala Ala Ala Ala Gly Pro
    130                 135                 140

Gly Ser Gly Gln Tyr Gly Gln Gly Pro Tyr Gly Pro Gly Ala Ser Gly
145                 150                 155                 160

Pro Gly Gln Tyr Gly Pro Gly Gln Gln Gly Pro Ser Ala Ser Ala Ala
                165                 170                 175

Ala Ala Ala Ala Ala Gly Ser Gly Gln Gln Gly Pro Gly Gln Tyr Gly
            180                 185                 190

Pro Tyr Ala Ser Ala Ala Ala Ala Ala Ala Ala Gly Ser Tyr Gly Ser
        195                 200                 205

Gly Pro Gly Gln Gln Gly Pro Tyr Gly Pro Gly Gln Ser Gly Ser Gly
    210                 215                 220

Gln Gln Gly Pro Gly Gln Gln Gly Pro Tyr Ala Ser Ala Ala Ala Ala
225                 230                 235                 240

Ala Ala Ala Gly Pro Gly Gln Gln Gly Pro Tyr Gly Pro Gly Ser Ser
                245                 250                 255

Ala Ala Ala Ala Ala Ala Ala Gly Ser Tyr Gly Tyr Gly Pro Gly Gln
            260                 265                 270

Gln Gly Pro Tyr Gly Pro Gly Ala Ser Gly Gln Asn Gly Pro Gly Ser
        275                 280                 285

Gly Gln Tyr Gly Pro Gly Gln Gln Gly Pro Gly Pro Ser Ala Ala Ala
    290                 295                 300

Ala Ala Ala Ala Gly Pro Gly Gln Gln Gly Pro Tyr Gly Pro Gly Ala
305                 310                 315                 320

Ser Ala Ala Ala Ala Ala Ala Ala Gly Ser Tyr Gly Pro Gly Gln Gln
                325                 330                 335

Gly Pro Gly Gln Tyr Gly Pro Gly Ser Ser Gly Pro Gly Gln Gln Gly
            340                 345                 350

Pro Tyr Gly Pro Gly Ser Ser Ala Ala Ala Ala Ala Ala Ala Gly Ser
        355                 360                 365

Tyr Gly Pro Gly Gln Gln Gly Pro Tyr Gly Pro Gly Pro Ser Ala Ala
    370                 375                 380

Ala Ala Ala Ala Ala Gly Ser Tyr Gln Gln Gly Pro Gly Gln Gln Gly
385                 390                 395                 400

Pro Tyr Gly Pro Gly Ala Ser Gly Pro Gly Gln Gln Gly Pro Tyr Gly
                405                 410                 415

Pro Gly Ala Ser Ala Ala Ala Ala Ala Ala Ala Gly Pro Gly Gln Tyr
            420                 425                 430

Gly Pro Gly Gln Gln Gly Pro Ser Ala Ser Ala Ala Ala Ala Ala Ala
        435                 440                 445

Ala Gly Ser Tyr Gly Ser Gly Pro Gly Gln Tyr Gly Pro Tyr Gly Pro
    450                 455                 460

Gly Gln Ser Gly Pro Gly Ser Gly Gln Gln Gly Gln Gly Pro Tyr Gly
465                 470                 475                 480

Pro Gly Ala Ser Ala Ala Ala Ala Ala Ala Ala Gly Ser Tyr Gly Pro
                485                 490                 495

Gly Gln Gln Gly Pro Tyr Gly Pro Gly Pro Ser Ala Ala Ala Ala Ala
                500                 505                 510

Ala Ala Gly Pro Gly Ser Gly Gln Tyr Gly Pro Gly Ala Ser Gly Gln
            515                 520                 525

Asn Gly Pro Gly Ser Gly Gln Tyr Gly Pro Gly Gln Gln Gly Pro Gly
    530                 535                 540

Pro Ser Ala Ala Ala Ala Ala Ala Ala Gly Pro Gly Ser Gly Gln Gln
545                 550                 555                 560

Gly Pro Gly Ala Ser
                565

<210>  24
<211>  623
<212>  PRT
<213>  Artificial Sequence

<220>
<223>  PRT468

<400>  24

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Gly Gln
            20                  25                  30

Asn Gly Pro Gly Ser Gly Gln Gln Gly Pro Gly Gln Ser Gly Gln Tyr
        35                  40                  45

Gly Pro Gly Gln Gln Gly Pro Gly Gln Gln Gly Pro Gly Ser Ser Ala
    50                  55                  60

Ala Ala Ala Ala Gly Pro Gly Gln Tyr Val Leu Ile Gly Pro Gly Gln
65                  70                  75                  80

Gln Val Leu Ile Gly Pro Ser Ala Ser Ala Ala Ala Ala Ala Gly Pro
                85                  90                  95

Gly Ser Gly Gln Gln Gly Pro Gly Ala Ser Gly Gln Tyr Gly Pro Gly
            100                 105                 110

Gln Gln Gly Pro Gly Gln Gln Gly Pro Gly Ser Ser Ala Ala Ala Ala
        115                 120                 125

Ala Gly Ser Tyr Gly Ser Val Leu Ile Gly Pro Gly Gln Gln Val Leu
    130                 135                 140

Ile Gly Pro Tyr Gly Ser Ala Ala Ala Ala Ala Gly Pro Gly Ser Gly
145                 150                 155                 160

Gln Tyr Gly Gln Gly Pro Tyr Gly Pro Gly Ala Ser Gly Pro Gly Gln
                165                 170                 175

Tyr Gly Pro Gly Gln Gln Gly Pro Ser Ala Ser Ala Ala Ala Ala Ala
            180                 185                 190

Gly Ser Gly Gln Gln Val Leu Ile Gly Pro Gly Gln Tyr Val Leu Ile
        195                 200                 205

Gly Pro Tyr Ala Ser Ala Ala Ala Ala Ala Gly Gln Tyr Gly Ser Gly
    210                 215                 220

Pro Gly Gln Gln Gly Pro Tyr Gly Pro Gly Gln Ser Gly Ser Gly Gln
225                 230                 235                 240

Gln Gly Pro Gly Gln Gln Gly Pro Tyr Ala Ser Ala Ala Ala Ala Ala
                245                 250                 255

Gly Pro Gly Gln Gln Val Leu Ile Gly Pro Tyr Val Leu Ile Gly Pro
            260                 265                 270

Gly Ser Ser Ala Ala Ala Ala Ala Gly Gln Tyr Gly Tyr Gly Pro Gly
        275                 280                 285

Gln Gln Gly Pro Tyr Gly Pro Gly Ala Ser Gly Gln Asn Gly Pro Gly
    290                 295                 300

Ser Gly Gln Tyr Gly Pro Gly Gln Gln Gly Pro Gly Gln Ser Ala Ala
305                 310                 315                 320

Ala Ala Ala Gly Pro Gly Gln Gln Val Leu Ile Gly Pro Tyr Val Leu
                325                 330                 335

Ile Gly Pro Gly Ala Ser Ala Ala Ala Ala Ala Gly Gln Tyr Gly Pro
            340                 345                 350

Gly Gln Gln Gly Pro Gly Gln Tyr Gly Pro Gly Ser Ser Gly Pro Gly
        355                 360                 365

Gln Gln Gly Pro Tyr Gly Pro Gly Ser Ser Ala Ala Ala Ala Ala Gly
    370                 375                 380

Ser Tyr Gly Pro Gly Gln Gln Val Leu Ile Gly Pro Tyr Val Leu Ile
385                 390                 395                 400

Gly Pro Gly Pro Ser Ala Ala Ala Ala Ala Gly Gln Tyr Gln Gln Gly
                405                 410                 415

Pro Gly Gln Gln Gly Pro Tyr Gly Pro Gly Ala Ser Gly Pro Gly Gln
            420                 425                 430

Gln Gly Pro Tyr Gly Pro Gly Ala Ser Ala Ala Ala Ala Ala Gly Pro
        435                 440                 445

Gly Gln Tyr Val Leu Ile Gly Pro Gly Gln Gln Val Leu Ile Gly Pro
    450                 455                 460

Ser Ala Ser Ala Ala Ala Ala Ala Gly Gln Tyr Gly Ser Gly Pro Gly
465                 470                 475                 480

Gln Tyr Gly Pro Tyr Gly Pro Gly Gln Ser Gly Pro Gly Ser Gly Gln
                485                 490                 495

Gln Gly Gln Gly Pro Tyr Gly Pro Gly Ala Ser Ala Ala Ala Ala Ala
            500                 505                 510

Gly Ser Tyr Gly Pro Gly Gln Gln Val Leu Ile Gly Pro Tyr Val Leu
        515                 520                 525

Ile Gly Pro Gly Pro Ser Ala Ala Ala Ala Ala Gly Pro Gly Ser Gly
    530                 535                 540

Gln Tyr Gly Pro Gly Ala Ser Gly Gln Asn Gly Pro Gly Ser Gly Gln
545                 550                 555                 560

Tyr Gly Pro Gly Gln Gln Gly Pro Gly Gln Ser Ala Ala Ala Ala Ala
                565                 570                 575

Gly Gln Tyr Gln Gln Val Leu Ile Gly Pro Gly Gln Gln Gly Pro Tyr
            580                 585                 590

Val Leu Ile Gly Pro Gly Ala Ser Ala Ala Ala Ala Ala Gly Pro Gly
        595                 600                 605

Ser Gly Gln Gln Val Leu Ile Gly Pro Gly Ala Ser Val Leu Ile
    610                 615                 620

<210>  25
<211>  603

<212>  PRT
<213>  Artificial Sequence

<220>
<223>  PRT665

<400>  25

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Ala Ala
            20                  25                  30

Gly Ser Asn Gly Pro Gly Ser Gly Gln Gln Gly Pro Gly Gln Ser Gly
        35                  40                  45

Gln Tyr Gly Pro Gly Gln Gln Gly Pro Gly Gln Gln Gly Pro Gly Ser
    50                  55                  60

Ser Ala Ala Ala Ala Ala Ala Ala Gly Pro Gly Gln Tyr Val Leu Ile
65                  70                  75                  80

Gly Pro Gly Gln Gln Gly Pro Ser Ala Ser Ala Ala Ala Ala Ala Ala
                85                  90                  95

Ala Gly Pro Gly Ser Gly Gln Gln Gly Pro Gly Ala Ser Gly Gln Tyr
            100                 105                 110

Gly Pro Gly Gln Gln Gly Pro Gly Gln Gln Gly Pro Gly Ser Ser Ala
        115                 120                 125

Ala Ala Ala Ala Ala Ala Gly Ser Tyr Gly Ser Val Leu Ile Gly Pro
    130                 135                 140

Gly Gln Gln Gly Pro Tyr Gly Ser Ala Ala Ala Ala Ala Ala Ala Gly
145                 150                 155                 160

Pro Gly Ser Gly Gln Tyr Gly Gln Gly Pro Tyr Gly Pro Gly Ala Ser
                165                 170                 175

Gly Pro Gly Gln Tyr Gly Pro Gly Gln Gln Gly Pro Ser Ala Ser Ala
            180                 185                 190

Ala Ala Ala Ala Ala Ala Gly Ser Gly Gln Gln Val Leu Ile Gly Pro
        195                 200                 205

Gly Gln Tyr Gly Pro Tyr Ala Ser Ala Ala Ala Ala Ala Ala Ala Gly
    210                 215                 220

Ser Tyr Gly Ser Gly Pro Gly Gln Gln Gly Pro Tyr Gly Pro Gly Gln
225                 230                 235                 240

Ser Gly Ser Gly Gln Gln Gly Pro Gly Gln Gln Gly Pro Tyr Ala Ser
                245                 250                 255

Ala Ala Ala Ala Ala Ala Ala Gly Pro Gly Gln Gln Val Leu Ile Gly
            260                 265                 270

Pro Tyr Gly Pro Gly Ser Ser Ala Ala Ala Ala Ala Ala Ala Gly Ser
        275                 280                 285

Tyr Gly Tyr Gly Pro Gly Gln Gln Gly Pro Tyr Gly Pro Gly Ala Ser
    290                 295                 300

Gly Gln Asn Gly Pro Gly Ser Gly Gln Tyr Gly Pro Gly Gln Gln Gly
305                 310                 315                 320

Pro Gly Pro Ser Ala Ala Ala Ala Ala Ala Ala Gly Pro Gly Gln Gln
                325                 330                 335

Val Leu Ile Gly Pro Tyr Gly Pro Gly Ala Ser Ala Ala Ala Ala Ala
            340                 345                 350

Ala Ala Gly Ser Tyr Gly Pro Gly Gln Gln Gly Pro Gly Gln Tyr Gly
        355                 360                 365

Pro Gly Ser Ser Gly Pro Gly Gln Gln Gly Pro Tyr Gly Pro Gly Ser
    370                 375                 380

Ser Ala Ala Ala Ala Ala Ala Ala Gly Ser Tyr Gly Pro Gly Gln Gln
385                 390                 395                 400

Val Leu Ile Gly Pro Tyr Gly Pro Gly Pro Ser Ala Ala Ala Ala Ala
                405                 410                 415

Ala Ala Gly Ser Tyr Gln Gln Gly Pro Gly Gln Gln Gly Pro Tyr Gly
            420                 425                 430

Pro Gly Ala Ser Gly Pro Gly Gln Gln Gly Pro Tyr Gly Pro Gly Ala
        435                 440                 445

Ser Ala Ala Ala Ala Ala Ala Ala Gly Pro Gly Gln Tyr Val Leu Ile
    450                 455                 460

Gly Pro Gly Gln Gln Gly Pro Ser Ala Ser Ala Ala Ala Ala Ala Ala
465                 470                 475                 480

Ala Gly Ser Tyr Gly Ser Gly Pro Gly Gln Tyr Gly Pro Tyr Gly Pro
                485                 490                 495

Gly Gln Ser Gly Pro Gly Ser Gly Gln Gln Gly Gln Gly Pro Tyr Gly
            500                 505                 510

Pro Gly Ala Ser Ala Ala Ala Ala Ala Ala Ala Gly Ser Tyr Gly Pro
        515                 520                 525

Gly Gln Gln Val Leu Ile Gly Pro Tyr Gly Pro Gly Pro Ser Ala Ala
    530                 535                 540

Ala Ala Ala Ala Ala Gly Pro Gly Ser Gly Gln Tyr Gly Pro Gly Ala
545                 550                 555                 560

Ser Gly Gln Asn Gly Pro Gly Ser Gly Gln Tyr Gly Pro Gly Gln Gln
                565                 570                 575

Gly Pro Gly Pro Ser Ala Ala Ala Ala Ala Ala Ala Gly Pro Gly Ser
            580                 585                 590

Gly Gln Gln Gly Pro Gly Ala Ser Val Leu Ile
        595                 600

<210>  26
<211>  630
<212>  PRT
<213>  Artificial Sequence

<220>
<223>  PRT666

<400>  26

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Ala Ala
            20                  25                  30

Gly Ser Asn Gly Pro Gly Ser Gly Gln Gln Gly Pro Gly Gln Ser Gly
        35                  40                  45

Gln Tyr Gly Pro Gly Gln Gln Gly Pro Gly Gln Gln Gly Pro Gly Ser
    50                  55                  60

Ser Ala Ala Ala Ala Ala Ala Ala Gly Pro Gly Gln Tyr Val Leu Ile
65                  70                  75                  80

Gly Pro Gly Gln Gln Val Leu Ile Gly Pro Ser Ala Ser Ala Ala Ala
                85                  90                  95

Ala Ala Ala Ala Gly Pro Gly Ser Gly Gln Gln Gly Pro Gly Ala Ser
            100                 105                 110

Gly Gln Tyr Gly Pro Gly Gln Gln Gly Pro Gly Gln Gln Gly Pro Gly
        115                 120                 125

Ser Ser Ala Ala Ala Ala Ala Ala Ala Gly Ser Tyr Gly Ser Val Leu
    130                 135                 140

Ile Gly Pro Gly Gln Gln Val Leu Ile Gly Pro Tyr Gly Ser Ala Ala
145                 150                 155                 160

Ala Ala Ala Ala Ala Gly Pro Gly Ser Gly Gln Tyr Gly Gln Gly Pro
                165                 170                 175

Tyr Gly Pro Gly Ala Ser Gly Pro Gly Gln Tyr Gly Pro Gly Gln Gln
            180                 185                 190

Gly Pro Ser Ala Ser Ala Ala Ala Ala Ala Ala Ala Gly Ser Gly Gln
        195                 200                 205

Gln Val Leu Ile Gly Pro Gly Gln Tyr Val Leu Ile Gly Pro Tyr Ala
    210                 215                 220

Ser Ala Ala Ala Ala Ala Ala Ala Gly Ser Tyr Gly Ser Gly Pro Gly
225                 230                 235                 240

Gln Gln Gly Pro Tyr Gly Pro Gly Gln Ser Gly Ser Gly Gln Gln Gly
                245                 250                 255

Pro Gly Gln Gln Gly Pro Tyr Ala Ser Ala Ala Ala Ala Ala Ala Ala
            260                 265                 270

Gly Pro Gly Gln Gln Val Leu Ile Gly Pro Tyr Val Leu Ile Gly Pro
        275                 280                 285

Gly Ser Ser Ala Ala Ala Ala Ala Ala Ala Gly Ser Tyr Gly Tyr Gly
    290                 295                 300

Pro Gly Gln Gln Gly Pro Tyr Gly Pro Gly Ala Ser Gly Gln Asn Gly
305                 310                 315                 320

Pro Gly Ser Gly Gln Tyr Gly Pro Gly Gln Gln Gly Pro Gly Pro Ser
                325                 330                 335

Ala Ala Ala Ala Ala Ala Ala Gly Pro Gly Gln Gln Val Leu Ile Gly
            340                 345                 350

Pro Tyr Val Leu Ile Gly Pro Gly Ala Ser Ala Ala Ala Ala Ala Ala
        355                 360                 365

Ala Gly Ser Tyr Gly Pro Gly Gln Gln Gly Pro Gly Gln Tyr Gly Pro
    370                 375                 380

Gly Ser Ser Gly Pro Gly Gln Gln Gly Pro Tyr Gly Pro Gly Ser Ser
385                 390                 395                 400

Ala Ala Ala Ala Ala Ala Ala Gly Ser Tyr Gly Pro Gly Gln Gln Val
                405                 410                 415

Leu Ile Gly Pro Tyr Val Leu Ile Gly Pro Gly Pro Ser Ala Ala Ala
            420                 425                 430

Ala Ala Ala Ala Gly Ser Tyr Gln Gln Gly Pro Gly Gln Gln Gly Pro
        435                 440                 445

Tyr Gly Pro Gly Ala Ser Gly Pro Gly Gln Gln Gly Pro Tyr Gly Pro
    450                 455                 460

Gly Ala Ser Ala Ala Ala Ala Ala Ala Ala Gly Pro Gly Gln Tyr Val
465                 470                 475                 480

Leu Ile Gly Pro Gly Gln Gln Val Leu Ile Gly Pro Ser Ala Ser Ala
                485                 490                 495

Ala Ala Ala Ala Ala Ala Gly Ser Tyr Gly Ser Gly Pro Gly Gln Tyr
            500                 505                 510

Gly Pro Tyr Gly Pro Gly Gln Ser Gly Pro Gly Ser Gly Gln Gln Gly
        515                 520                 525

Gln Gly Pro Tyr Gly Pro Gly Ala Ser Ala Ala Ala Ala Ala Ala Ala
    530                 535                 540

Gly Ser Tyr Gly Pro Gly Gln Gln Val Leu Ile Gly Pro Tyr Val Leu
545                 550                 555                 560

Ile Gly Pro Gly Pro Ser Ala Ala Ala Ala Ala Ala Ala Gly Pro Gly
                565                 570                 575

Ser Gly Gln Tyr Gly Pro Gly Ala Ser Gly Gln Asn Gly Pro Gly Ser

580                 585                 590

Gly Gln Tyr Gly Pro Gly Gln Gln Gly Pro Gly Pro Ser Ala Ala Ala
        595                 600                 605

Ala Ala Ala Ala Gly Pro Gly Ser Gly Gln Gln Val Leu Ile Gly Pro
    610                 615                 620

Gly Ala Ser Val Leu Ile
625                 630

<210>  27
<211>  593
<212>  PRT
<213>  Artificial Sequence

<220>
<223>  M_PRT888

<400>  27

Met Gly Ser Ser Gly Pro Gly Val Leu Gly Pro Tyr Gly Pro Gly Ala
1               5                   10                  15

Ser Ala Ala Ala Ala Ala Gly Gln Asn Gly Pro Gly Ser Gly Val Leu
            20                  25                  30

Gly Pro Gly Gln Ser Gly Gln Tyr Gly Pro Gly Val Leu Gly Pro Gly
        35                  40                  45

Val Leu Gly Pro Gly Ser Ser Ala Ala Ala Ala Ala Gly Pro Gly Gln
    50                  55                  60

Tyr Gly Pro Gly Val Leu Gly Pro Ser Ala Ser Ala Ala Ala Ala Ala
65                  70                  75                  80

Gly Pro Gly Ser Gly Val Leu Gly Pro Gly Ala Ser Gly Gln Tyr Gly
                85                  90                  95

Pro Gly Val Leu Gly Pro Gly Val Leu Gly Pro Gly Ser Ser Ala Ala
            100                 105                 110

Ala Ala Ala Gly Gln Tyr Gly Ser Gly Pro Gly Val Leu Gly Pro Tyr
        115                 120                 125

Gly Ser Ala Ala Ala Ala Ala Gly Pro Gly Ser Gly Gln Tyr Gly Gln
    130                 135                 140

Gly Pro Tyr Gly Pro Gly Ala Ser Gly Pro Gly Gln Tyr Gly Pro Gly
145                 150                 155                 160

Val Leu Gly Pro Ser Ala Ser Ala Ala Ala Ala Ala Gly Ser Gly Val
                165                 170                 175

Leu Gly Pro Gly Gln Tyr Gly Pro Tyr Ala Ser Ala Ala Ala Ala Ala
            180                 185                 190

Gly Gln Tyr Gly Ser Gly Pro Gly Val Leu Gly Pro Tyr Gly Pro Gly
        195                 200                 205

Gln Ser Gly Ser Gly Val Leu Gly Pro Gly Val Leu Gly Pro Tyr Ala
    210                 215                 220

Ser Ala Ala Ala Ala Ala Gly Pro Gly Val Leu Gly Pro Tyr Gly Pro
225                 230                 235                 240

Gly Ser Ser Ala Ala Ala Ala Ala Gly Gln Tyr Gly Tyr Gly Pro Gly
                245                 250                 255

Val Leu Gly Pro Tyr Gly Pro Gly Ala Ser Gly Gln Asn Gly Pro Gly
            260                 265                 270

Ser Gly Gln Tyr Gly Pro Gly Val Leu Gly Pro Gly Gln Ser Ala Ala
        275                 280                 285

Ala Ala Ala Gly Pro Gly Val Leu Gly Pro Tyr Gly Pro Gly Ala Ser
    290                 295                 300

Ala Ala Ala Ala Ala Gly Gln Tyr Gly Pro Gly Val Leu Gly Pro Gly
305                 310                 315                 320

Gln Tyr Gly Pro Gly Ser Ser Gly Pro Gly Val Leu Gly Pro Tyr Gly
                325                 330                 335

Pro Gly Ser Ser Ala Ala Ala Ala Ala Gly Gln Tyr Gly Pro Gly Val
            340                 345                 350

Leu Gly Pro Tyr Gly Pro Gly Gln Ser Ala Ala Ala Ala Ala Gly Gln
        355                 360                 365

Tyr Val Leu Gly Pro Gly Val Leu Gly Pro Tyr Gly Pro Gly Ala Ser
    370                 375                 380

Gly Pro Gly Val Leu Gly Pro Tyr Gly Pro Gly Ala Ser Ala Ala Ala
385                 390                 395                 400

Ala Ala Gly Pro Gly Gln Tyr Gly Pro Gly Val Leu Gly Pro Ser Ala

405                 410                 415

Ser Ala Ala Ala Ala Ala Gly Gln Tyr Gly Ser Gly Pro Gly Gln Tyr
            420                 425                 430

Gly Pro Tyr Gly Pro Gly Gln Ser Gly Pro Gly Ser Gly Val Leu Gly
        435                 440                 445

Gln Gly Pro Tyr Gly Pro Gly Ala Ser Ala Ala Ala Ala Ala Gly Gln
    450                 455                 460

Tyr Gly Pro Gly Val Leu Gly Pro Tyr Gly Pro Gly Gln Ser Ala Ala
465                 470                 475                 480

Ala Ala Ala Gly Pro Gly Ser Gly Gln Tyr Gly Pro Gly Ala Ser Gly
                485                 490                 495

Gln Asn Gly Pro Gly Ser Gly Gln Tyr Gly Pro Gly Val Leu Gly Pro
            500                 505                 510

Gly Gln Ser Ala Ala Ala Ala Ala Gly Gln Tyr Val Leu Gly Pro Gly
        515                 520                 525

Val Leu Gly Pro Tyr Gly Pro Gly Ala Ser Ala Ala Ala Ala Ala Gly
    530                 535                 540

Gln Tyr Gly Ser Gly Pro Gly Val Leu Gly Pro Tyr Gly Pro Gly Gln
545                 550                 555                 560

Ser Gly Ser Gly Val Leu Gly Pro Gly Val Leu Gly Pro Tyr Ala Ser
                565                 570                 575

Ala Ala Ala Ala Ala Gly Pro Gly Ser Gly Val Leu Gly Pro Gly Ala
            580                 585                 590

Ser

<210>  28
<211>  590
<212>  PRT
<213>  Artificial Sequence

<220>
<223>  M_PRT965

<400>  28

Met Gly Pro Gly Thr Ser Gly Pro Tyr Gly Pro Gly Ala Ser Ala Ala
1               5                   10                  15

Ala Ala Ala Gly Ala Asn Gly Pro Gly Ser Gly Thr Ser Gly Pro Gly
            20                  25                  30

Ala Ser Gly Ala Tyr Gly Pro Gly Thr Ser Gly Pro Gly Thr Ser Gly
        35                  40                  45

Pro Gly Ser Ser Ala Ala Ala Ala Ala Gly Pro Gly Ala Tyr Gly Pro
    50                  55                  60

Gly Thr Ser Gly Pro Ser Ala Ser Ala Ala Ala Ala Ala Gly Pro Gly
65                  70                  75                  80

Ser Gly Thr Ser Gly Pro Gly Ala Ser Gly Ala Tyr Gly Pro Gly Thr
                85                  90                  95

Ser Gly Pro Gly Thr Ser Gly Pro Gly Ser Ser Ala Ala Ala Ala Ala
            100                 105                 110

Gly Ala Tyr Gly Ser Gly Pro Gly Thr Ser Gly Pro Tyr Gly Ser Ala
        115                 120                 125

Ala Ala Ala Ala Gly Pro Gly Ser Gly Ala Tyr Gly Ala Gly Pro Tyr
    130                 135                 140

Gly Pro Gly Ala Ser Gly Pro Gly Ala Tyr Gly Pro Gly Thr Ser Gly
145                 150                 155                 160

Pro Ser Ala Ser Ala Ala Ala Ala Ala Gly Ser Gly Thr Ser Gly Pro
                165                 170                 175

Gly Ala Tyr Gly Pro Tyr Ala Ser Ala Ala Ala Ala Ala Gly Ala Tyr
            180                 185                 190

Gly Ser Gly Pro Gly Thr Ser Gly Pro Tyr Gly Pro Gly Ala Ser Gly
        195                 200                 205

Ser Gly Thr Ser Gly Pro Gly Thr Ser Gly Pro Tyr Ala Ser Ala Ala
    210                 215                 220

Ala Ala Ala Gly Pro Gly Thr Ser Gly Pro Tyr Gly Pro Gly Ser Ser
225                 230                 235                 240

Ala Ala Ala Ala Ala Gly Ala Tyr Gly Tyr Gly Pro Gly Thr Ser Gly
                245                 250                 255

Pro Tyr Gly Pro Gly Ala Ser Gly Ala Asn Gly Pro Gly Ser Gly Ala

            260                 265                 270

Tyr Gly Pro Gly Thr Ser Gly Pro Gly Ala Ser Ala Ala Ala Ala Ala
        275                 280                 285

Gly Pro Gly Thr Ser Gly Pro Tyr Gly Pro Gly Ala Ser Ala Ala Ala
    290                 295                 300

Ala Ala Gly Ala Tyr Gly Pro Gly Thr Ser Gly Pro Gly Ala Tyr Gly
305                 310                 315                 320

Pro Gly Ser Ser Gly Pro Gly Thr Ser Gly Pro Tyr Gly Pro Gly Ser
                325                 330                 335

Ser Ala Ala Ala Ala Ala Gly Ala Tyr Gly Pro Gly Thr Ser Gly Pro
            340                 345                 350

Tyr Gly Pro Gly Ala Ser Ala Ala Ala Ala Ala Gly Ala Tyr Thr Ser
        355                 360                 365

Gly Pro Gly Thr Ser Gly Pro Tyr Gly Pro Gly Ala Ser Gly Pro Gly
    370                 375                 380

Thr Ser Gly Pro Tyr Gly Pro Gly Ala Ser Ala Ala Ala Ala Ala Gly
385                 390                 395                 400

Pro Gly Ala Tyr Gly Pro Gly Thr Ser Gly Pro Ser Ala Ser Ala Ala
                405                 410                 415

Ala Ala Ala Gly Ala Tyr Gly Ser Gly Pro Gly Ala Tyr Gly Pro Tyr
            420                 425                 430

Gly Pro Gly Ala Ser Gly Pro Gly Ser Gly Thr Ser Gly Ala Gly Pro
        435                 440                 445

Tyr Gly Pro Gly Ala Ser Ala Ala Ala Ala Ala Gly Ala Tyr Gly Pro
    450                 455                 460

Gly Thr Ser Gly Pro Tyr Gly Pro Gly Ala Ser Ala Ala Ala Ala Ala
465                 470                 475                 480

Gly Pro Gly Ser Gly Ala Tyr Gly Pro Gly Ala Ser Gly Ala Asn Gly
                485                 490                 495

Pro Gly Ser Gly Ala Tyr Gly Pro Gly Thr Ser Gly Pro Gly Ala Ser
            500                 505                 510

Ala Ala Ala Ala Ala Gly Ala Tyr Thr Ser Gly Pro Gly Thr Ser Gly
        515                 520                 525

Pro Tyr Gly Pro Gly Ala Ser Ala Ala Ala Ala Ala Gly Ala Tyr Gly
    530                 535                 540

Ser Gly Pro Gly Thr Ser Gly Pro Tyr Gly Pro Gly Ala Ser Gly Ser
545                 550                 555                 560

Gly Thr Ser Gly Pro Gly Thr Ser Gly Pro Tyr Ala Ser Ala Ala Ala
                565                 570                 575

Ala Ala Gly Pro Gly Ser Gly Thr Ser Gly Pro Gly Ala Ser
            580                 585                 590

<210>  29
<211>  593
<212>  PRT
<213>  Artificial Sequence

<220>
<223>  M_PRT889

<400>  29

Met Gly Ser Ser Gly Pro Gly Val Leu Gly Pro Tyr Gly Pro Gly Ala
1               5                   10                  15

Ser Ala Ala Ala Ala Ala Gly Ile Asn Gly Pro Gly Ser Gly Val Leu
            20                  25                  30

Gly Pro Gly Ile Ser Gly Ile Tyr Gly Pro Gly Val Leu Gly Pro Gly
        35                  40                  45

Val Leu Gly Pro Gly Ser Ser Ala Ala Ala Ala Ala Gly Pro Gly Ile
    50                  55                  60

Tyr Gly Pro Gly Val Leu Gly Pro Ser Ala Ser Ala Ala Ala Ala Ala
65                  70                  75                  80

Gly Pro Gly Ser Gly Val Leu Gly Pro Gly Ala Ser Gly Ile Tyr Gly
                85                  90                  95

Pro Gly Val Leu Gly Pro Gly Val Leu Gly Pro Gly Ser Ser Ala Ala
            100                 105                 110

Ala Ala Ala Gly Ile Tyr Gly Ser Gly Pro Gly Val Leu Gly Pro Tyr
        115                 120                 125

Gly Ser Ala Ala Ala Ala Ala Gly Pro Gly Ser Gly Ile Tyr Gly Ile

    130                 135                 140

Gly Pro Tyr Gly Pro Gly Ala Ser Gly Pro Gly Ile Tyr Gly Pro Gly
145                 150                 155                 160

Val Leu Gly Pro Ser Ala Ser Ala Ala Ala Ala Ala Gly Ser Gly Val
                165                 170                 175

Leu Gly Pro Gly Ile Tyr Gly Pro Tyr Ala Ser Ala Ala Ala Ala Ala
            180                 185                 190

Gly Ile Tyr Gly Ser Gly Pro Gly Val Leu Gly Pro Tyr Gly Pro Gly
        195                 200                 205

Ile Ser Gly Ser Gly Val Leu Gly Pro Gly Val Leu Gly Pro Tyr Ala
    210                 215                 220

Ser Ala Ala Ala Ala Ala Gly Pro Gly Val Leu Gly Pro Tyr Gly Pro
225                 230                 235                 240

Gly Ser Ser Ala Ala Ala Ala Ala Gly Ile Tyr Gly Tyr Gly Pro Gly
                245                 250                 255

Val Leu Gly Pro Tyr Gly Pro Gly Ala Ser Gly Ile Asn Gly Pro Gly
            260                 265                 270

Ser Gly Ile Tyr Gly Pro Gly Val Leu Gly Pro Gly Ile Ser Ala Ala
        275                 280                 285

Ala Ala Ala Gly Pro Gly Val Leu Gly Pro Tyr Gly Pro Gly Ala Ser
    290                 295                 300

Ala Ala Ala Ala Ala Gly Ile Tyr Gly Pro Gly Val Leu Gly Pro Gly
305                 310                 315                 320

Ile Tyr Gly Pro Gly Ser Ser Gly Pro Gly Val Leu Gly Pro Tyr Gly
                325                 330                 335

Pro Gly Ser Ser Ala Ala Ala Ala Ala Gly Ile Tyr Gly Pro Gly Val
            340                 345                 350

Leu Gly Pro Tyr Gly Pro Gly Ile Ser Ala Ala Ala Ala Ala Gly Ile
        355                 360                 365

Tyr Val Leu Gly Pro Gly Val Leu Gly Pro Tyr Gly Pro Gly Ala Ser
    370                 375                 380

Gly Pro Gly Val Leu Gly Pro Tyr Gly Pro Gly Ala Ser Ala Ala Ala
385                 390                 395                 400

Ala Ala Gly Pro Gly Ile Tyr Gly Pro Gly Val Leu Gly Pro Ser Ala
                405                 410                 415

Ser Ala Ala Ala Ala Ala Gly Ile Tyr Gly Ser Gly Pro Gly Ile Tyr
            420                 425                 430

Gly Pro Tyr Gly Pro Gly Ile Ser Gly Pro Gly Ser Gly Val Leu Gly
        435                 440                 445

Ile Gly Pro Tyr Gly Pro Gly Ala Ser Ala Ala Ala Ala Ala Gly Ile
    450                 455                 460

Tyr Gly Pro Gly Val Leu Gly Pro Tyr Gly Pro Gly Ile Ser Ala Ala
465                 470                 475                 480

Ala Ala Ala Gly Pro Gly Ser Gly Ile Tyr Gly Pro Gly Ala Ser Gly
                485                 490                 495

Ile Asn Gly Pro Gly Ser Gly Ile Tyr Gly Pro Gly Val Leu Gly Pro
            500                 505                 510

Gly Ile Ser Ala Ala Ala Ala Ala Gly Ile Tyr Val Leu Gly Pro Gly
        515                 520                 525

Val Leu Gly Pro Tyr Gly Pro Gly Ala Ser Ala Ala Ala Ala Ala Gly
    530                 535                 540

Ile Tyr Gly Ser Gly Pro Gly Val Leu Gly Pro Tyr Gly Pro Gly Ile
545                 550                 555                 560

Ser Gly Ser Gly Val Leu Gly Pro Gly Val Leu Gly Pro Tyr Ala Ser
                565                 570                 575

Ala Ala Ala Ala Ala Gly Pro Gly Ser Gly Val Leu Gly Pro Gly Ala
            580                 585                 590

Ser

<210>  30
<211>  590
<212>  PRT
<213>  Artificial Sequence

<220>
<223>  M_PRT916

<400>  30

Met Gly Pro Gly Val Ile Gly Pro Tyr Gly Pro Gly Ala Ser Ala Ala
1               5                   10                  15

Ala Ala Ala Gly Leu Asn Gly Pro Gly Ser Gly Val Ile Gly Pro Gly
            20                  25                  30

Leu Ser Gly Leu Tyr Gly Pro Gly Val Ile Gly Pro Gly Val Ile Gly
        35                  40                  45

Pro Gly Ser Ser Ala Ala Ala Ala Ala Gly Pro Gly Leu Tyr Gly Pro
    50                  55                  60

Gly Val Ile Gly Pro Ser Ala Ser Ala Ala Ala Ala Ala Gly Pro Gly
65                  70                  75                  80

Ser Gly Val Ile Gly Pro Gly Ala Ser Gly Leu Tyr Gly Pro Gly Val
                85                  90                  95

Ile Gly Pro Gly Val Ile Gly Pro Gly Ser Ser Ala Ala Ala Ala Ala
            100                 105                 110

Gly Leu Tyr Gly Ser Gly Pro Gly Val Ile Gly Pro Tyr Gly Ser Ala
        115                 120                 125

Ala Ala Ala Ala Gly Pro Gly Ser Gly Leu Tyr Gly Leu Gly Pro Tyr
    130                 135                 140

Gly Pro Gly Ala Ser Gly Pro Gly Leu Tyr Gly Pro Gly Val Ile Gly
145                 150                 155                 160

Pro Ser Ala Ser Ala Ala Ala Ala Ala Gly Ser Gly Val Ile Gly Pro
                165                 170                 175

Gly Leu Tyr Gly Pro Tyr Ala Ser Ala Ala Ala Ala Ala Gly Leu Tyr
            180                 185                 190

Gly Ser Gly Pro Gly Val Ile Gly Pro Tyr Gly Pro Gly Leu Ser Gly
        195                 200                 205

Ser Gly Val Ile Gly Pro Gly Val Ile Gly Pro Tyr Ala Ser Ala Ala
    210                 215                 220

Ala Ala Ala Gly Pro Gly Val Ile Gly Pro Tyr Gly Pro Gly Ser Ser
225                 230                 235                 240

Ala Ala Ala Ala Ala Gly Leu Tyr Gly Tyr Gly Pro Gly Val Ile Gly
                245                 250                 255

Pro Tyr Gly Pro Gly Ala Ser Gly Leu Asn Gly Pro Gly Ser Gly Leu
            260                 265                 270

Tyr Gly Pro Gly Val Ile Gly Pro Gly Leu Ser Ala Ala Ala Ala Ala
        275                 280                 285

Gly Pro Gly Val Ile Gly Pro Tyr Gly Pro Gly Ala Ser Ala Ala Ala
    290                 295                 300

Ala Ala Gly Leu Tyr Gly Pro Gly Val Ile Gly Pro Gly Leu Tyr Gly
305                 310                 315                 320

Pro Gly Ser Ser Gly Pro Gly Val Ile Gly Pro Tyr Gly Pro Gly Ser
                325                 330                 335

Ser Ala Ala Ala Ala Ala Gly Leu Tyr Gly Pro Gly Val Ile Gly Pro
            340                 345                 350

Tyr Gly Pro Gly Leu Ser Ala Ala Ala Ala Ala Gly Leu Tyr Val Ile
        355                 360                 365

Gly Pro Gly Val Ile Gly Pro Tyr Gly Pro Gly Ala Ser Gly Pro Gly
    370                 375                 380

Val Ile Gly Pro Tyr Gly Pro Gly Ala Ser Ala Ala Ala Ala Ala Gly
385                 390                 395                 400

Pro Gly Leu Tyr Gly Pro Gly Val Ile Gly Pro Ser Ala Ser Ala Ala
                405                 410                 415

Ala Ala Ala Gly Leu Tyr Gly Ser Gly Pro Gly Leu Tyr Gly Pro Tyr
            420                 425                 430

Gly Pro Gly Leu Ser Gly Pro Gly Ser Gly Val Ile Gly Leu Gly Pro
        435                 440                 445

Tyr Gly Pro Gly Ala Ser Ala Ala Ala Ala Ala Gly Leu Tyr Gly Pro
    450                 455                 460

Gly Val Ile Gly Pro Tyr Gly Pro Gly Leu Ser Ala Ala Ala Ala Ala
465                 470                 475                 480

Gly Pro Gly Ser Gly Leu Tyr Gly Pro Gly Ala Ser Gly Leu Asn Gly
                485                 490                 495

Pro Gly Ser Gly Leu Tyr Gly Pro Gly Val Ile Gly Pro Gly Leu Ser
            500                 505                 510

Ala Ala Ala Ala Ala Gly Leu Tyr Val Ile Gly Pro Gly Val Ile Gly
        515                 520                 525

Pro Tyr Gly Pro Gly Ala Ser Ala Ala Ala Ala Ala Gly Leu Tyr Gly
    530                 535                 540

Ser Gly Pro Gly Val Ile Gly Pro Tyr Gly Pro Gly Leu Ser Gly Ser
545                 550                 555                 560

Gly Val Ile Gly Pro Gly Val Ile Gly Pro Tyr Ala Ser Ala Ala Ala
                565                 570                 575

Ala Ala Gly Pro Gly Ser Gly Val Ile Gly Pro Gly Ala Ser
            580                 585                 590

<210> 31
<211> 590
<212> PRT
<213> Artificial Sequence

<220>
<223> M_ PRT918

<400> 31

Met Gly Pro Gly Val Phe Gly Pro Tyr Gly Pro Gly Ala Ser Ala Ala
1               5                   10                  15

Ala Ala Ala Gly Ile Asn Gly Pro Gly Ser Gly Val Phe Gly Pro Gly
            20                  25                  30

Ile Ser Gly Ile Tyr Gly Pro Gly Val Phe Gly Pro Gly Val Phe Gly
        35                  40                  45

Pro Gly Ser Ser Ala Ala Ala Ala Ala Gly Pro Gly Ile Tyr Gly Pro
    50                  55                  60

Gly Val Phe Gly Pro Ser Ala Ser Ala Ala Ala Ala Ala Gly Pro Gly
65                  70                  75                  80

Ser Gly Val Phe Gly Pro Gly Ala Ser Gly Ile Tyr Gly Pro Gly Val
                85                  90                  95

Phe Gly Pro Gly Val Phe Gly Pro Gly Ser Ser Ala Ala Ala Ala Ala
            100                 105                 110

Gly Ile Tyr Gly Ser Gly Pro Gly Val Phe Gly Pro Tyr Gly Ser Ala
        115                 120                 125

Ala Ala Ala Ala Gly Pro Gly Ser Gly Ile Tyr Gly Ile Gly Pro Tyr
    130                 135                 140

Gly Pro Gly Ala Ser Gly Pro Gly Ile Tyr Gly Pro Gly Val Phe Gly
145                 150                 155                 160

Pro Ser Ala Ser Ala Ala Ala Ala Ala Gly Ser Gly Val Phe Gly Pro
                165                 170                 175

Gly Ile Tyr Gly Pro Tyr Ala Ser Ala Ala Ala Ala Ala Gly Ile Tyr
            180                 185                 190

Gly Ser Gly Pro Gly Val Phe Gly Pro Tyr Gly Pro Gly Ile Ser Gly
        195                 200                 205

Ser Gly Val Phe Gly Pro Gly Val Phe Gly Pro Tyr Ala Ser Ala Ala
    210                 215                 220

Ala Ala Ala Gly Pro Gly Val Phe Gly Pro Tyr Gly Pro Gly Ser Ser
225                 230                 235                 240

Ala Ala Ala Ala Ala Gly Ile Tyr Gly Tyr Gly Pro Gly Val Phe Gly
                245                 250                 255

Pro Tyr Gly Pro Gly Ala Ser Gly Ile Asn Gly Pro Gly Ser Gly Ile
            260                 265                 270

Tyr Gly Pro Gly Val Phe Gly Pro Gly Ile Ser Ala Ala Ala Ala Ala
        275                 280                 285

Gly Pro Gly Val Phe Gly Pro Tyr Gly Pro Gly Ala Ser Ala Ala Ala
    290                 295                 300

Ala Ala Gly Ile Tyr Gly Pro Gly Val Phe Gly Pro Gly Ile Tyr Gly
305                 310                 315                 320

Pro Gly Ser Ser Gly Pro Gly Val Phe Gly Pro Tyr Gly Pro Gly Ser
                325                 330                 335

Ser Ala Ala Ala Ala Ala Gly Ile Tyr Gly Pro Gly Val Phe Gly Pro
            340                 345                 350

Tyr Gly Pro Gly Ile Ser Ala Ala Ala Ala Ala Gly Ile Tyr Val Phe
        355                 360                 365

Gly Pro Gly Val Phe Gly Pro Tyr Gly Pro Gly Ala Ser Gly Pro Gly
    370                 375                 380

Val Phe Gly Pro Tyr Gly Pro Gly Ala Ser Ala Ala Ala Ala Ala Gly
385                 390                 395                 400

Pro Gly Ile Tyr Gly Pro Gly Val Phe Gly Pro Ser Ala Ser Ala Ala
                405                 410                 415

Ala Ala Ala Gly Ile Tyr Gly Ser Gly Pro Gly Ile Tyr Gly Pro Tyr
            420                 425                 430

Gly Pro Gly Ile Ser Gly Pro Gly Ser Gly Val Phe Gly Ile Gly Pro
        435                 440                 445

Tyr Gly Pro Gly Ala Ser Ala Ala Ala Ala Ala Gly Ile Tyr Gly Pro
    450                 455                 460

Gly Val Phe Gly Pro Tyr Gly Pro Gly Ile Ser Ala Ala Ala Ala Ala
465                 470                 475                 480

Gly Pro Gly Ser Gly Ile Tyr Gly Pro Gly Ala Ser Gly Ile Asn Gly
                485                 490                 495

Pro Gly Ser Gly Ile Tyr Gly Pro Gly Val Phe Gly Pro Gly Ile Ser
            500                 505                 510

Ala Ala Ala Ala Ala Gly Ile Tyr Val Phe Gly Pro Gly Val Phe Gly
        515                 520                 525

Pro Tyr Gly Pro Gly Ala Ser Ala Ala Ala Ala Ala Gly Ile Tyr Gly
    530                 535                 540

Ser Gly Pro Gly Val Phe Gly Pro Tyr Gly Pro Gly Ile Ser Gly Ser
545                 550                 555                 560

Gly Val Phe Gly Pro Gly Val Phe Gly Pro Tyr Ala Ser Ala Ala Ala
                565                 570                 575

Ala Ala Gly Pro Gly Ser Gly Val Phe Gly Pro Gly Ala Ser
            580                 585                 590

<210>  32
<211>  565
<212>  PRT
<213>  Artificial Sequence

<220>

<223>  M_PRT699

<400>  32
   Met Gly Pro Gly Val Leu Gly Pro Tyr Gly Pro Gly Ala Ser Ala Ala
   1               5                   10                  15
   Ala Ala Ala Ala Ala Gly Ser Asn Gly Pro Gly Ser Gly Val Leu Gly
               20                  25                  30
   Pro Gly Gln Ser Gly Gln Tyr Gly Pro Gly Val Leu Gly Pro Gly Val
           35                  40                  45
   Leu Gly Pro Gly Ser Ser Ala Ala Ala Ala Ala Ala Ala Gly Pro Gly
       50                  55                  60
   Gln Tyr Gly Pro Gly Val Leu Gly Pro Ser Ala Ser Ala Ala Ala Ala
   65                  70                  75                  80
   Ala Ala Ala Gly Pro Gly Ser Gly Val Leu Gly Pro Gly Ala Ser Gly
                   85                  90                  95
   Gln Tyr Gly Pro Gly Val Leu Gly Pro Gly Val Leu Gly Pro Gly Ser
               100                 105                 110
   Ser Ala Ala Ala Ala Ala Ala Ala Gly Ser Tyr Gly Ser Gly Pro Gly
           115                 120                 125
   Val Leu Gly Pro Tyr Gly Ser Ala Ala Ala Ala Ala Ala Ala Gly Pro
       130                 135                 140
   Gly Ser Gly Gln Tyr Gly Gln Gly Pro Tyr Gly Pro Gly Ala Ser Gly
   145                 150                 155                 160
   Pro Gly Gln Tyr Gly Pro Gly Val Leu Gly Pro Ser Ala Ser Ala Ala
                   165                 170                 175
   Ala Ala Ala Ala Ala Gly Ser Gly Val Leu Gly Pro Gly Gln Tyr Gly
               180                 185                 190
   Pro Tyr Ala Ser Ala Ala Ala Ala Ala Ala Ala Gly Ser Tyr Gly Ser
           195                 200                 205
   Gly Pro Gly Val Leu Gly Pro Tyr Gly Pro Gly Gln Ser Gly Ser Gly
       210                 215                 220
   Val Leu Gly Pro Gly Val Leu Gly Pro Tyr Ala Ser Ala Ala Ala Ala
   225                 230                 235                 240
   Ala Ala Ala Gly Pro Gly Val Leu Gly Pro Tyr Gly Pro Gly Ser Ser
   245 250 255
   Ala Ala Ala Ala Ala Ala Ala Gly Ser Tyr Gly Tyr Gly Pro Gly Val
   260 265 270
   Leu Gly Pro Tyr Gly Pro Gly Ala Ser Gly Gln Asn Gly Pro Gly Ser
   275 280 285
   Gly Gln Tyr Gly Pro Gly Val Leu Gly Pro Gly Pro Ser Ala Ala Ala
   290 295 300
   Ala Ala Ala Ala Gly Pro Gly Val Leu Gly Pro Tyr Gly Pro Gly Ala
   305 310 315 320
   Ser Ala Ala Ala Ala Ala Ala Ala Gly Ser Tyr Gly Pro Gly Val Leu
   325 330 335
   Gly Pro Gly Gln Tyr Gly Pro Gly Ser Ser Gly Pro Gly Val Leu Gly
   340 345 350
   Pro Tyr Gly Pro Gly Ser Ser Ala Ala Ala Ala Ala Ala Ala Gly Ser
   355 360 365
   Tyr Gly Pro Gly Val Leu Gly Pro Tyr Gly Pro Gly Pro Ser Ala Ala
   370 375 380
   Ala Ala Ala Ala Ala Gly Ser Tyr Val Leu Gly Pro Gly Val Leu Gly
   385 390 395 400
   Pro Tyr Gly Pro Gly Ala Ser Gly Pro Gly Val Leu Gly Pro Tyr Gly
   405 410 415
   Pro Gly Ala Ser Ala Ala Ala Ala Ala Ala Ala Gly Pro Gly Gln Tyr
   420 425 430
   Gly Pro Gly Val Leu Gly Pro Ser Ala Ser Ala Ala Ala Ala Ala Ala
   435 440 445
   Ala Gly Ser Tyr Gly Ser Gly Pro Gly Gln Tyr Gly Pro Tyr Gly Pro
   450 455 460
   Gly Gln Ser Gly Pro Gly Ser Gly Val Leu Gly Gln Gly Pro Tyr Gly
   465 470 475 480
   Pro Gly Ala Ser Ala Ala Ala Ala Ala Ala Ala Gly Ser Tyr Gly Pro
   485 490 495
   Gly Val Leu Gly Pro Tyr Gly Pro Gly Pro Ser Ala Ala Ala Ala Ala
                   500                 505                 510
   Ala Ala Gly Pro Gly Ser Gly Gln Tyr Gly Pro Gly Ala Ser Gly Gln
           515                 520                 525
   Asn Gly Pro Gly Ser Gly Gln Tyr Gly Pro Gly Val Leu Gly Pro Gly
       530                 535                 540
   Pro Ser Ala Ala Ala Ala Ala Ala Ala Gly Pro Gly Ser Gly Val Leu
   545                 550                 555                 560
   Gly Pro Gly Ala Ser
                   565

<210>  33
<211>  565
<212>  PRT
<213>  Artificial Sequence

<220>
<223>  M_PRT698

<400>  33

Met Gly Pro Gly Val Leu Gly Pro Tyr Gly Pro Gly Ala Ser Ala Ala
1               5                   10                  15

Ala Ala Ala Ala Ala Gly Ser Asn Gly Pro Gly Ser Gly Val Leu Gly
            20                  25                  30

Pro Gly Ile Ser Gly Ile Tyr Gly Pro Gly Val Leu Gly Pro Gly Val
        35                  40                  45

Leu Gly Pro Gly Ser Ser Ala Ala Ala Ala Ala Ala Ala Gly Pro Gly
    50                  55                  60

Ile Tyr Gly Pro Gly Val Leu Gly Pro Ser Ala Ser Ala Ala Ala Ala
65                  70                  75                  80

Ala Ala Ala Gly Pro Gly Ser Gly Val Leu Gly Pro Gly Ala Ser Gly
                85                  90                  95

Ile Tyr Gly Pro Gly Val Leu Gly Pro Gly Val Leu Gly Pro Gly Ser
            100                 105                 110

Ser Ala Ala Ala Ala Ala Ala Ala Gly Ser Tyr Gly Ser Gly Pro Gly
        115                 120                 125

Val Leu Gly Pro Tyr Gly Ser Ala Ala Ala Ala Ala Ala Ala Gly Pro
    130                 135                 140

Gly Ser Gly Ile Tyr Gly Ile Gly Pro Tyr Gly Pro Gly Ala Ser Gly
145                 150                 155                 160

Pro Gly Ile Tyr Gly Pro Gly Val Leu Gly Pro Ser Ala Ser Ala Ala
                165                 170                 175

Ala Ala Ala Ala Ala Gly Ser Gly Val Leu Gly Pro Gly Ile Tyr Gly
            180                 185                 190

Pro Tyr Ala Ser Ala Ala Ala Ala Ala Ala Ala Gly Ser Tyr Gly Ser
        195                 200                 205

Gly Pro Gly Val Leu Gly Pro Tyr Gly Pro Gly Ile Ser Gly Ser Gly
    210                 215                 220

Val Leu Gly Pro Gly Val Leu Gly Pro Tyr Ala Ser Ala Ala Ala Ala
225                 230                 235                 240

Ala Ala Ala Gly Pro Gly Val Leu Gly Pro Tyr Gly Pro Gly Ser Ser
                245                 250                 255

Ala Ala Ala Ala Ala Ala Ala Gly Ser Tyr Gly Tyr Gly Pro Gly Val
            260                 265                 270

Leu Gly Pro Tyr Gly Pro Gly Ala Ser Gly Ile Asn Gly Pro Gly Ser
        275                 280                 285

Gly Ile Tyr Gly Pro Gly Val Leu Gly Pro Gly Pro Ser Ala Ala Ala
    290                 295                 300

Ala Ala Ala Ala Gly Pro Gly Val Leu Gly Pro Tyr Gly Pro Gly Ala
305                 310                 315                 320

Ser Ala Ala Ala Ala Ala Ala Ala Gly Ser Tyr Gly Pro Gly Val Leu
                325                 330                 335

Gly Pro Gly Ile Tyr Gly Pro Gly Ser Ser Gly Pro Gly Val Leu Gly
            340                 345                 350

Pro Tyr Gly Pro Gly Ser Ser Ala Ala Ala Ala Ala Ala Ala Gly Ser
        355                 360                 365

Tyr Gly Pro Gly Val Leu Gly Pro Tyr Gly Pro Gly Pro Ser Ala Ala
    370                 375                 380

Ala Ala Ala Ala Ala Gly Ser Tyr Val Leu Gly Pro Gly Val Leu Gly
385                 390                 395                 400

Pro Tyr Gly Pro Gly Ala Ser Gly Pro Gly Val Leu Gly Pro Tyr Gly
                405                 410                 415

Pro Gly Ala Ser Ala Ala Ala Ala Ala Ala Ala Gly Pro Gly Ile Tyr
            420                 425                 430

Gly Pro Gly Val Leu Gly Pro Ser Ala Ser Ala Ala Ala Ala Ala Ala
        435                 440                 445

Ala Gly Ser Tyr Gly Ser Gly Pro Gly Ile Tyr Gly Pro Tyr Gly Pro
    450                 455                 460

Gly Ile Ser Gly Pro Gly Ser Gly Val Leu Gly Ile Gly Pro Tyr Gly
465                 470                 475                 480

Pro Gly Ala Ser Ala Ala Ala Ala Ala Ala Ala Gly Ser Tyr Gly Pro
                485                 490                 495

Gly Val Leu Gly Pro Tyr Gly Pro Gly Pro Ser Ala Ala Ala Ala Ala
            500                 505                 510

Ala Ala Gly Pro Gly Ser Gly Ile Tyr Gly Pro Gly Ala Ser Gly Ile
        515                 520                 525

Asn Gly Pro Gly Ser Gly Ile Tyr Gly Pro Gly Val Leu Gly Pro Gly
    530                 535                 540

Pro Ser Ala Ala Ala Ala Ala Ala Ala Gly Pro Gly Ser Gly Val Leu
545                 550                 555                 560

Gly Pro Gly Ala Ser
                565

<210>  34
<211>  565
<212>  PRT
<213>  Artificial Sequence

<220>
<223>  M_PRT525

<400>  34

Met Gly Pro Gly Gln Gln Gly Pro Tyr Gly Pro Gly Ala Ser Ala Ala
1               5                   10                  15

Ala Ala Ala Ala Ala Gly Ser Asn Gly Pro Gly Ser Gly Gln Gln Gly
                20                  25                  30

Pro Gly Gln Ser Gly Gln Tyr Gly Pro Gly Gln Gln Gly Pro Gly Gln
            35                  40                  45

Gln Gly Pro Gly Ser Ser Ala Ala Ala Ala Ala Ala Ala Gly Pro Gly
    50                  55                  60

Gln Tyr Gly Pro Gly Gln Gln Gly Pro Ser Ala Ser Ala Ala Ala Ala
65                  70                  75                  80

Ala Ala Ala Gly Pro Gly Ser Gly Gln Gln Gly Pro Gly Ala Ser Gly
                85                  90                  95

Gln Tyr Gly Pro Gly Gln Gln Gly Pro Gly Gln Gln Gly Pro Gly Ser
            100                 105                 110

Ser Ala Ala Ala Ala Ala Ala Ala Gly Ser Tyr Gly Ser Gly Pro Gly
        115                 120                 125

Gln Gln Gly Pro Tyr Gly Ser Ala Ala Ala Ala Ala Ala Ala Gly Pro
    130                 135                 140

Gly Ser Gly Gln Tyr Gly Gln Gly Pro Tyr Gly Pro Gly Ala Ser Gly
145                 150                 155                 160

Pro Gly Gln Tyr Gly Pro Gly Gln Gln Gly Pro Ser Ala Ser Ala Ala
                165                 170                 175

Ala Ala Ala Ala Ala Gly Ser Gly Gln Gln Gly Pro Gly Gln Tyr Gly
            180                 185                 190

Pro Tyr Ala Ser Ala Ala Ala Ala Ala Ala Ala Gly Ser Tyr Gly Ser
        195                 200                 205

Gly Pro Gly Gln Gln Gly Pro Tyr Gly Pro Gly Gln Ser Gly Ser Gly
    210                 215                 220

Gln Gln Gly Pro Gly Gln Gln Gly Pro Tyr Ala Ser Ala Ala Ala Ala
225                 230                 235                 240

Ala Ala Ala Gly Pro Gly Gln Gln Gly Pro Tyr Gly Pro Gly Ser Ser
                245                 250                 255

Ala Ala Ala Ala Ala Ala Ala Gly Ser Tyr Gly Tyr Gly Pro Gly Gln
            260                 265                 270

Gln Gly Pro Tyr Gly Pro Gly Ala Ser Gly Gln Asn Gly Pro Gly Ser
        275                 280                 285

Gly Gln Tyr Gly Pro Gly Gln Gln Gly Pro Gly Pro Ser Ala Ala Ala
    290                 295                 300

Ala Ala Ala Ala Gly Pro Gly Gln Gln Gly Pro Tyr Gly Pro Gly Ala
305                 310                 315                 320

Ser Ala Ala Ala Ala Ala Ala Ala Gly Ser Tyr Gly Pro Gly Gln Gln
                325                 330                 335

Gly Pro Gly Gln Tyr Gly Pro Gly Ser Ser Gly Pro Gly Gln Gln Gly
            340                 345                 350

Pro Tyr Gly Pro Gly Ser Ser Ala Ala Ala Ala Ala Ala Ala Gly Ser
        355                 360                 365

Tyr Gly Pro Gly Gln Gln Gly Pro Tyr Gly Pro Gly Pro Ser Ala Ala
    370                 375                 380

Ala Ala Ala Ala Ala Gly Ser Tyr Gln Gln Gly Pro Gly Gln Gln Gly
385                 390                 395                 400

Pro Tyr Gly Pro Gly Ala Ser Gly Pro Gly Gln Gln Gly Pro Tyr Gly
                405                 410                 415

Pro Gly Ala Ser Ala Ala Ala Ala Ala Ala Ala Gly Pro Gly Gln Tyr
            420                 425                 430

Gly Pro Gly Gln Gln Gly Pro Ser Ala Ser Ala Ala Ala Ala Ala Ala
        435                 440                 445

Ala Gly Ser Tyr Gly Ser Gly Pro Gly Gln Tyr Gly Pro Tyr Gly Pro
    450                 455                 460

Gly Gln Ser Gly Pro Gly Ser Gly Gln Gln Gly Gln Gly Pro Tyr Gly
465                 470                 475                 480

Pro Gly Ala Ser Ala Ala Ala Ala Ala Ala Ala Gly Ser Tyr Gly Pro
                485                 490                 495

Gly Gln Gln Gly Pro Tyr Gly Pro Gly Pro Ser Ala Ala Ala Ala Ala
            500                 505                 510

Ala Ala Gly Pro Gly Ser Gly Gln Tyr Gly Pro Gly Ala Ser Gly Gln

515                 520                 525

Asn Gly Pro Gly Ser Gly Gln Tyr Gly Pro Gly Gln Gln Gly Pro Gly
    530                 535                 540

Pro Ser Ala Ala Ala Ala Ala Ala Ala Gly Pro Gly Ser Gly Gln Gln
545                 550                 555                 560

Gly Pro Gly Ala Ser
                565

<210>  35
<211>  601
<212>  PRT
<213>  Artificial Sequence

<220>
<223>  PRT888

<400>  35

Met His His His His His His Ser Ser Gly Ser Ser Gly Pro Gly Val
1               5                   10                  15

Leu Gly Pro Tyr Gly Pro Gly Ala Ser Ala Ala Ala Ala Ala Gly Gln
            20                  25                  30

Asn Gly Pro Gly Ser Gly Val Leu Gly Pro Gly Gln Ser Gly Gln Tyr
        35                  40                  45

Gly Pro Gly Val Leu Gly Pro Gly Val Leu Gly Pro Gly Ser Ser Ala
    50                  55                  60

Ala Ala Ala Ala Gly Pro Gly Gln Tyr Gly Pro Gly Val Leu Gly Pro
65                  70                  75                  80

Ser Ala Ser Ala Ala Ala Ala Ala Gly Pro Gly Ser Gly Val Leu Gly
                85                  90                  95

Pro Gly Ala Ser Gly Gln Tyr Gly Pro Gly Val Leu Gly Pro Gly Val
            100                 105                 110

Leu Gly Pro Gly Ser Ser Ala Ala Ala Ala Ala Gly Gln Tyr Gly Ser
        115                 120                 125

Gly Pro Gly Val Leu Gly Pro Tyr Gly Ser Ala Ala Ala Ala Ala Gly
    130                 135                 140

Pro Gly Ser Gly Gln Tyr Gly Gln Gly Pro Tyr Gly Pro Gly Ala Ser
145                 150                 155                 160

Gly Pro Gly Gln Tyr Gly Pro Gly Val Leu Gly Pro Ser Ala Ser Ala
                165                 170                 175

Ala Ala Ala Ala Gly Ser Gly Val Leu Gly Pro Gly Gln Tyr Gly Pro
            180                 185                 190

Tyr Ala Ser Ala Ala Ala Ala Ala Gly Gln Tyr Gly Ser Gly Pro Gly
        195                 200                 205

Val Leu Gly Pro Tyr Gly Pro Gly Gln Ser Gly Ser Gly Val Leu Gly
    210                 215                 220

Pro Gly Val Leu Gly Pro Tyr Ala Ser Ala Ala Ala Ala Ala Gly Pro
225                 230                 235                 240

Gly Val Leu Gly Pro Tyr Gly Pro Gly Ser Ser Ala Ala Ala Ala Ala
                245                 250                 255

Gly Gln Tyr Gly Tyr Gly Pro Gly Val Leu Gly Pro Tyr Gly Pro Gly
            260                 265                 270

Ala Ser Gly Gln Asn Gly Pro Gly Ser Gly Gln Tyr Gly Pro Gly Val
        275                 280                 285

Leu Gly Pro Gly Gln Ser Ala Ala Ala Ala Ala Gly Pro Gly Val Leu
    290                 295                 300

Gly Pro Tyr Gly Pro Gly Ala Ser Ala Ala Ala Ala Ala Gly Gln Tyr
305                 310                 315                 320

Gly Pro Gly Val Leu Gly Pro Gly Gln Tyr Gly Pro Gly Ser Ser Gly
                325                 330                 335

Pro Gly Val Leu Gly Pro Tyr Gly Pro Gly Ser Ser Ala Ala Ala Ala
            340                 345                 350

Ala Gly Gln Tyr Gly Pro Gly Val Leu Gly Pro Tyr Gly Pro Gly Gln
        355                 360                 365

Ser Ala Ala Ala Ala Ala Gly Gln Tyr Val Leu Gly Pro Gly Val Leu
    370                 375                 380

Gly Pro Tyr Gly Pro Gly Ala Ser Gly Pro Gly Val Leu Gly Pro Tyr
385                 390                 395                 400

Gly Pro Gly Ala Ser Ala Ala Ala Ala Ala Gly Pro Gly Gln Tyr Gly

405                 410                 415

Pro Gly Val Leu Gly Pro Ser Ala Ser Ala Ala Ala Ala Ala Gly Gln
            420                 425                 430

Tyr Gly Ser Gly Pro Gly Gln Tyr Gly Pro Tyr Gly Pro Gly Gln Ser
        435                 440                 445

Gly Pro Gly Ser Gly Val Leu Gly Gln Gly Pro Tyr Gly Pro Gly Ala
    450                 455                 460

Ser Ala Ala Ala Ala Ala Gly Gln Tyr Gly Pro Gly Val Leu Gly Pro
465                 470                 475                 480

Tyr Gly Pro Gly Gln Ser Ala Ala Ala Ala Ala Gly Pro Gly Ser Gly
                485                 490                 495

Gln Tyr Gly Pro Gly Ala Ser Gly Gln Asn Gly Pro Gly Ser Gly Gln
            500                 505                 510

Tyr Gly Pro Gly Val Leu Gly Pro Gly Gln Ser Ala Ala Ala Ala Ala
        515                 520                 525

Gly Gln Tyr Val Leu Gly Pro Gly Val Leu Gly Pro Tyr Gly Pro Gly
    530                 535                 540

Ala Ser Ala Ala Ala Ala Ala Gly Gln Tyr Gly Ser Gly Pro Gly Val
545                 550                 555                 560

Leu Gly Pro Tyr Gly Pro Gly Gln Ser Gly Ser Gly Val Leu Gly Pro
                565                 570                 575

Gly Val Leu Gly Pro Tyr Ala Ser Ala Ala Ala Ala Ala Gly Pro Gly
            580                 585                 590

Ser Gly Val Leu Gly Pro Gly Ala Ser
        595                 600

<210>  36
<211>  601
<212>  PRT
<213>  Artificial Sequence

<220>
<223>  PRT965

<400>  36
   Met His His His His His His Ser Ser Gly Ser Ser Gly Pro Gly Thr
   1               5                   10                  15
   Ser Gly Pro Tyr Gly Pro Gly Ala Ser Ala Ala Ala Ala Ala Gly Ala
               20                  25                  30
   Asn Gly Pro Gly Ser Gly Thr Ser Gly Pro Gly Ala Ser Gly Ala Tyr
           35                  40                  45
   Gly Pro Gly Thr Ser Gly Pro Gly Thr Ser Gly Pro Gly Ser Ser Ala
       50                  55                  60
   Ala Ala Ala Ala Gly Pro Gly Ala Tyr Gly Pro Gly Thr Ser Gly Pro
   65                  70                  75                  80
   Ser Ala Ser Ala Ala Ala Ala Ala Gly Pro Gly Ser Gly Thr Ser Gly
                   85                  90                  95
   Pro Gly Ala Ser Gly Ala Tyr Gly Pro Gly Thr Ser Gly Pro Gly Thr
               100                 105                 110
   Ser Gly Pro Gly Ser Ser Ala Ala Ala Ala Ala Gly Ala Tyr Gly Ser
           115                 120                 125
   Gly Pro Gly Thr Ser Gly Pro Tyr Gly Ser Ala Ala Ala Ala Ala Gly
       130                 135                 140
   Pro Gly Ser Gly Ala Tyr Gly Ala Gly Pro Tyr Gly Pro Gly Ala Ser
   145                 150                 155                 160
   Gly Pro Gly Ala Tyr Gly Pro Gly Thr Ser Gly Pro Ser Ala Ser Ala
                   165                 170                 175
   Ala Ala Ala Ala Gly Ser Gly Thr Ser Gly Pro Gly Ala Tyr Gly Pro
               180                 185                 190
   Tyr Ala Ser Ala Ala Ala Ala Ala Gly Ala Tyr Gly Ser Gly Pro Gly
           195                 200                 205
   Thr Ser Gly Pro Tyr Gly Pro Gly Ala Ser Gly Ser Gly Thr Ser Gly
       210                 215                 220
   Pro Gly Thr Ser Gly Pro Tyr Ala Ser Ala Ala Ala Ala Ala Gly Pro
   225                 230                 235                 240
   Gly Thr Ser Gly Pro Tyr Gly Pro Gly Ser Ser Ala Ala Ala Ala Ala
                   245                 250                 255
   Gly Ala Tyr Gly Tyr Gly Pro Gly Thr Ser Gly Pro Tyr Gly Pro Gly
   260 265 270
   Ala Ser Gly Ala Asn Gly Pro Gly Ser Gly Ala Tyr Gly Pro Gly Thr
   275 280 285
   Ser Gly Pro Gly Ala Ser Ala Ala Ala Ala Ala Gly Pro Gly Thr Ser
   290 295 300
   Gly Pro Tyr Gly Pro Gly Ala Ser Ala Ala Ala Ala Ala Gly Ala Tyr
   305 310 315 320
   Gly Pro Gly Thr Ser Gly Pro Gly Ala Tyr Gly Pro Gly Ser Ser Gly
   325 330 335
   Pro Gly Thr Ser Gly Pro Tyr Gly Pro Gly Ser Ser Ala Ala Ala Ala
   340 345 350
   Ala Gly Ala Tyr Gly Pro Gly Thr Ser Gly Pro Tyr Gly Pro Gly Ala
   355 360 365
   Ser Ala Ala Ala Ala Ala Gly Ala Tyr Thr Ser Gly Pro Gly Thr Ser
   370 375 380
   Gly Pro Tyr Gly Pro Gly Ala Ser Gly Pro Gly Thr Ser Gly Pro Tyr
   385 390 395 400
   Gly Pro Gly Ala Ser Ala Ala Ala Ala Ala Gly Pro Gly Ala Tyr Gly
   405 410 415
   Pro Gly Thr Ser Gly Pro Ser Ala Ser Ala Ala Ala Ala Ala Gly Ala
   420 425 430
   Tyr Gly Ser Gly Pro Gly Ala Tyr Gly Pro Tyr Gly Pro Gly Ala Ser
   435 440 445
   Gly Pro Gly Ser Gly Thr Ser Gly Ala Gly Pro Tyr Gly Pro Gly Ala
   450 455 460
   Ser Ala Ala Ala Ala Ala Gly Ala Tyr Gly Pro Gly Thr Ser Gly Pro
   465 470 475 480
   Tyr Gly Pro Gly Ala Ser Ala Ala Ala Ala Ala Gly Pro Gly Ser Gly
   485 490 495
   Ala Tyr Gly Pro Gly Ala Ser Gly Ala Asn Gly Pro Gly Ser Gly Ala
   500 505 510
   Tyr Gly Pro Gly Thr Ser Gly Pro Gly Ala Ser Ala Ala Ala Ala Ala
           515                 520                 525
   Gly Ala Tyr Thr Ser Gly Pro Gly Thr Ser Gly Pro Tyr Gly Pro Gly
       530                 535                 540
   Ala Ser Ala Ala Ala Ala Ala Gly Ala Tyr Gly Ser Gly Pro Gly Thr
   545                 550                 555                 560
   Ser Gly Pro Tyr Gly Pro Gly Ala Ser Gly Ser Gly Thr Ser Gly Pro
                   565                 570                 575
   Gly Thr Ser Gly Pro Tyr Ala Ser Ala Ala Ala Ala Ala Gly Pro Gly
               580                 585                 590
   Ser Gly Thr Ser Gly Pro Gly Ala Ser
           595                 600

<210>  37
<211>  601
<212>  PRT
<213>  Artificial Sequence

<220>
<223>  PRT889

<400>  37
   Met His His His His His His Ser Ser Gly Ser Ser Gly Pro Gly Val
   1               5                   10                  15
   Leu Gly Pro Tyr Gly Pro Gly Ala Ser Ala Ala Ala Ala Ala Gly Ile
               20                  25                  30
   Asn Gly Pro Gly Ser Gly Val Leu Gly Pro Gly Ile Ser Gly Ile Tyr
           35                  40                  45
   Gly Pro Gly Val Leu Gly Pro Gly Val Leu Gly Pro Gly Ser Ser Ala
       50                  55                  60
   Ala Ala Ala Ala Gly Pro Gly Ile Tyr Gly Pro Gly Val Leu Gly Pro
   65                  70                  75                  80
   Ser Ala Ser Ala Ala Ala Ala Ala Gly Pro Gly Ser Gly Val Leu Gly
                   85                  90                  95
   Pro Gly Ala Ser Gly Ile Tyr Gly Pro Gly Val Leu Gly Pro Gly Val
               100                 105                 110
   Leu Gly Pro Gly Ser Ser Ala Ala Ala Ala Ala Gly Ile Tyr Gly Ser
   115 120 125
   Gly Pro Gly Val Leu Gly Pro Tyr Gly Ser Ala Ala Ala Ala Ala Gly
       130                 135                 140
   Pro Gly Ser Gly Ile Tyr Gly Ile Gly Pro Tyr Gly Pro Gly Ala Ser
   145                 150                 155                 160
   Gly Pro Gly Ile Tyr Gly Pro Gly Val Leu Gly Pro Ser Ala Ser Ala
                   165                 170                 175
   Ala Ala Ala Ala Gly Ser Gly Val Leu Gly Pro Gly Ile Tyr Gly Pro
               180                 185                 190
   Tyr Ala Ser Ala Ala Ala Ala Ala Gly Ile Tyr Gly Ser Gly Pro Gly
           195                 200                 205
   Val Leu Gly Pro Tyr Gly Pro Gly Ile Ser Gly Ser Gly Val Leu Gly
       210                 215                 220
   Pro Gly Val Leu Gly Pro Tyr Ala Ser Ala Ala Ala Ala Ala Gly Pro
   225                 230                 235                 240
   Gly Val Leu Gly Pro Tyr Gly Pro Gly Ser Ser Ala Ala Ala Ala Ala
                   245                 250                 255
   Gly Ile Tyr Gly Tyr Gly Pro Gly Val Leu Gly Pro Tyr Gly Pro Gly
               260                 265                 270
   Ala Ser Gly Ile Asn Gly Pro Gly Ser Gly Ile Tyr Gly Pro Gly Val
           275                 280                 285
   Leu Gly Pro Gly Ile Ser Ala Ala Ala Ala Ala Gly Pro Gly Val Leu
       290                 295                 300
   Gly Pro Tyr Gly Pro Gly Ala Ser Ala Ala Ala Ala Ala Gly Ile Tyr
   305                 310                 315                 320
   Gly Pro Gly Val Leu Gly Pro Gly Ile Tyr Gly Pro Gly Ser Ser Gly
                   325                 330                 335
   Pro Gly Val Leu Gly Pro Tyr Gly Pro Gly Ser Ser Ala Ala Ala Ala
               340                 345                 350
   Ala Gly Ile Tyr Gly Pro Gly Val Leu Gly Pro Tyr Gly Pro Gly Ile
           355                 360                 365
   Ser Ala Ala Ala Ala Ala Gly Ile Tyr Val Leu Gly Pro Gly Val Leu
       370                 375                 380
   Gly Pro Tyr Gly Pro Gly Ala Ser Gly Pro Gly Val Leu Gly Pro Tyr
   385                 390                 395                 400
   Gly Pro Gly Ala Ser Ala Ala Ala Ala Ala Gly Pro Gly Ile Tyr Gly
                   405                 410                 415
   Pro Gly Val Leu Gly Pro Ser Ala Ser Ala Ala Ala Ala Ala Gly Ile
               420                 425                 430
   Tyr Gly Ser Gly Pro Gly Ile Tyr Gly Pro Tyr Gly Pro Gly Ile Ser
           435                 440                 445
   Gly Pro Gly Ser Gly Val Leu Gly Ile Gly Pro Tyr Gly Pro Gly Ala
       450                 455                 460
   Ser Ala Ala Ala Ala Ala Gly Ile Tyr Gly Pro Gly Val Leu Gly Pro
   465                 470                 475                 480
   Tyr Gly Pro Gly Ile Ser Ala Ala Ala Ala Ala Gly Pro Gly Ser Gly
                   485                 490                 495
   Ile Tyr Gly Pro Gly Ala Ser Gly Ile Asn Gly Pro Gly Ser Gly Ile
               500                 505                 510
   Tyr Gly Pro Gly Val Leu Gly Pro Gly Ile Ser Ala Ala Ala Ala Ala
           515                 520                 525
   Gly Ile Tyr Val Leu Gly Pro Gly Val Leu Gly Pro Tyr Gly Pro Gly
       530                 535                 540
   Ala Ser Ala Ala Ala Ala Ala Gly Ile Tyr Gly Ser Gly Pro Gly Val
   545                 550                 555                 560
   Leu Gly Pro Tyr Gly Pro Gly Ile Ser Gly Ser Gly Val Leu Gly Pro
                   565                 570                 575
   Gly Val Leu Gly Pro Tyr Ala Ser Ala Ala Ala Ala Ala Gly Pro Gly
               580                 585                 590
   Ser Gly Val Leu Gly Pro Gly Ala Ser
           595                 600

<210>  38
<211>  601
<212>  PRT

<213>  Artificial Sequence

<220>
<223>  PRT916

<400>  38
   Met His His His His His His Ser Ser Gly Ser Ser Gly Pro Gly Val
   1               5                   10                  15
   Ile Gly Pro Tyr Gly Pro Gly Ala Ser Ala Ala Ala Ala Ala Gly Leu
               20                  25                  30
   Asn Gly Pro Gly Ser Gly Val Ile Gly Pro Gly Leu Ser Gly Leu Tyr
           35                  40                  45
   Gly Pro Gly Val Ile Gly Pro Gly Val Ile Gly Pro Gly Ser Ser Ala
       50                  55                  60
   Ala Ala Ala Ala Gly Pro Gly Leu Tyr Gly Pro Gly Val Ile Gly Pro
   65                  70                  75                  80
   Ser Ala Ser Ala Ala Ala Ala Ala Gly Pro Gly Ser Gly Val Ile Gly
                   85                  90                  95
   Pro Gly Ala Ser Gly Leu Tyr Gly Pro Gly Val Ile Gly Pro Gly Val
               100                 105                 110
   Ile Gly Pro Gly Ser Ser Ala Ala Ala Ala Ala Gly Leu Tyr Gly Ser
           115                 120                 125
   Gly Pro Gly Val Ile Gly Pro Tyr Gly Ser Ala Ala Ala Ala Ala Gly
       130                 135                 140
   Pro Gly Ser Gly Leu Tyr Gly Leu Gly Pro Tyr Gly Pro Gly Ala Ser
   145                 150                 155                 160
   Gly Pro Gly Leu Tyr Gly Pro Gly Val Ile Gly Pro Ser Ala Ser Ala
                   165                 170                 175
   Ala Ala Ala Ala Gly Ser Gly Val Ile Gly Pro Gly Leu Tyr Gly Pro
               180                 185                 190
   Tyr Ala Ser Ala Ala Ala Ala Ala Gly Leu Tyr Gly Ser Gly Pro Gly
           195                 200                 205
   Val Ile Gly Pro Tyr Gly Pro Gly Leu Ser Gly Ser Gly Val Ile Gly
       210                 215                 220
   Pro Gly Val Ile Gly Pro Tyr Ala Ser Ala Ala Ala Ala Ala Gly Pro
   225                 230                 235                 240
   Gly Val Ile Gly Pro Tyr Gly Pro Gly Ser Ser Ala Ala Ala Ala Ala
                   245                 250                 255
   Gly Leu Tyr Gly Tyr Gly Pro Gly Val Ile Gly Pro Tyr Gly Pro Gly
               260                 265                 270
   Ala Ser Gly Leu Asn Gly Pro Gly Ser Gly Leu Tyr Gly Pro Gly Val
           275                 280                 285
   Ile Gly Pro Gly Leu Ser Ala Ala Ala Ala Ala Gly Pro Gly Val Ile
       290                 295                 300
   Gly Pro Tyr Gly Pro Gly Ala Ser Ala Ala Ala Ala Ala Gly Leu Tyr
   305                 310                 315                 320
   Gly Pro Gly Val Ile Gly Pro Gly Leu Tyr Gly Pro Gly Ser Ser Gly
                   325                 330                 335
   Pro Gly Val Ile Gly Pro Tyr Gly Pro Gly Ser Ser Ala Ala Ala Ala
               340                 345                 350
   Ala Gly Leu Tyr Gly Pro Gly Val Ile Gly Pro Tyr Gly Pro Gly Leu
           355                 360                 365
   Ser Ala Ala Ala Ala Ala Gly Leu Tyr Val Ile Gly Pro Gly Val Ile
       370                 375                 380
   Gly Pro Tyr Gly Pro Gly Ala Ser Gly Pro Gly Val Ile Gly Pro Tyr
   385                 390                 395                 400
   Gly Pro Gly Ala Ser Ala Ala Ala Ala Ala Gly Pro Gly Leu Tyr Gly
                   405                 410                 415
   Pro Gly Val Ile Gly Pro Ser Ala Ser Ala Ala Ala Ala Ala Gly Leu
               420                 425                 430
   Tyr Gly Ser Gly Pro Gly Leu Tyr Gly Pro Tyr Gly Pro Gly Leu Ser
           435                 440                 445
   Gly Pro Gly Ser Gly Val Ile Gly Leu Gly Pro Tyr Gly Pro Gly Ala
       450                 455                 460
   Ser Ala Ala Ala Ala Ala Gly Leu Tyr Gly Pro Gly Val Ile Gly Pro
   465                 470                 475                 480
   Tyr Gly Pro Gly Leu Ser Ala Ala Ala Ala Ala Gly Pro Gly Ser Gly
                   485                 490                 495
   Leu Tyr Gly Pro Gly Ala Ser Gly Leu Asn Gly Pro Gly Ser Gly Leu
               500                 505                 510
   Tyr Gly Pro Gly Val Ile Gly Pro Gly Leu Ser Ala Ala Ala Ala Ala
           515                 520                 525
   Gly Leu Tyr Val Ile Gly Pro Gly Val Ile Gly Pro Tyr Gly Pro Gly
       530                 535                 540
   Ala Ser Ala Ala Ala Ala Ala Gly Leu Tyr Gly Ser Gly Pro Gly Val
   545                 550                 555                 560
   Ile Gly Pro Tyr Gly Pro Gly Leu Ser Gly Ser Gly Val Ile Gly Pro
                   565                 570                 575
   Gly Val Ile Gly Pro Tyr Ala Ser Ala Ala Ala Ala Ala Gly Pro Gly
               580                 585                 590
   Ser Gly Val Ile Gly Pro Gly Ala Ser
           595                 600

<210>  39
<211>  601
<212>  PRT
<213>  Artificial Sequence

<220>
<223>  PRT918

<400>  39
   Met His His His His His His Ser Ser Gly Ser Ser Gly Pro Gly Val
   1               5                   10                  15
   Phe Gly Pro Tyr Gly Pro Gly Ala Ser Ala Ala Ala Ala Ala Gly Ile
               20                  25                  30
   Asn Gly Pro Gly Ser Gly Val Phe Gly Pro Gly Ile Ser Gly Ile Tyr
           35                  40                  45
   Gly Pro Gly Val Phe Gly Pro Gly Val Phe Gly Pro Gly Ser Ser Ala
       50                  55                  60
   Ala Ala Ala Ala Gly Pro Gly Ile Tyr Gly Pro Gly Val Phe Gly Pro
   65                  70                  75                  80
   Ser Ala Ser Ala Ala Ala Ala Ala Gly Pro Gly Ser Gly Val Phe Gly
                   85                  90                  95
   Pro Gly Ala Ser Gly Ile Tyr Gly Pro Gly Val Phe Gly Pro Gly Val
               100                 105                 110
   Phe Gly Pro Gly Ser Ser Ala Ala Ala Ala Ala Gly Ile Tyr Gly Ser
           115                 120                 125
   Gly Pro Gly Val Phe Gly Pro Tyr Gly Ser Ala Ala Ala Ala Ala Gly
       130                 135                 140
   Pro Gly Ser Gly Ile Tyr Gly Ile Gly Pro Tyr Gly Pro Gly Ala Ser
   145                 150                 155                 160
   Gly Pro Gly Ile Tyr Gly Pro Gly Val Phe Gly Pro Ser Ala Ser Ala
                   165                 170                 175
   Ala Ala Ala Ala Gly Ser Gly Val Phe Gly Pro Gly Ile Tyr Gly Pro
               180                 185                 190
   Tyr Ala Ser Ala Ala Ala Ala Ala Gly Ile Tyr Gly Ser Gly Pro Gly
           195                 200                 205
   Val Phe Gly Pro Tyr Gly Pro Gly Ile Ser Gly Ser Gly Val Phe Gly
       210                 215                 220
   Pro Gly Val Phe Gly Pro Tyr Ala Ser Ala Ala Ala Ala Ala Gly Pro
   225                 230                 235                 240
   Gly Val Phe Gly Pro Tyr Gly Pro Gly Ser Ser Ala Ala Ala Ala Ala
                   245                 250                 255
   Gly Ile Tyr Gly Tyr Gly Pro Gly Val Phe Gly Pro Tyr Gly Pro Gly
               260                 265                 270
   Ala Ser Gly Ile Asn Gly Pro Gly Ser Gly Ile Tyr Gly Pro Gly Val
           275                 280                 285
   Phe Gly Pro Gly Ile Ser Ala Ala Ala Ala Ala Gly Pro Gly Val Phe
       290                 295                 300
   Gly Pro Tyr Gly Pro Gly Ala Ser Ala Ala Ala Ala Ala Gly Ile Tyr
   305                 310                 315                 320
   Gly Pro Gly Val Phe Gly Pro Gly Ile Tyr Gly Pro Gly Ser Ser Gly
                   325                 330                 335
   Pro Gly Val Phe Gly Pro Tyr Gly Pro Gly Ser Ser Ala Ala Ala Ala
                   340                 345                 350
   Ala Gly Ile Tyr Gly Pro Gly Val Phe Gly Pro Tyr Gly Pro Gly Ile
               355                 360                 365
   Ser Ala Ala Ala Ala Ala Gly Ile Tyr Val Phe Gly Pro Gly Val Phe
           370                 375                 380
   Gly Pro Tyr Gly Pro Gly Ala Ser Gly Pro Gly Val Phe Gly Pro Tyr
   385                 390                 395                 400
   Gly Pro Gly Ala Ser Ala Ala Ala Ala Ala Gly Pro Gly Ile Tyr Gly
                   405                 410                 415
   Pro Gly Val Phe Gly Pro Ser Ala Ser Ala Ala Ala Ala Ala Gly Ile
                   420                 425                 430
   Tyr Gly Ser Gly Pro Gly Ile Tyr Gly Pro Tyr Gly Pro Gly Ile Ser
               435                 440                 445
   Gly Pro Gly Ser Gly Val Phe Gly Ile Gly Pro Tyr Gly Pro Gly Ala
           450                 455                 460
   Ser Ala Ala Ala Ala Ala Gly Ile Tyr Gly Pro Gly Val Phe Gly Pro
   465                 470                 475                 480
   Tyr Gly Pro Gly Ile Ser Ala Ala Ala Ala Ala Gly Pro Gly Ser Gly
                   485                 490                 495
   Ile Tyr Gly Pro Gly Ala Ser Gly Ile Asn Gly Pro Gly Ser Gly Ile
                   500                 505                 510
   Tyr Gly Pro Gly Val Phe Gly Pro Gly Ile Ser Ala Ala Ala Ala Ala
               515                 520                 525
   Gly Ile Tyr Val Phe Gly Pro Gly Val Phe Gly Pro Tyr Gly Pro Gly
           530                 535                 540
   Ala Ser Ala Ala Ala Ala Ala Gly Ile Tyr Gly Ser Gly Pro Gly Val
   545                 550                 555                 560
   Phe Gly Pro Tyr Gly Pro Gly Ile Ser Gly Ser Gly Val Phe Gly Pro
                   565                 570                 575
   Gly Val Phe Gly Pro Tyr Ala Ser Ala Ala Ala Ala Ala Gly Pro Gly
                   580                 585                 590
   Ser Gly Val Phe Gly Pro Gly Ala Ser
           595                 600

<210>  40
<211>  576
<212>  PRT
<213>  Artificial Sequence

<220>
<223>  PRT699

<400>  40
   Met His His His His His His Ser Ser Gly Ser Ser Gly Pro Gly Val
   1               5                   10                  15
   Leu Gly Pro Tyr Gly Pro Gly Ala Ser Ala Ala Ala Ala Ala Ala Ala
               20                  25                  30
   Gly Ser Asn Gly Pro Gly Ser Gly Val Leu Gly Pro Gly Gln Ser Gly
           35                  40                  45
   Gln Tyr Gly Pro Gly Val Leu Gly Pro Gly Val Leu Gly Pro Gly Ser
       50                  55                  60
   Ser Ala Ala Ala Ala Ala Ala Ala Gly Pro Gly Gln Tyr Gly Pro Gly
   65                  70                  75                  80
   Val Leu Gly Pro Ser Ala Ser Ala Ala Ala Ala Ala Ala Ala Gly Pro
                   85                  90                  95
   Gly Ser Gly Val Leu Gly Pro Gly Ala Ser Gly Gln Tyr Gly Pro Gly
               100                 105                 110
   Val Leu Gly Pro Gly Val Leu Gly Pro Gly Ser Ser Ala Ala Ala Ala
           115                 120                 125
   Ala Ala Ala Gly Ser Tyr Gly Ser Gly Pro Gly Val Leu Gly Pro Tyr
       130                 135                 140
   Gly Ser Ala Ala Ala Ala Ala Ala Ala Gly Pro Gly Ser Gly Gln Tyr
   145                 150                 155                 160
   Gly Gln Gly Pro Tyr Gly Pro Gly Ala Ser Gly Pro Gly Gln Tyr Gly
                   165                 170                 175
   Pro Gly Val Leu Gly Pro Ser Ala Ser Ala Ala Ala Ala Ala Ala Ala
               180                 185                 190
   Gly Ser Gly Val Leu Gly Pro Gly Gln Tyr Gly Pro Tyr Ala Ser Ala
           195                 200                 205
   Ala Ala Ala Ala Ala Ala Gly Ser Tyr Gly Ser Gly Pro Gly Val Leu
       210                 215                 220
   Gly Pro Tyr Gly Pro Gly Gln Ser Gly Ser Gly Val Leu Gly Pro Gly
   225                 230                 235                 240
   Val Leu Gly Pro Tyr Ala Ser Ala Ala Ala Ala Ala Ala Ala Gly Pro
                   245                 250                 255
   Gly Val Leu Gly Pro Tyr Gly Pro Gly Ser Ser Ala Ala Ala Ala Ala
               260                 265                 270
   Ala Ala Gly Ser Tyr Gly Tyr Gly Pro Gly Val Leu Gly Pro Tyr Gly
           275                 280                 285
   Pro Gly Ala Ser Gly Gln Asn Gly Pro Gly Ser Gly Gln Tyr Gly Pro
       290                 295                 300
   Gly Val Leu Gly Pro Gly Pro Ser Ala Ala Ala Ala Ala Ala Ala Gly
   305                 310                 315                 320
   Pro Gly Val Leu Gly Pro Tyr Gly Pro Gly Ala Ser Ala Ala Ala Ala
                   325                 330                 335
   Ala Ala Ala Gly Ser Tyr Gly Pro Gly Val Leu Gly Pro Gly Gln Tyr
               340                 345                 350
   Gly Pro Gly Ser Ser Gly Pro Gly Val Leu Gly Pro Tyr Gly Pro Gly
           355                 360                 365
   Ser Ser Ala Ala Ala Ala Ala Ala Ala Gly Ser Tyr Gly Pro Gly Val
       370                 375                 380
   Leu Gly Pro Tyr Gly Pro Gly Pro Ser Ala Ala Ala Ala Ala Ala Ala
   385                 390                 395                 400
   Gly Ser Tyr Val Leu Gly Pro Gly Val Leu Gly Pro Tyr Gly Pro Gly
                   405                 410                 415
   Ala Ser Gly Pro Gly Val Leu Gly Pro Tyr Gly Pro Gly Ala Ser Ala
               420                 425                 430
   Ala Ala Ala Ala Ala Ala Gly Pro Gly Gln Tyr Gly Pro Gly Val Leu
           435                 440                 445
   Gly Pro Ser Ala Ser Ala Ala Ala Ala Ala Ala Ala Gly Ser Tyr Gly
       450                 455                 460
   Ser Gly Pro Gly Gln Tyr Gly Pro Tyr Gly Pro Gly Gln Ser Gly Pro
   465                 470                 475                 480
   Gly Ser Gly Val Leu Gly Gln Gly Pro Tyr Gly Pro Gly Ala Ser Ala
                   485                 490                 495
   Ala Ala Ala Ala Ala Ala Gly Ser Tyr Gly Pro Gly Val Leu Gly Pro
               500                 505                 510
   Tyr Gly Pro Gly Pro Ser Ala Ala Ala Ala Ala Ala Ala Gly Pro Gly
           515                 520                 525
   Ser Gly Gln Tyr Gly Pro Gly Ala Ser Gly Gln Asn Gly Pro Gly Ser
       530                 535                 540
   Gly Gln Tyr Gly Pro Gly Val Leu Gly Pro Gly Pro Ser Ala Ala Ala
   545                 550                 555                 560
   Ala Ala Ala Ala Gly Pro Gly Ser Gly Val Leu Gly Pro Gly Ala Ser
                   565                 570                 575

<210>  41
<211>  576
<212>  PRT
<213>  Artificial Sequence

<220>
<223>  PRT698

<400>  41

Met His His His His His His Ser Ser Gly Ser Ser Gly Pro Gly Val
1               5                   10                  15

Leu Gly Pro Tyr Gly Pro Gly Ala Ser Ala Ala Ala Ala Ala Ala Ala
            20                  25                  30

Gly Ser Asn Gly Pro Gly Ser Gly Val Leu Gly Pro Gly Ile Ser Gly
        35                  40                  45

Ile Tyr Gly Pro Gly Val Leu Gly Pro Gly Val Leu Gly Pro Gly Ser
    50                  55                  60

Ser Ala Ala Ala Ala Ala Ala Ala Gly Pro Gly Ile Tyr Gly Pro Gly
65                  70                  75                  80

Val Leu Gly Pro Ser Ala Ser Ala Ala Ala Ala Ala Ala Ala Gly Pro
                85                  90                  95

Gly Ser Gly Val Leu Gly Pro Gly Ala Ser Gly Ile Tyr Gly Pro Gly
            100                 105                 110

Val Leu Gly Pro Gly Val Leu Gly Pro Gly Ser Ser Ala Ala Ala Ala
        115                 120                 125

Ala Ala Ala Gly Ser Tyr Gly Ser Gly Pro Gly Val Leu Gly Pro Tyr
    130                 135                 140

Gly Ser Ala Ala Ala Ala Ala Ala Ala Gly Pro Gly Ser Gly Ile Tyr
145                 150                 155                 160

Gly Ile Gly Pro Tyr Gly Pro Gly Ala Ser Gly Pro Gly Ile Tyr Gly
                165                 170                 175

Pro Gly Val Leu Gly Pro Ser Ala Ser Ala Ala Ala Ala Ala Ala Ala
            180                 185                 190

Gly Ser Gly Val Leu Gly Pro Gly Ile Tyr Gly Pro Tyr Ala Ser Ala
        195                 200                 205

Ala Ala Ala Ala Ala Ala Gly Ser Tyr Gly Ser Gly Pro Gly Val Leu
    210                 215                 220

Gly Pro Tyr Gly Pro Gly Ile Ser Gly Ser Gly Val Leu Gly Pro Gly
225                 230                 235                 240

Val Leu Gly Pro Tyr Ala Ser Ala Ala Ala Ala Ala Ala Ala Gly Pro
                245                 250                 255

Gly Val Leu Gly Pro Tyr Gly Pro Gly Ser Ser Ala Ala Ala Ala Ala
            260                 265                 270

Ala Ala Gly Ser Tyr Gly Tyr Gly Pro Gly Val Leu Gly Pro Tyr Gly
        275                 280                 285

Pro Gly Ala Ser Gly Ile Asn Gly Pro Gly Ser Gly Ile Tyr Gly Pro
    290                 295                 300

Gly Val Leu Gly Pro Gly Pro Ser Ala Ala Ala Ala Ala Ala Ala Gly
305                 310                 315                 320

Pro Gly Val Leu Gly Pro Tyr Gly Pro Gly Ala Ser Ala Ala Ala Ala
                325                 330                 335

Ala Ala Ala Gly Ser Tyr Gly Pro Gly Val Leu Gly Pro Gly Ile Tyr
            340                 345                 350

Gly Pro Gly Ser Ser Gly Pro Gly Val Leu Gly Pro Tyr Gly Pro Gly
        355                 360                 365

Ser Ser Ala Ala Ala Ala Ala Ala Ala Gly Ser Tyr Gly Pro Gly Val
    370                 375                 380

Leu Gly Pro Tyr Gly Pro Gly Pro Ser Ala Ala Ala Ala Ala Ala Ala
385                 390                 395                 400

Gly Ser Tyr Val Leu Gly Pro Gly Val Leu Gly Pro Tyr Gly Pro Gly
                405                 410                 415

Ala Ser Gly Pro Gly Val Leu Gly Pro Tyr Gly Pro Gly Ala Ser Ala
            420                 425                 430

Ala Ala Ala Ala Ala Ala Gly Pro Gly Ile Tyr Gly Pro Gly Val Leu
        435                 440                 445

Gly Pro Ser Ala Ser Ala Ala Ala Ala Ala Ala Ala Gly Ser Tyr Gly
    450                 455                 460

Ser Gly Pro Gly Ile Tyr Gly Pro Tyr Gly Pro Gly Ile Ser Gly Pro
465                 470                 475                 480

Gly Ser Gly Val Leu Gly Ile Gly Pro Tyr Gly Pro Gly Ala Ser Ala
                485                 490                 495

Ala Ala Ala Ala Ala Ala Gly Ser Tyr Gly Pro Gly Val Leu Gly Pro
            500                 505                 510

Tyr Gly Pro Gly Pro Ser Ala Ala Ala Ala Ala Ala Ala Gly Pro Gly
        515                 520                 525

Ser Gly Ile Tyr Gly Pro Gly Ala Ser Gly Ile Asn Gly Pro Gly Ser
    530                 535                 540

Gly Ile Tyr Gly Pro Gly Val Leu Gly Pro Gly Pro Ser Ala Ala Ala
545                 550                 555                 560

Ala Ala Ala Ala Gly Pro Gly Ser Gly Val Leu Gly Pro Gly Ala Ser
                565                 570                 575

<210>  42

<211>  17
<212>  PRT
<213>  Artificial Sequence

<220>
<223>  Hinge+His-tag

<400>  42

Ser Ser Val Asp Lys Leu Ala Ala Ala Leu Glu His His His His His
1               5                   10                  15

His

<210>  43
<211>  1179
<212>  PRT
<213>  Artificial Sequence

<220>
<223>  Met-PRT966

<400>  43

Met Gly Pro Gly Val Phe Gly Pro Tyr Gly Pro Gly Ala Ser Ala Ala
1               5                   10                  15

Ala Ala Ala Gly Ile Asn Gly Pro Gly Ser Gly Val Phe Gly Pro Gly
            20                  25                  30

Ile Ser Gly Ile Tyr Gly Pro Gly Val Phe Gly Pro Gly Val Phe Gly
        35                  40                  45

Pro Gly Ser Ser Ala Ala Ala Ala Ala Gly Pro Gly Ile Tyr Gly Pro
    50                  55                  60

Gly Val Phe Gly Pro Ser Ala Ser Ala Ala Ala Ala Ala Gly Pro Gly
65                  70                  75                  80

Ser Gly Val Phe Gly Pro Gly Ala Ser Gly Ile Tyr Gly Pro Gly Val
                85                  90                  95

Phe Gly Pro Gly Val Phe Gly Pro Gly Ser Ser Ala Ala Ala Ala Ala
            100                 105                 110

Gly Ile Tyr Gly Ser Gly Pro Gly Val Phe Gly Pro Tyr Gly Ser Ala
        115                 120                 125

Ala Ala Ala Ala Gly Pro Gly Ser Gly Ile Tyr Gly Ile Gly Pro Tyr
    130                 135                 140

Gly Pro Gly Ala Ser Gly Pro Gly Ile Tyr Gly Pro Gly Val Phe Gly
145                 150                 155                 160

Pro Ser Ala Ser Ala Ala Ala Ala Ala Gly Ser Gly Val Phe Gly Pro
                165                 170                 175

Gly Ile Tyr Gly Pro Tyr Ala Ser Ala Ala Ala Ala Ala Gly Ile Tyr
            180                 185                 190

Gly Ser Gly Pro Gly Val Phe Gly Pro Tyr Gly Pro Gly Ile Ser Gly
        195                 200                 205

Ser Gly Val Phe Gly Pro Gly Val Phe Gly Pro Tyr Ala Ser Ala Ala
    210                 215                 220

Ala Ala Ala Gly Pro Gly Val Phe Gly Pro Tyr Gly Pro Gly Ser Ser
225                 230                 235                 240

Ala Ala Ala Ala Ala Gly Ile Tyr Gly Tyr Gly Pro Gly Val Phe Gly
                245                 250                 255

Pro Tyr Gly Pro Gly Ala Ser Gly Ile Asn Gly Pro Gly Ser Gly Ile
            260                 265                 270

Tyr Gly Pro Gly Val Phe Gly Pro Gly Ile Ser Ala Ala Ala Ala Ala
        275                 280                 285

Gly Pro Gly Val Phe Gly Pro Tyr Gly Pro Gly Ala Ser Ala Ala Ala
    290                 295                 300

Ala Ala Gly Ile Tyr Gly Pro Gly Val Phe Gly Pro Gly Ile Tyr Gly
305                 310                 315                 320

Pro Gly Ser Ser Gly Pro Gly Val Phe Gly Pro Tyr Gly Pro Gly Ser
                325                 330                 335

Ser Ala Ala Ala Ala Ala Gly Ile Tyr Gly Pro Gly Val Phe Gly Pro
            340                 345                 350

Tyr Gly Pro Gly Ile Ser Ala Ala Ala Ala Ala Gly Ile Tyr Val Phe
        355                 360                 365

Gly Pro Gly Val Phe Gly Pro Tyr Gly Pro Gly Ala Ser Gly Pro Gly
    370                 375                 380

Val Phe Gly Pro Tyr Gly Pro Gly Ala Ser Ala Ala Ala Ala Ala Gly
385                 390                 395                 400

Pro Gly Ile Tyr Gly Pro Gly Val Phe Gly Pro Ser Ala Ser Ala Ala
                405                 410                 415

Ala Ala Ala Gly Ile Tyr Gly Ser Gly Pro Gly Ile Tyr Gly Pro Tyr
            420                 425                 430

Gly Pro Gly Ile Ser Gly Pro Gly Ser Gly Val Phe Gly Ile Gly Pro
        435                 440                 445

Tyr Gly Pro Gly Ala Ser Ala Ala Ala Ala Ala Gly Ile Tyr Gly Pro
    450                 455                 460

Gly Val Phe Gly Pro Tyr Gly Pro Gly Ile Ser Ala Ala Ala Ala Ala
465                 470                 475                 480

Gly Pro Gly Ser Gly Ile Tyr Gly Pro Gly Ala Ser Gly Ile Asn Gly
                485                 490                 495

Pro Gly Ser Gly Ile Tyr Gly Pro Gly Val Phe Gly Pro Gly Ile Ser
            500                 505                 510

Ala Ala Ala Ala Ala Gly Ile Tyr Val Phe Gly Pro Gly Val Phe Gly
        515                 520                 525

Pro Tyr Gly Pro Gly Ala Ser Ala Ala Ala Ala Ala Gly Ile Tyr Gly
    530                 535                 540

Ser Gly Pro Gly Val Phe Gly Pro Tyr Gly Pro Gly Ile Ser Gly Ser
545                 550                 555                 560

Gly Val Phe Gly Pro Gly Val Phe Gly Pro Tyr Ala Ser Ala Ala Ala
                565                 570                 575

Ala Ala Gly Pro Gly Ser Gly Val Phe Gly Pro Gly Ala Ser Gly Pro
            580                 585                 590

Gly Val Phe Gly Pro Tyr Gly Pro Gly Ala Ser Ala Ala Ala Ala Ala
        595                 600                 605

Gly Ile Asn Gly Pro Gly Ser Gly Val Phe Gly Pro Gly Ile Ser Gly
    610                 615                 620

Ile Tyr Gly Pro Gly Val Phe Gly Pro Gly Val Phe Gly Pro Gly Ser
625                 630                 635                 640

Ser Ala Ala Ala Ala Ala Gly Pro Gly Ile Tyr Gly Pro Gly Val Phe
                645                 650                 655

Gly Pro Ser Ala Ser Ala Ala Ala Ala Ala Gly Pro Gly Ser Gly Val
            660                 665                 670

Phe Gly Pro Gly Ala Ser Gly Ile Tyr Gly Pro Gly Val Phe Gly Pro
        675                 680                 685

Gly Val Phe Gly Pro Gly Ser Ser Ala Ala Ala Ala Ala Gly Ile Tyr
    690                 695                 700

Gly Ser Gly Pro Gly Val Phe Gly Pro Tyr Gly Ser Ala Ala Ala Ala
705                 710                 715                 720

Ala Gly Pro Gly Ser Gly Ile Tyr Gly Ile Gly Pro Tyr Gly Pro Gly
                725                 730                 735

Ala Ser Gly Pro Gly Ile Tyr Gly Pro Gly Val Phe Gly Pro Ser Ala
            740                 745                 750

Ser Ala Ala Ala Ala Ala Gly Ser Gly Val Phe Gly Pro Gly Ile Tyr
        755                 760                 765

Gly Pro Tyr Ala Ser Ala Ala Ala Ala Ala Gly Ile Tyr Gly Ser Gly
    770                 775                 780

Pro Gly Val Phe Gly Pro Tyr Gly Pro Gly Ile Ser Gly Ser Gly Val
785                 790                 795                 800

Phe Gly Pro Gly Val Phe Gly Pro Tyr Ala Ser Ala Ala Ala Ala Ala
                805                 810                 815

Gly Pro Gly Val Phe Gly Pro Tyr Gly Pro Gly Ser Ser Ala Ala Ala
            820                 825                 830

Ala Ala Gly Ile Tyr Gly Tyr Gly Pro Gly Val Phe Gly Pro Tyr Gly
        835                 840                 845

Pro Gly Ala Ser Gly Ile Asn Gly Pro Gly Ser Gly Ile Tyr Gly Pro
    850                 855                 860

Gly Val Phe Gly Pro Gly Ile Ser Ala Ala Ala Ala Ala Gly Pro Gly
865                 870                 875                 880

Val Phe Gly Pro Tyr Gly Pro Gly Ala Ser Ala Ala Ala Ala Ala Gly
                885                 890                 895

Ile Tyr Gly Pro Gly Val Phe Gly Pro Gly Ile Tyr Gly Pro Gly Ser

900                 905                 910

Ser Gly Pro Gly Val Phe Gly Pro Tyr Gly Pro Gly Ser Ser Ala Ala
        915                 920                 925

Ala Ala Ala Gly Ile Tyr Gly Pro Gly Val Phe Gly Pro Tyr Gly Pro
    930                 935                 940

Gly Ile Ser Ala Ala Ala Ala Ala Gly Ile Tyr Val Phe Gly Pro Gly
945                 950                 955                 960

Val Phe Gly Pro Tyr Gly Pro Gly Ala Ser Gly Pro Gly Val Phe Gly
                965                 970                 975

Pro Tyr Gly Pro Gly Ala Ser Ala Ala Ala Ala Ala Gly Pro Gly Ile
            980                 985                 990

Tyr Gly Pro Gly Val Phe Gly Pro  Ser Ala Ser Ala Ala  Ala Ala Ala
        995                 1000                 1005

Gly Ile  Tyr Gly Ser Gly Pro  Gly Ile Tyr Gly Pro  Tyr Gly Pro
    1010                 1015                 1020

Gly Ile  Ser Gly Pro Gly Ser  Gly Val Phe Gly Ile  Gly Pro Tyr
    1025                 1030                 1035

Gly Pro  Gly Ala Ser Ala Ala  Ala Ala Ala Gly Ile  Tyr Gly Pro
    1040                 1045                 1050

Gly Val  Phe Gly Pro Tyr Gly  Pro Gly Ile Ser Ala  Ala Ala Ala
    1055                 1060                 1065

Ala Gly  Pro Gly Ser Gly Ile  Tyr Gly Pro Gly Ala  Ser Gly Ile
    1070                 1075                 1080

Asn Gly  Pro Gly Ser Gly Ile  Tyr Gly Pro Gly Val  Phe Gly Pro
    1085                 1090                 1095

Gly Ile  Ser Ala Ala Ala Ala  Ala Gly Ile Tyr Val  Phe Gly Pro
    1100                 1105                 1110

Gly Val  Phe Gly Pro Tyr Gly  Pro Gly Ala Ser Ala  Ala Ala Ala
    1115                 1120                 1125

Ala Gly  Ile Tyr Gly Ser Gly  Pro Gly Val Phe Gly  Pro Tyr Gly
    1130                 1135                 1140

Pro Gly  Ile Ser Gly Ser Gly  Val Phe Gly Pro Gly  Val Phe Gly
    1145                    1150                    1155

Pro Tyr  Ala Ser Ala Ala Ala  Ala Ala Gly Pro Gly  Ser Gly Val
    1160                    1165                    1170

Phe Gly  Pro Gly Ala Ser
    1175

<210>  44
<211>  1190
<212>  PRT
<213>  Artificial Sequence

<220>
<223>  PRT966

<400>  44
   Met His His His His His His Ser Ser Gly Ser Ser Gly Pro Gly Val
   1               5                   10                  15
   Phe Gly Pro Tyr Gly Pro Gly Ala Ser Ala Ala Ala Ala Ala Gly Ile
               20                  25                  30
   Asn Gly Pro Gly Ser Gly Val Phe Gly Pro Gly Ile Ser Gly Ile Tyr
           35                  40                  45
   Gly Pro Gly Val Phe Gly Pro Gly Val Phe Gly Pro Gly Ser Ser Ala
       50                  55                  60
   Ala Ala Ala Ala Gly Pro Gly Ile Tyr Gly Pro Gly Val Phe Gly Pro
   65                  70                  75                  80
   Ser Ala Ser Ala Ala Ala Ala Ala Gly Pro Gly Ser Gly Val Phe Gly
                   85                  90                  95
   Pro Gly Ala Ser Gly Ile Tyr Gly Pro Gly Val Phe Gly Pro Gly Val
               100                 105                 110
   Phe Gly Pro Gly Ser Ser Ala Ala Ala Ala Ala Gly Ile Tyr Gly Ser
           115                 120                 125
   Gly Pro Gly Val Phe Gly Pro Tyr Gly Ser Ala Ala Ala Ala Ala Gly
       130                 135                 140
   Pro Gly Ser Gly Ile Tyr Gly Ile Gly Pro Tyr Gly Pro Gly Ala Ser
   145                 150                 155                 160
   Gly Pro Gly Ile Tyr Gly Pro Gly Val Phe Gly Pro Ser Ala Ser Ala
   165 170 175
   Ala Ala Ala Ala Gly Ser Gly Val Phe Gly Pro Gly Ile Tyr Gly Pro
   180 185 190
   Tyr Ala Ser Ala Ala Ala Ala Ala Gly Ile Tyr Gly Ser Gly Pro Gly
   195 200 205
   Val Phe Gly Pro Tyr Gly Pro Gly Ile Ser Gly Ser Gly Val Phe Gly
   210 215 220
   Pro Gly Val Phe Gly Pro Tyr Ala Ser Ala Ala Ala Ala Ala Gly Pro
   225 230 235 240
   Gly Val Phe Gly Pro Tyr Gly Pro Gly Ser Ser Ala Ala Ala Ala Ala
   245 250 255
   Gly Ile Tyr Gly Tyr Gly Pro Gly Val Phe Gly Pro Tyr Gly Pro Gly
   260 265 270
   Ala Ser Gly Ile Asn Gly Pro Gly Ser Gly Ile Tyr Gly Pro Gly Val
   275 280 285
   Phe Gly Pro Gly Ile Ser Ala Ala Ala Ala Ala Gly Pro Gly Val Phe
   290 295 300
   Gly Pro Tyr Gly Pro Gly Ala Ser Ala Ala Ala Ala Ala Gly Ile Tyr
   305 310 315 320
   Gly Pro Gly Val Phe Gly Pro Gly Ile Tyr Gly Pro Gly Ser Ser Gly
   325 330 335
   Pro Gly Val Phe Gly Pro Tyr Gly Pro Gly Ser Ser Ala Ala Ala Ala
   340 345 350
   Ala Gly Ile Tyr Gly Pro Gly Val Phe Gly Pro Tyr Gly Pro Gly Ile
   355 360 365
   Ser Ala Ala Ala Ala Ala Gly Ile Tyr Val Phe Gly Pro Gly Val Phe
   370 375 380
   Gly Pro Tyr Gly Pro Gly Ala Ser Gly Pro Gly Val Phe Gly Pro Tyr
   385 390 395 400
   Gly Pro Gly Ala Ser Ala Ala Ala Ala Ala Gly Pro Gly Ile Tyr Gly
   405 410 415
   Pro Gly Val Phe Gly Pro Ser Ala Ser Ala Ala Ala Ala Ala Gly Ile
               420                 425                 430
   Tyr Gly Ser Gly Pro Gly Ile Tyr Gly Pro Tyr Gly Pro Gly Ile Ser
           435                 440                 445
   Gly Pro Gly Ser Gly Val Phe Gly Ile Gly Pro Tyr Gly Pro Gly Ala
       450                 455                 460
   Ser Ala Ala Ala Ala Ala Gly Ile Tyr Gly Pro Gly Val Phe Gly Pro
   465                 470                 475                 480
   Tyr Gly Pro Gly Ile Ser Ala Ala Ala Ala Ala Gly Pro Gly Ser Gly
                   485                 490                 495
   Ile Tyr Gly Pro Gly Ala Ser Gly Ile Asn Gly Pro Gly Ser Gly Ile
               500                 505                 510
   Tyr Gly Pro Gly Val Phe Gly Pro Gly Ile Ser Ala Ala Ala Ala Ala
           515                 520                 525
   Gly Ile Tyr Val Phe Gly Pro Gly Val Phe Gly Pro Tyr Gly Pro Gly
       530                 535                 540
   Ala Ser Ala Ala Ala Ala Ala Gly Ile Tyr Gly Ser Gly Pro Gly Val
   545                 550                 555                 560
   Phe Gly Pro Tyr Gly Pro Gly Ile Ser Gly Ser Gly Val Phe Gly Pro
                   565                 570                 575
   Gly Val Phe Gly Pro Tyr Ala Ser Ala Ala Ala Ala Ala Gly Pro Gly
               580                 585                 590
   Ser Gly Val Phe Gly Pro Gly Ala Ser Gly Pro Gly Val Phe Gly Pro
           595                 600                 605
   Tyr Gly Pro Gly Ala Ser Ala Ala Ala Ala Ala Gly Ile Asn Gly Pro
       610                 615                 620
   Gly Ser Gly Val Phe Gly Pro Gly Ile Ser Gly Ile Tyr Gly Pro Gly
   625                 630                 635                 640
   Val Phe Gly Pro Gly Val Phe Gly Pro Gly Ser Ser Ala Ala Ala Ala
                   645                 650                 655
   Ala Gly Pro Gly Ile Tyr Gly Pro Gly Val Phe Gly Pro Ser Ala Ser
               660                 665                 670
   Ala Ala Ala Ala Ala Gly Pro Gly Ser Gly Val Phe Gly Pro Gly Ala
           675                 680                 685
   Ser Gly Ile Tyr Gly Pro Gly Val Phe Gly Pro Gly Val Phe Gly Pro
       690                 695                 700
   Gly Ser Ser Ala Ala Ala Ala Ala Gly Ile Tyr Gly Ser Gly Pro Gly
   705                 710                 715                 720
   Val Phe Gly Pro Tyr Gly Ser Ala Ala Ala Ala Ala Gly Pro Gly Ser
                   725                 730                 735
   Gly Ile Tyr Gly Ile Gly Pro Tyr Gly Pro Gly Ala Ser Gly Pro Gly
               740                 745                 750
   Ile Tyr Gly Pro Gly Val Phe Gly Pro Ser Ala Ser Ala Ala Ala Ala
           755                 760                 765
   Ala Gly Ser Gly Val Phe Gly Pro Gly Ile Tyr Gly Pro Tyr Ala Ser
       770                 775                 780
   Ala Ala Ala Ala Ala Gly Ile Tyr Gly Ser Gly Pro Gly Val Phe Gly
   785                 790                 795                 800
   Pro Tyr Gly Pro Gly Ile Ser Gly Ser Gly Val Phe Gly Pro Gly Val
                   805                 810                 815
   Phe Gly Pro Tyr Ala Ser Ala Ala Ala Ala Ala Gly Pro Gly Val Phe
               820                 825                 830
   Gly Pro Tyr Gly Pro Gly Ser Ser Ala Ala Ala Ala Ala Gly Ile Tyr
           835                 840                 845
   Gly Tyr Gly Pro Gly Val Phe Gly Pro Tyr Gly Pro Gly Ala Ser Gly
       850                 855                 860
   Ile Asn Gly Pro Gly Ser Gly Ile Tyr Gly Pro Gly Val Phe Gly Pro
   865                 870                 875                 880
   Gly Ile Ser Ala Ala Ala Ala Ala Gly Pro Gly Val Phe Gly Pro Tyr
                   885                 890                 895
   Gly Pro Gly Ala Ser Ala Ala Ala Ala Ala Gly Ile Tyr Gly Pro Gly
               900                 905                 910
   Val Phe Gly Pro Gly Ile Tyr Gly Pro Gly Ser Ser Gly Pro Gly Val
           915                 920                 925
   Phe Gly Pro Tyr Gly Pro Gly Ser Ser Ala Ala Ala Ala Ala Gly Ile
       930                 935                 940
   Tyr Gly Pro Gly Val Phe Gly Pro Tyr Gly Pro Gly Ile Ser Ala Ala
   945                 950                 955                 960
   Ala Ala Ala Gly Ile Tyr Val Phe Gly Pro Gly Val Phe Gly Pro Tyr
                   965                 970                 975
   Gly Pro Gly Ala Ser Gly Pro Gly Val Phe Gly Pro Tyr Gly Pro Gly
               980                 985                 990
   Ala Ser Ala Ala Ala Ala Ala Gly Pro Gly Ile Tyr Gly Pro Gly Val
           995                 1000                1005
   Phe Gly Pro Ser Ala Ser Ala Ala Ala Ala Ala Gly Ile Tyr Gly
       1010                1015                1020
   Ser Gly Pro Gly Ile Tyr Gly Pro Tyr Gly Pro Gly Ile Ser Gly
       1025                1030                1035
   Pro Gly Ser Gly Val Phe Gly Ile Gly Pro Tyr Gly Pro Gly Ala
       1040                1045                1050
   Ser Ala Ala Ala Ala Ala Gly Ile Tyr Gly Pro Gly Val Phe Gly
       1055                1060                1065
   Pro Tyr Gly Pro Gly Ile Ser Ala Ala Ala Ala Ala Gly Pro Gly
       1070                1075                1080
   Ser Gly Ile Tyr Gly Pro Gly Ala Ser Gly Ile Asn Gly Pro Gly
       1085                1090                1095
   Ser Gly Ile Tyr Gly Pro Gly Val Phe Gly Pro Gly Ile Ser Ala
       1100                1105                1110
   Ala Ala Ala Ala Gly Ile Tyr Val Phe Gly Pro Gly Val Phe Gly
       1115                1120                1125
   Pro Tyr Gly Pro Gly Ala Ser Ala Ala Ala Ala Ala Gly Ile Tyr
       1130                1135                1140
   Gly Ser Gly Pro Gly Val Phe Gly Pro Tyr Gly Pro Gly Ile Ser
       1145                1150                1155
   Gly Ser Gly Val Phe Gly Pro Gly Val Phe Gly Pro Tyr Ala Ser
   1160                    1165                    1170
   Ala Ala  Ala Ala Ala Gly Pro  Gly Ser Gly Val Phe  Gly Pro Gly
       1175                    1180                    1185
   Ala Ser
       1190

## Description

### Technical Field

The present invention relates to a muscle tissue-regenerating agent.

### Background Art

Patent Literature 1 discloses an implantable prosthesis for anatomical defects such as tissue or muscle defects. This prosthesis allows tissue growth within the prosthesis, enables adhesion with a tissue or muscle, and also reinforces a defect area.

### Citation List

### Patent Literature

Patent Literature 1: WO 2005/103158 A

### Summary of Invention

### Technical Problem

The prosthesis disclosed in Patent Literature 1 has a plurality of therapeutically effective features such as cellular adhesiveness and reduction of postoperative adhesions. However, myoblast differentiation required in the process of proliferating myocytes and other steps have not been verified, and the prosthesis leaves room for improvement in terms of therapeutic effects.

In addition, some prostheses include polypropylene or other materials for reinforcement. Those materials remain in the body semipermanently without being biodegraded, and the residual materials may cause unwanted foreign-body responses (such as thrombus, infection, thickening, and encapsulation). Furthermore, using a prosthesis for a growing infant may require removal of the prosthesis in the process of his/her growth. Therefore, there is a demand for the development of a material that regenerates a muscle tissue using a biodegradable material.

An object of the invention is to provide a muscle tissue-regenerating agent containing a fibroin protein.

### Solution to Problem

The invention relates to, for example, the following agent and method.
[1] A muscle tissue-regenerating agent containing a modified fibroin protein.
[2] The muscle tissue-regenerating agent according to [1], in which the muscle tissue-regenerating agent is a prosthetic and regenerating agent for a muscle tissue defect.
[3] The muscle tissue-regenerating agent according to [1] or [2], in which the muscle tissue-regenerating agent is an activator for a satellite cell in a muscle tissue.
[4] The muscle tissue-regenerating agent according to any one of [1] to [3], in which the muscle tissue-regenerating agent is an agent for differentiating a myoblast into a myocyte.
[5] The muscle tissue-regenerating agent according to any one of [1] to [4], in which the muscle tissue-regenerating agent is a myoblast adhesive.
[6] The muscle tissue-regenerating agent according to any one of [1] to [5], in which the modified fibroin protein is a recombinant fibroin protein, and the muscle tissue-regenerating agent further contains a substance derived from a host used in production of the recombinant fibroin protein.
[7] The muscle tissue-regenerating agent according to [6], in which the host is a prokaryote.
[8] The muscle tissue-regenerating agent according to any one of [1] to [7], in which the muscle tissue-regenerating agent is biodegraded within 80 days in a mammalian living organism.
[9] The muscle tissue-regenerating agent according to any one of [1] to [8], in which the muscle tissue-regenerating agent further contains one or both of an alcohol and dimethyl sulfoxide in amount of 0.01 to 50 mass%.
[10] The muscle tissue-regenerating agent according to any one of [1] to [9], in which the modified fibroin protein is a modified spider silk fibroin protein.
[11] The muscle tissue-regenerating agent according to any one of [1] to [10], in which the modified fibroin protein has an average hydropathy index (average HI) of 0 or less.
[12] The muscle tissue-regenerating agent according to any one of [1] to [11], in which the muscle tissue-regenerating agent is a therapeutic agent for one selected from abdominal incisional hernia, diaphragmatic hernia, inguinal hernia, site of amputation stump plasty, decubitus, and wound.
[13] The muscle tissue-regenerating agent according to any one of [1] to [12], in which the muscle tissue-regenerating agent is formed into any one of a film, sheet, fiber, resin body, powder, and gel or formed by any combination of these forms.
[14] The muscle tissue-regenerating agent according to any one of [1] to [13], in which the muscle tissue-regenerating agent is formed into a film or a sheet or formed by a combination of these forms.
[15] A treatment of a condition in need of muscle tissue regeneration, the treatment involving bringing the muscle tissue-regenerating agent according to any one of [1] to [14] into contact with a site having a deficient myocyte in a non-human animal.
[16] The muscle tissue-regenerating agent according to any one of [1] to [14], in which the muscle tissue-regenerating agent contains 5 mass% or more and 100 mass% or less of the modified fibroin protein with respect to the total mass of the muscle tissue-regenerating agent.
[17] The treatment according to [15], in which the muscle tissue-regenerating agent contains 5 mass% or more and 100 mass% or less of the modified fibroin protein with respect to the total mass of the muscle tissue-regenerating agent.

### Advantageous Effects of Invention

According to the invention, there is provided a muscle tissue-regenerating agent containing a modified fibroin protein. The muscle tissue-regenerating agent plays roles of prosthesis and regeneration of a defect and is degradable in vivo after the regeneration of a tissue.

### Brief Description of Drawings

Fig. 1 is a schematic view illustrating an example of a domain sequence of a spider silk fibroin.
Fig. 2 is a schematic view illustrating an example of a domain sequence of a spider silk fibroin.
Fig. 3 is a schematic view illustrating an example of a domain sequence of a spider silk fibroin.
Fig. 4 is a 6-week postoperative photo of a tissue site having an abdominal wall defect reinforced. High-grade inflammation 1 and a residual film 2 are observed. Formation of a thin skeletal muscle fiber bundle 3 is also observed at the boundary with a known skeletal muscle.
Fig. 5 is a 6-week postoperative photo of the tissue site having the abdominal wall defect reinforced. The background shows the high-grade inflammation, and a portion surrounded by a circle indicates a foreign-body response. In addition, the residual film 2 is observed.
Fig. 6 is a 12-week postoperative photo of a tissue site having an abdominal wall defect reinforced. An arrow indicates a defect.
Fig. 7 is a 12-week postoperative photo of the tissue site having the abdominal wall defect reinforced, enlarging an area surrounding the defect.
Fig. 8 is a 12-week postoperative photo of a tissue site of a mouse having an abdominal wall defect reinforced.
Fig. 9 is a 15-week postoperative photo of a tissue site of a mouse having an abdominal wall defect reinforced.

### Description of Embodiments

Hereinafter, embodiments of the invention will be described in detail. However, the invention is not limited to the following embodiments.

### <Modified Fibroin Protein>

A "modified fibroin protein" herein represents a fibroin protein having an amino acid sequence different from that of a naturally derived fibroin protein. The modified fibroin protein herein may be a recombinant fibroin protein produced from a microorganism or the like by genetic recombination or may be a synthetic fibroin protein produced by synthesis. Fibroin proteins (including naturally derived fibroin proteins and modified fibroin proteins) hardly cause immune responses and are preferable for medical use.

The modified fibroin protein according to this embodiment may be, for example, a protein including a domain sequence represented by Formula 1: [(A)ₙ motif-REP]ₘ or Formula 2: [(A)ₙ motif-REP]ₘ-(A)ₙ motif. In the domain sequence of the modified spider silk fibroin according to this embodiment, another amino acid sequence may be added to either or both of the N-terminus and the C-terminus (N-terminal sequence and C-terminal sequence). The N-terminal sequence and the C-terminal sequence are typically, but are not limited to, regions not having repeats of fibroin-specific amino acid motifs and having about 100 amino acid residues.

The "domain sequence" herein represents an amino acid sequence which produces a crystalline region (typically corresponding to an (A)ₙ motif of the amino acid sequence) and a non-crystalline region (typically corresponding to an REP of the amino acid sequence) specific to a fibroin and is represented by Formula 1: [(A)ₙ motif-REP]ₘ or Formula 2: [(A)ₙ motif-REP]ₘ-(A)ₙ motif. Herein, the "(A)ₙ motif" represents an amino acid sequence mainly having alanine residues, and the number of amino acid residues is 2 to 27. The number of amino acid residues in the (A)ₙ motif may be 2 to 20, 4 to 27, 4 to 20, 8 to 20, 10 to 20, 4 to 16, 8 to 16, or 10 to 16. The number of alanine residues may account for 40% or more, 60% or more, 70% or more, 80% or more, 83% or more, 85% or more, 86% or more, 90% or more, 95% or more, or 100% (meaning that the (A)ₙ motif consists of alanine residues) to the total number of amino acid residues in the (A)ₙ motif. At least seven of a plurality of (A)ₙ motifs in the domain sequence may consist of alanine residues. The "REP" represents an amino acid sequence consisting of 2 to 200 amino acid residues. The "REP" may be an amino acid sequence consisting of 10 to 200 amino acid residues. The symbol "m" represents an integer from 2 to 300 and may be an integer from 10 to 300. A plurality of (A)ₙ motifs may be identical amino acid sequences or different amino acid sequences. A plurality of REPs may be identical amino acid sequences or different amino acid sequences.

The modified fibroin may be a fibroin having an amino acid sequence modified based on an amino acid sequence of a naturally derived fibroin (for example, a fibroin having an amino acid sequence modified by modification of a cloned gene sequence of a naturally derived spider silk fibroin) or a fibroin artificially designed and synthesized independently of the naturally derived fibroin (for example, a fibroin having a desired amino acid sequence by chemical synthesis of a nucleic acid that encodes a designed amino acid sequence).

The modified fibroin protein may be a modified spider silk fibroin protein. The modified spider silk fibroin protein is obtained by, for example, modification of an amino acid sequence corresponding to modification of a cloned gene sequence of a naturally derived fibroin in such a manner that at least one amino acid residue is substituted, deleted, inserted, and/or added. The substitution, deletion, insertion, and/or addition of amino acid residues are performed by site-directed mutagenesis and other methods known to those skilled in the art. Specifically, the substitution, deletion, insertion, and/or addition of amino acid residues are performed according to the methods described in Nucleic Acid Res. 10, 6487 (1982) and Methods in Enzymology, 100, 448 (1983).

The "modified spider silk fibroin" herein represents a spider silk fibroin having an amino acid sequence different from that of a naturally derived spider silk fibroin, and the "naturally derived spider silk fibroin" herein represents a spider silk fibroin having an amino acid sequence identical to that of a naturally derived spider silk fibroin.

Examples of the naturally derived spider silk fibroin include spider silk fibroins produced by spiders such as major ampullate dragline silk proteins, flagelliform silk proteins, and minor ampullate gland proteins. The major ampullate dragline silk has repetitive regions of crystalline and non-crystalline regions (or amorphous region) and has a combination of high stress and stretchability. The flagelliform silk of spiders has no crystalline regions but has repetitive regions of non-crystalline regions. The flagelliform silk has lower stress than that of the major ampullate dragline silk but is high in stretchability.

The major ampullate dragline silk proteins are produced in the major ampullate gland of a spider and have excellent toughness. Examples of the major ampullate dragline silk proteins include major ampullate spidroins MaSp1 and MaSp2 derived from *Nephila clavipes* and ADF3 and ADF4 derived from *Araneus diadematus.* ADF3 is one of the two major ampullate dragline silk proteins of *Araneus diadematus.* ADF3-derived spider silk proteins are relatively easy to synthesize and have excellent strength, elongation, and toughness.

The flagelliform silk proteins are produced in the flagelliform gland of a spider. Examples of the flagelliform silk protein include *Nephila clavipes*-derived flagelliform silk proteins.

Examples of the spider silk fibroins produced by spiders include spider silk proteins produced by spiders belonging to the genus *Araneus* such as *Araneus ventricosus, Araneus diadematus, Araneus pinguis, Araneus pentagrammicus,* and *Araneus nojimai;* spiders belonging to the genus *Neoscona* such as *Neoscona scylla, Neoscona nautica, Neoscona adianta,* and *Neoscona scylloides;* spiders belonging to the genus *Pronus* such as *Pronous minutus;* spiders belonging to the genus *Cyrtarachne* such as *Cyrtarachne bufo* and *Cyrtarachne akirai;* spiders belonging to the genus *Gasteracantha* such as *Gasteracantha kuhli* and *Gasteracantha mammosa;* spiders belonging to the genus *Ordgarius* such as *Ordgarius hobsoni* and *Ordgarius sexspinosus;* spiders belonging to the genus *Argiope* such as *Argiope amoena, Argiope minuta,* and *Argiope bruennichi;* spiders belonging to the genus *Arachnura* such as *Arachnura logio;* spiders belonging to the genus *Acusilas* such as *Acusilas coccineus;* spiders belonging to the genus *Cytophora* such as *Cyrtophora ikomosanensis, Cyrtophora exanthematica,* and *Cyrtophora unicolor;* spiders belonging to the genus *Poltys* such as *Poltys illepidus;* spiders belonging to the genus *Cyclosa* such as *Cyclosa octotuberculata, Cyclosa sedeculata, Cyclosa vallata,* and *Cyclosa atrata;* spiders belonging to the genus *Chorizopes* such as *Chorizopes nipponicus;* and spider silk proteins produced by spiders belonging to the family *Tetragnathidae,* for example, spiders belonging to the genus *Tetragnatha* such as *Tetragnatha praedonia, Tetragnatha maxillosa, Tetragnatha extensa,* and *Tetragnatha squamata;* spiders belonging to the genus *Leucauge* such as *Leucauge magnifica, Leucauge blanda,* and *Leucauge subblanda;* spiders belonging to the genus *Nephila* such as *Nephila clavata* and *Nephila pilipes;* spiders belonging to the genus *Menosira* such as *Menosira ornata;* spiders belonging to the genus *Dyschiriognatha* such as *Dyschiriognatha tenera;* spiders belonging to the genus *Latrodectus* such as *Latrodectus mactans, Latrodectus hasselti, Latrodectus geometricus,* and *Latrodectus tredecimguttatus;* and spiders belonging to the genus *Euprosthenops.*

More specific examples of the spider silk proteins produced by spiders include fibroin-3 (adf-3) [derived from *Araneus diadematus*] (GenBank Accession No. AAC47010 (amino acid sequence), U47855 (nucleotide sequence)), fibroin-4 (adf-4) [derived from *Araneus diadematus*] (GenBank Accession No. AAC47011 (amino acid sequence), U47856 (nucleotide sequence)), dragline silk protein spidroin 1 [derived from *Nephila clavipes*] (GenBank Accession No. AAC04504 (amino acid sequence), U37520 (nucleotide sequence)), major ampullate spidroin 1 [derived from *Latrodectus hesperus*] (GenBank Accession No. ABR68856 (amino acid sequence), EF595246 (nucleotide sequence)), dragline silk protein spidroin 2 [derived from *Nephila clavata*] (GenBank Accession No. AAL32472 (amino acid sequence), AF441245 (nucleotide sequence)), major ampullate spidroin 1 [derived from *Euprosthenops australis*] (GenBank Accession No. CAJ00428 (amino acid sequence), AJ973155 (nucleotide sequence)), major ampullate spidroin 2 [*Euprosthenops australis*] (GenBank Accession No. CAM32249.1 (amino acid sequence), AM490169 (nucleotide sequence)), minor ampullate silk protein 1 [*Nephila clavipes*] (GenBank Accession No. AAC14589.1 (amino acid sequence)), minor ampullate silk protein 2 [*Nephila clavipes*] (GenBank Accession No. AAC14591.1 (amino acid sequence)), and minor ampullate spidroin-like protein [*Nephilengys cruentata*] (GenBank Accession No. ABR37278.1 (amino acid sequence).

Specific examples of the modified spider silk fibroin include a modified spider silk fibroin derived from a major ampullate dragline silk protein produced in the major ampullate gland of a spider (first modified spider silk fibroin), a modified spider silk fibroin having a reduced glycine residue content (second modified spider silk fibroin), a modified spider silk fibroin having a reduced (A)ₙ motif content (third modified spider silk fibroin), a modified spider silk fibroin having a reduced glycine residue content and the (A)ₙ motif content reduced (fourth modified spider silk fibroin), a modified spider silk fibroin having a domain sequence including a region locally having a high hydropathy index (fifth modified spider silk fibroin), and a modified spider silk fibroin having a domain sequence having a reduced glutamine residue content (sixth modified spider silk fibroin).

An example of the modified spider silk fibroin derived from a major ampullate dragline silk protein produced in the major ampullate gland of a spider (first modified spider silk fibroin) includes a protein including a domain sequence represented by Formula 1: [(A)ₙ motif-REP]ₘ. In the first modified spider silk fibroin, "n" in Formula 1 is preferably an integer from 3 to 20, more preferably an integer from 4 to 20, still more preferably an integer from 8 to 20, still more preferably an integer from 10 to 20, still more preferably an integer from 4 to 16, particularly preferably an integer from 8 to 16, and most preferably an integer from 10 to 16. In the first modified spider silk fibroin, the number of amino acid residues included in the REP in Formula 1 is preferably 10 to 200, more preferably 10 to 150, and still more preferably 20 to 100, and still more preferably 20 to 75. In the first modified spider silk fibroin, the total number of glycine residues, serine residues, and alanine residues included in an amino acid sequence represented by Formula 1: [(A)ₙ motif-REP]ₘ preferably accounts for 40% or more, more preferably 60% or more, and still more preferably 70% or more of the total number of amino acid residues.

The first modified spider silk fibroin may be a protein including units of the amino acid sequence represented by Formula 1: [(A)ₙ motif-REP]ₘ and having a C-terminal sequence corresponding to the amino acid sequence of any one of SEQ ID NOs: 1 to 3 or an amino acid sequence having a homology of 90% or more to the amino acid sequence of any one of SEQ ID NOs: 1 to 3.

The amino acid sequence of SEQ ID NO: 1 is identical to an amino acid sequence consisting of 50 amino acid residues included in the C-terminus of the amino acid sequence of ADF3 (GI: 1263287, NCBI). The amino acid sequence of SEQ ID NO: 2 is identical to the amino acid sequence of SEQ ID NO: 1 except that 20 amino acid residues are removed from the C-terminus. The amino acid sequence of SEQ ID NO: 3 is identical to the amino acid sequence of SEQ ID NO: 1 except that 29 amino acid residues are removed from the C-terminus.

More specific examples of the first modified spider silk fibroin include (1-i) a modified spider silk fibroin having the amino acid sequence of SEQ ID NO: 4 and (1-ii) a modified spider silk fibroin having an amino acid sequence having a sequence identity of 90% or more to the amino acid sequence of SEQ ID NO: 4. The sequence identity is preferably 95% or more.

The amino acid sequence of SEQ ID NO: 4 is obtained by the following mutation. That is, in the amino acid sequence of ADF3 having the N-terminus to which an amino acid sequence including a start codon, a His10 tag, and a human rhinovirus 3C protease (HRV3C protease) recognition site (SEQ ID NO: 5) are added, the first to thirteenth repetitive regions are roughly doubled and the translation ends at the 1154th amino acid residue. The C-terminal amino acid sequence of the amino acid sequence of SEQ ID NO: 4 is identical to the amino acid sequence of SEQ ID NO: 3.

The modified spider silk fibroin (1-i) may have the amino acid sequence of SEQ ID NO: 4.

A domain sequence of the modified spider silk fibroin having a reduced glycine residue content (second modified spider silk fibroin) has the amino acid sequence of the naturally derived spider silk fibroin except that the glycine residue content is reduced. The second modified spider silk fibroin may have the amino acid sequence of the naturally derived spider silk fibroin except that at least one glycine residue in an REP is substituted by another amino acid residue.

The domain sequence of the second modified spider silk fibroin may have the amino acid sequence of the naturally derived spider silk fibroin except that one glycine residue in at least one motif sequence selected from GGX and GPGXX in an REP is substituted by another amino acid residue (where G is glycine residue, P is proline residue, and X is amino acid residue other than glycine).

In the second modified spider silk fibroin, a proportion of motif sequences where the glycine residue is substituted with another amino acid residue may be 10% or more of the total motif sequences.

The second modified spider silk fibroin may have a domain sequence represented by Formula 1: [(A)ₙ motif-REP]ₘ and may have an amino acid sequence in which z/w is 30% or more, 40% or more, 50% or more, or 50.9% or more where "z" represents the total number of amino acid residues in amino acid sequences consisting of XGX (X is an amino acid residue other than glycine) included in all REPs in the domain sequence excluding a range from the (A)ₙ motif closest to the C-terminus of the domain sequence to the C-terminus, and "w" represents the total number of amino acid residues included in the domain sequence excluding the range from the (A)ₙ motif closest to the C-terminus of the domain sequence to the C-terminus. The number of alanine residues in an (A)ₙ motif may account for 83% or more, preferably 86% or more, more preferably 90% or more, still more preferably 95% or more, and still more preferably 100% (meaning that the (A)ₙ motif consists of alanine residues) to the total number of amino acid residues.

In the second modified spider silk fibroin, a proportion of amino acid sequences consisting of XGX is preferably increased by substituting one glycine residue of a GGX motif by another amino acid residue. In the second modified fibroin, a proportion of amino acid sequences consisting of GGX in the domain sequence is preferably 30% or less, more preferably 20% or less, still more preferably 10% or less, still more preferably 6% or less, still more preferably 4% or less, and particularly preferably 2% or less. A proportion of amino acid sequences consisting of GGX in the domain sequence is calculated by a method similar to the following method for calculating a proportion of amino acid sequences consisting of XGX (z/w).

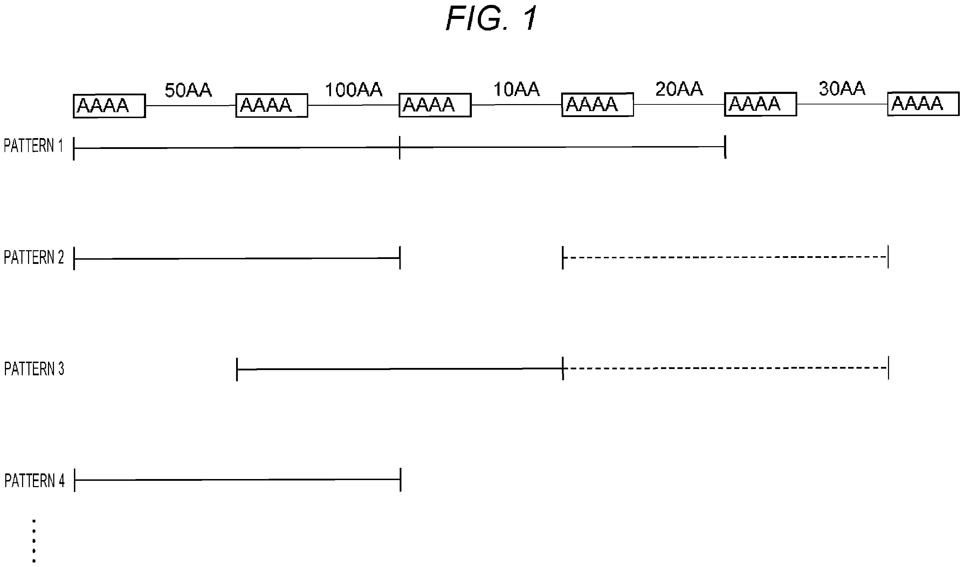

The method for calculating z/w will be described in more detail. First, in a spider silk fibroin (modified spider silk fibroin) having a domain sequence represented by Formula 1: [(A)ₙ motif-REP]ₘ, amino acid sequences consisting of XGX are extracted from all REPs of the domain sequence excluding the range from the (A)ₙ motif closest to the C-terminus of the domain sequence to the C-terminus. The total number of amino acid residues included in XGX is represented by "z". For example, when 50 amino acid sequences consisting of XGX are extracted (no overlap), "z" is 50 × 3 = 150. In addition, for example, in an amino acid sequence consisting of XGXGX, one X (X in the center) is included in two XGXs, and the overlapping portion is subtracted to calculate "z" (that is, there are 5 amino acid residues in XGXGX). The symbol "w" represents the total number of amino acid residues in the domain sequence excluding the range from the (A)ₙ motif closest to the C-terminus of the domain sequence to the C-terminus. For example, in the domain sequence shown in Fig. 1, w is 4 + 50 + 4 + 100 + 4 + 10 + 4 + 20 + 4 + 30 = 230 (excluding the (A)ₙ motif closest to the C-terminus). Next, "z" is divided by "w" to calculate z/w (%).

In the second modified spider silk fibroin, z/w is preferably 50.9% or more, more preferably 56.1% or more, still more preferably 58.7% or more, still more preferably 70% or more, and still more preferably 80% or more. The upper limit of z/w is not particularly limited but may be, for example, 95% or less.

The second modified spider silk fibroin is obtained by, for example, modifying the cloned gene sequence of the naturally derived spider silk fibroin in such a manner that at least part of a nucleotide sequence that encodes a glycine residue is substituted so as to encode another amino acid residue. In this case, one glycine residue in a GGX motif and GPGXX motif may be selected as a glycine residue to be modified or may be substituted so that z/w becomes 50.9% or more. Alternatively, the second modified spider silk fibroin is obtained by, for example, designing an amino acid sequence that satisfies the above aspect from the amino acid sequence of the naturally derived spider silk fibroin and by chemically synthesizing a nucleic acid that encodes the designed amino acid sequence. In any case, the second modified spider silk fibroin may be modified to have the amino acid sequence of the naturally derived spider silk fibroin except that a glycine residue in an REP is substituted by another amino acid residue and at least one amino acid residue is substituted, deleted, inserted, and/or added.

The other amino acid residue is not particularly limited as long as it is an amino acid residue other than glycine residue but is preferably a hydrophobic amino acid residue such as valine (V) residue, leucine (L) residue, isoleucine (I) residue, methionine (M) residue, proline (P) residue, phenylalanine (F) residue, and tryptophan (W) residue or a hydrophilic amino acid residue such as glutamine (Q) residue, asparagine (N) residue, serine (S) residue, lysine (K) residue, or glutamic acid (E) residue. Among these examples, valine (V) residue, leucine (L) residue, isoleucine (I) residue, phenylalanine (F) residue, and glutamine (Q) residue are more preferable, and glutamine (Q) residue is still more preferable.

More specific examples of the second modified spider silk fibroin include (2-i) a modified spider silk fibroin having the amino acid sequence of SEQ ID NO: 6, SEQ ID NO: 7, SEQ ID NO: 8, or SEQ ID NO: 9 and (2-ii) a modified spider silk fibroin having an amino acid sequence having a sequence identity of 90% or more to the amino acid sequence of SEQ ID NO: 6, SEQ ID NO: 7, SEQ ID NO: 8, or SEQ ID NO: 9.

The modified spider silk fibroin (2-i) will be described. The amino acid sequence of SEQ ID NO: 6 is the same as the amino acid sequence of SEQ ID NO: 10 corresponding to the naturally derived spider silk fibroin except that all GGXs in REPs are substituted by GQX. The amino acid sequence of SEQ ID NO: 7 is the same as the amino acid sequence of SEQ ID NO: 6 except that every two (A)ₙ motifs are deleted from the N-terminus to the C-terminus and one [(A)ₙ motif-REP] is inserted before the C-terminal sequence. The amino acid sequence of SEQ ID NO: 8 is the same as the amino acid sequence of SEQ ID NO: 7 except that two alanine residues are inserted into the C-terminus of each (A)ₙ motif and glutamine (Q) residues are partially substituted by serine (S) residues and amino acids close to the N-terminus are partially deleted so as to be substantially equal to the amino acid sequence of SEQ ID NO: 7 in molecular weight. The amino acid sequence of SEQ ID NO: 9 is the same as the amino acid sequence of SEQ ID NO: 11 except that a His tag is added to the C-terminus of a sequence obtained by repeating a region having 20 domain sequences (where several amino acid residues close to the C-terminus of the region are substituted) four times.

The value of z/w in the amino acid sequence of SEQ ID NO: 10 (corresponding to the naturally derived spider silk fibroin) is 46.8%. The values of z/w in the amino acid sequence of SEQ ID NO: 6, the amino acid sequence of SEQ ID NO: 7, the amino acid sequence of SEQ ID NO: 8, and the amino acid sequence of SEQ ID NO: 9 are 58.7%, 70.1%, 66.1%, and 70.0%, respectively. Furthermore, with a GISA ratio (described later) of 1 : 1.8 to 11.3, the values of x/y in the amino acid sequences of SEQ ID NO: 10, SEQ ID NO: 6, SEQ ID NO: 7, SEQ ID NO: 8, and SEQ ID NO: 9 are 15.0%, 15.0%, 93.4%, 92.7%, and 89.3%, respectively.

The modified spider silk fibroin (2-i) may have the amino acid sequence of SEQ ID NO: 6, SEQ ID NO: 7, SEQ ID NO: 8, or SEQ ID NO: 9.

The modified spider silk fibroin (2-ii) has an amino acid sequence having a sequence identity of 90% or more to the amino acid sequence of SEQ ID NO: 6, SEQ ID NO: 7, SEQ ID NO: 8, or SEQ ID NO: 9. The modified spider silk fibroin (2-ii) is also a protein having a domain sequence represented by Formula 1: [(A)ₙ motif-REP]ₘ. The sequence identity is preferably 95% or more.

The modified spider silk fibroin (2-ii) preferably has a sequence identity of 90% or more to the amino acid sequence of SEQ ID NO: 6, SEQ ID NO: 7, SEQ ID NO: 8, or SEQ ID NO: 9 and has an amino acid sequence in which z/w is 50.9% or more where "z" represents the total number of amino acid residues in amino acid sequences consisting of XGX (X is an amino acid residue other than glycine) included in REPs, and "w" represents the total number of amino acid residues in REPs in the domain sequence.

The second modified spider silk fibroin may have a tag sequence at either or both of the N-terminus and the C-terminus. This enables isolation, immobilization, detection, and visualization of the modified spider silk fibroin.

An example of the tag sequence includes an affinity tag utilizing specific affinity (binding property) for another molecule. A specific example of the affinity tag includes a histidine tag (His tag). His tags are short peptides having about 4 to 10 histidine residues and specifically binding to metal ions such as nickel. Accordingly, it is possible to use a His tag to isolate a modified fibroin by chelating metal chromatography. A specific example of the tag sequence includes the amino acid sequence of SEQ ID NO: 12 (amino acid sequence including a His tag sequence and a hinge sequence).

In addition, it is possible to employ a tag sequence such as glutathione-S-transferase (GST) that specifically binds to glutathione or a maltose binding protein (MBP) that specifically binds to maltose.

An "epitope tag" utilizing an antigen-antibody reaction is employable. Adding a peptide (epitope) exhibiting antigenicity as a tag sequence allows binding of an antibody to the epitope. Examples of the epitope tag include HA tag (peptide sequence of influenza hemagglutinin), myc tag, and FLAG tag. Using the epitope tag facilitates purification of a modified spider silk fibroin with high specificity.

Furthermore, it is possible to use a tag sequence which is cleaved with a specific protease. A protein adsorbed through the tag sequence may be subjected to proteolysis so as to collect a modified spider silk fibroin from which the tag sequence has been cleaved.

More specific examples of the second modified fibroin including a tag sequence include (2-iii) a modified spider silk fibroin having the amino acid sequence of SEQ ID NO: 13, SEQ ID NO: 11, SEQ ID NO: 14, or SEQ ID NO: 15 and (2-iv) a modified spider silk fibroin having an amino acid sequence having a sequence identity of 90% or more to the amino acid sequence of SEQ ID NO: 13, SEQ ID NO: 11, SEQ ID NO: 14, or SEQ ID NO: 15.

The amino acid sequences of SEQ ID NO: 16, SEQ ID NO: 17, SEQ ID NO: 13, SEQ ID NO: 11, SEQ ID NO: 14, and SEQ ID NO: 15 are obtained by adding the amino acid sequence of SEQ ID NO: 12 (including a His tag sequence and a hinge sequence) to the N-termini of the amino acid sequences of SEQ ID NO: 10, SEQ ID NO: 18, SEQ ID NO: 6, SEQ ID NO: 7, SEQ ID NO: 8, and SEQ ID NO: 9, respectively.

The modified spider silk fibroin (2-iii) may have the amino acid sequence of SEQ ID NO: 13, SEQ ID NO: 11, SEQ ID NO: 14, or SEQ ID NO: 15.

The modified spider silk fibroin (2-iv) has an amino acid sequence having a sequence identity of 90% or more to the amino acid sequence of SEQ ID NO: 13, SEQ ID NO: 11, SEQ ID NO: 14, or SEQ ID NO: 15. The modified spider silk fibroin (2-iv) is also a protein having a domain sequence represented by Formula 1: [(A)ₙ motif-REP]ₘ. The sequence identity is preferably 95% or more.

The modified spider silk fibroin (2-iv) preferably has a sequence identity of 90% or more to the amino acid sequence of SEQ ID NO: 13, SEQ ID NO: 11, SEQ ID NO: 14, or SEQ ID NO: 15 and has an amino acid sequence in which z/w is 50.9% or more where "z" represents the total number of amino acid residues in amino acid sequences consisting of XGX (X is an amino acid residue other than glycine) included in REPs, and "w" represents the total number of amino acid residues in REPs in the domain sequence.

The second modified spider silk fibroin may include a secretory signal for releasing a protein produced in a recombinant protein production system to the outside of a host. A sequence of the secretory signal is designed appropriately depending on the type of the host.

A domain sequence of the modified spider silk fibroin having a reduced (A)ₙ motif content (third modified spider silk fibroin) has the amino acid sequence of the naturally derived spider silk fibroin except that the (A)ₙ motif content is reduced. The domain sequence of the third modified fibroin may have the amino acid sequence of the naturally derived spider silk fibroin except that at least one (A)ₙ motif is deleted.

The third modified spider silk fibroin may have the amino acid sequence of the naturally derived spider silk fibroin except that 10 to 40% of (A)ₙ motifs is deleted.

The domain sequence of the third modified spider silk fibroin may have the amino acid sequence of the naturally derived spider silk fibroin except that at least one (A)ₙ motif is deleted per one to three (A)ₙ motifs from the N-terminus to the C-terminus.

The domain sequence of the third modified spider silk fibroin may have the amino acid sequence of the naturally derived spider silk fibroin except that deletion of at least two consecutive (A)ₙ motifs and deletion of one (A)ₙ motif are repeated in this order from the N-terminus to the C-terminus.

The domain sequence of the third modified spider silk fibroin may have an amino acid sequence in which at least every two (A)ₙ motifs are deleted from the N-terminus to the C-terminus.

The third modified spider silk fibroin has a domain sequence represented by Formula 1: [(A)ₙ motif-REP]ₘ and may have an amino acid sequence in which x/y is 20% or more, 30% or more, 40% or more, or 50% or more where, when comparing the number of amino acid residues in REPs of two consecutive [(A)ₙ motif-REP] units sequentially from the N-terminus to the C-terminus, "x" represents the maximum total number of amino acid residues in two consecutive [(A)ₙ motif-REP] units in which a ratio of amino acid residues in one REP to amino acid residues in the other REP (having fewer amino acid residues and defined as 1) is 1.8 to 11.3, and "y" represents the total number of amino acid residues in the domain sequence. The number of alanine residues in an (A)ₙ motif may account for 83% or more, preferably 86% or more, more preferably 90% or more, still more preferably 95% or more, and still more preferably 100% (meaning that the (A)ₙ motif consists of alanine residues) to the total number of amino acid residues.

A method for calculating x/y will be described in more detail with reference to Fig. 1. Fig. 1 shows a domain sequence excluding the N-terminal sequence and the C-terminal sequence from a spider silk fibroin. The domain sequence has a sequence of "(A)ₙ motif-first REP (50 amino acid residues)-(A)ₙ motif-second REP (100 amino acid residues)-(A)ₙ motif-third REP (10 amino acid residues)-(A)ₙ motif-fourth REP (20 amino acid residues)-(A)ₙ motif-fifth REP (30 amino acid residues)-(A)ₙ motif" in this order from the N-terminus (left side).

The two consecutive [(A)ₙ motif-REP] units are sequentially selected from the N-terminus to the C-terminus with no overlap. There may be [(A)ₙ motif-REP] units that are not selected. Fig. 1 shows Pattern 1 (a comparison between the first REP and the second REP, and a comparison between the third REP and the fourth REP), Pattern 2 (a comparison between the first REP and the second REP, and a comparison between the fourth REP and the fifth REP), Pattern 3 (a comparison between the second REP and the third REP, and a comparison between the fourth REP and the fifth REP), and Pattern 4 (a comparison between the first REP and the second REP). Note that [(A)ₙ motif-REP] units may be selected in other ways.

Next, for each pattern, the number of amino acid residues is compared between REPs of the selected two consecutive [(A)ₙ motif-REP] units. Those units are compared by defining one REP having fewer amino acid residues as 1 and by determining a ratio of amino acid residues in the other REP to amino acid residues in the one having fewer amino acid residues. For example, comparing the first REP (50 amino acid residues) and the second REP (100 amino acid residues), a ratio of amino acid residues in the second REP is 100/50 = 2, defining the first REP having fewer amino acid residues as 1. Similarly, comparing the fourth REP (20 amino acid residues) and the fifth REP (30 amino acid residues), a ratio of amino acid residues in the fifth REP is 30/20 = 1.5, defining the fourth REP having fewer amino acid residues as 1.

In Fig. 1, solid lines indicate sets of [(A)ₙ motif-REP] units in which a ratio of amino acid residues in one REP to amino acid residues in the other REP having fewer amino acid residues (defined as 1) is 1.8 to 11.3. Herein, this ratio is referred to as "GISA ratio". Dashed lines indicate sets of [(A)ₙ motif-REP] units in which a ratio of amino acid residues in one REP to amino acid residues in the other REP having fewer amino acid residues (defined as 1) is less than 1.8 or over 11.3.

For each pattern, all amino acid residues in two consecutive [(A)ₙ motif-REP] units indicated by the solid lines are added up (including not only the number of amino acid residues in REPs but also the number of amino acid residues in (A)ₙ motifs). The total values of the patterns are compared, and one that has the highest value (the maximum total value) is defined as "x". In the example shown in Fig. 1, the total value of Pattern 1 is the maximum.

Then, "x" is divided by the total number of amino acid residues "y" of the domain sequence to calculate x/y (%).

In the third modified spider silk fibroin, x/y is preferably 50% or more, more preferably 60% or more, still more preferably 65% or more, still more preferably 70% or more, still more preferably 75% or more, and particularly preferably 80% or more. The upper limit of x/y is not particularly limited but may be, for example, 100% or less. With a GISA ratio of 1 : 1.9 to 11.3, x/y is preferably 89.6% or more. With a GISA ratio of 1 : 1.8 to 3.4, x/y is preferably 77.1% or more. With a GISA ratio of 1 : 1.9 to 8.4, x/y is preferably 75.9% or more. With a GISA ratio of 1 : 1.9 to 4.1, x/y is preferably 64.2% or more.

In a case where the third modified spider silk fibroin is a modified spider silk fibroin in which at least seven of a plurality of (A)ₙ motifs in the domain sequence consists of alanine residues, x/y is preferably 46.4% or more, more preferably 50% or more, still more preferably 55% or more, even still more preferably 60% or more, still further preferably 70% or more, and particularly preferably 80% or more. The upper limit of x/y is not particularly limited as long as the value is 100% or less.

The third modified spider silk fibroin is obtained by, for example, by deleting at least one sequence that encodes an (A)ₙ motif from the cloned gene sequence of the naturally derived spider silk fibroin so that x/y becomes 64.2% or more. Alternatively, the third modified spider silk fibroin is obtained by, for example, designing an amino acid sequence corresponding to the amino acid sequence of the naturally derived spider silk fibroin except that at least one (A)ₙ motif is deleted so that x/y becomes 64.2% or more and by chemically synthesizing a nucleic acid that encodes the designed amino acid sequence. In any case, the third modified spider silk fibroin may be modified to have the amino acid sequence of the naturally derived spider silk fibroin except that an (A)ₙ motif is deleted and at least one amino acid residue is substituted, deleted, inserted, and/or added.

More specific examples of the third modified spider silk fibroin include (3-i) a modified fibroin including the amino acid sequence of SEQ ID NO: 18, SEQ ID NO: 7, SEQ ID NO: 8, or SEQ ID NO: 9 and (3-ii) a modified fibroin having an amino acid sequence having a sequence identity of 90% or more to the amino acid sequence of SEQ ID NO: 18, SEQ ID NO: 7, SEQ ID NO: 8, or SEQ ID NO: 9.

The modified spider silk fibroin (3-i) will be described. The amino acid sequence of SEQ ID NO: 18 is the same as the amino acid sequence of SEQ ID NO: 10 corresponding to the naturally derived spider silk fibroin except that every two (A)ₙ motifs are deleted from the N-terminus to the C-terminus and one [(A)ₙ motif-REP] is inserted before the C-terminal sequence. The amino acid sequence of SEQ ID NO: 7 is the same as the amino acid sequence of SEQ ID NO: 18 except that all GGXs in REPs are substituted by GQX. The amino acid sequence of SEQ ID NO: 8 is the same as the amino acid sequence of SEQ ID NO: 7 except that two alanine residues are inserted into the C-terminus of each (A)ₙ motif and glutamine (Q) residues are partially substituted by serine (S) residues and amino acids close to the N-terminus are partially deleted so as to be substantially equal to the amino acid sequence of SEQ ID NO: 7 in molecular weight. The amino acid sequence of SEQ ID NO: 9 is the same as the amino acid sequence of SEQ ID NO: 11 except that a His tag is added to the C-terminus of a sequence obtained by repeating a region having 20 domain sequences (where several amino acid residues close to the C-terminus of the region are substituted) four times.

With a GISA ratio of 1 : 1.8 to 11.3, the value of x/y in the amino acid sequence of SEQ ID NO: 10 (corresponding to the naturally derived spider silk fibroin) is 15.0%. The values of x/y in the amino acid sequence of SEQ ID NO: 18 and the amino acid sequence of SEQ ID NO: 7 are both 93.4%. The value of x/y in the amino acid sequence of SEQ ID NO: 8 is 92.7%. The value of x/y in the amino acid sequence of SEQ ID NO: 9 is 89.3%. The values of z/w in the amino acid sequences of SEQ ID NO: 10, SEQ ID NO: 18, SEQ ID NO: 7, SEQ ID NO: 8, and SEQ ID NO: 9 are 46.8%, 56.2%, 70.1%, 66.1%, and 70.0%, respectively.

The modified spider silk fibroin (3-i) may have the amino acid sequence of SEQ ID NO: 18, SEQ ID NO: 7, SEQ ID NO: 8, or SEQ ID NO: 9.

The modified spider silk fibroin (3-ii) has an amino acid sequence having a sequence identity of 90% or more to the amino acid sequence of SEQ ID NO: 18, SEQ ID NO: 7, SEQ ID NO: 8, or SEQ ID NO: 9. The modified spider silk fibroin (3-ii) is also a protein having a domain sequence represented by Formula 1: [(A)ₙ motif-REP]ₘ. The sequence identity is preferably 95% or more.

The modified spider silk fibroin (3-ii) preferably has a sequence identity of 90% or more to the amino acid sequence of SEQ ID NO: 18, SEQ ID NO: 7, SEQ ID NO: 8, or SEQ ID NO: 9 and has an amino acid sequence in which x/y is 64.2% or more where, when comparing the number of amino acid residues in REPs of two consecutive [(A)ₙ motif-REP] units sequentially from the N-terminus to the C-terminus, "x" represents the maximum total number of amino acid residues in two consecutive [(A)ₙ motif-REP] units in which a ratio of amino acid residues in one REP to amino acid residues in the other REP (having fewer amino acid residues and defined as 1) is 1.8 to 11.3 (GISA ratio of 1 : 1.8 to 11.3), and "y" represents the total number of amino acid residues in the domain sequence.

The third modified spider silk fibroin may include a tag sequence at either or both of the N-terminus and the C-terminus.

More specific examples of the third modified spider silk fibroin including a tag sequence include (3-iii) a modified spider silk fibroin having the amino acid sequence of SEQ ID NO: 17, SEQ ID NO: 11, SEQ ID NO: 14, or SEQ ID NO: 15 and (3-iv) a modified spider silk fibroin having an amino acid sequence having a sequence identity of 90% or more to the amino acid sequence of SEQ ID NO: 17, SEQ ID NO: 11, SEQ ID NO: 14, or SEQ ID NO: 15.

The amino acid sequences of SEQ ID NO: 16, SEQ ID NO: 17, SEQ ID NO: 13, SEQ ID NO: 11, SEQ ID NO: 14, and SEQ ID NO: 15 are obtained by adding the amino acid sequence of SEQ ID NO: 12 (including a His tag sequence and a hinge sequence) to the N-termini of the amino acid sequences of SEQ ID NO: 10, SEQ ID NO: 18, SEQ ID NO: 6, SEQ ID NO: 7, SEQ ID NO: 8, and SEQ ID NO: 9, respectively.

The modified spider silk fibroin (3-iii) may include the amino acid sequence of SEQ ID NO: 17, SEQ ID NO: 11, SEQ ID NO: 14, or SEQ ID NO: 15.

The modified spider silk fibroin (3-iv) has an amino acid sequence having a sequence identity of 90% or more to the amino acid sequence of SEQ ID NO: 17, SEQ ID NO: 11, SEQ ID NO: 14, or SEQ ID NO: 15. The modified spider silk fibroin (3-iv) is also a protein having a domain sequence represented by Formula 1: [(A)ₙ motif-REP]ₘ. The sequence identity is preferably 95% or more.

The modified spider silk fibroin (3-iv) preferably has a sequence identity of 90% or more to the amino acid sequence of SEQ ID NO: 17, SEQ ID NO: 11, SEQ ID NO: 14, or SEQ ID NO: 15 and has an amino acid sequence in which x/y is 64.2% or more where, when comparing the number of amino acid residues in REPs of two consecutive [(A)ₙ motif-REP] units sequentially from the N-terminus to the C-terminus, "x" represents the maximum total number of amino acid residues in two consecutive [(A)ₙ motif-REP] units in which a ratio of amino acid residues in one REP to amino acid residues in the other REP (having fewer amino acid residues and defined as 1) is 1.8 to 11.3, and "y" represents the total number of amino acid residues in the domain sequence.

The third modified spider silk fibroin may include a secretory signal for releasing a protein produced in a recombinant protein production system to the outside of a host. A sequence of the secretory signal is designed appropriately depending on the type of the host.

A domain sequence of the modified spider silk fibroin having a reduced glycine residue content and a reduced (A)ₙ motif content(fourth modified spider silk fibroin) has the amino acid sequence of the naturally derived spider silk fibroin except that the glycine residue content and the (A)ₙ motif content are reduced. The domain sequence of the fourth modified spider silk fibroin may have the amino acid sequence of the naturally derived spider silk fibroin except that at least one (A)ₙ motif is deleted and at least one glycine residue in an REP is substituted by another amino acid residue. In other words, the fourth modified spider silk fibroin is a modified spider silk fibroin having properties of both the modified spider silk fibroin having a reduced glycine residue content (second modified spider silk fibroin) and the modified spider silk fibroin having a reduced (A)ₙ motif content (third modified spider silk fibroin). Specific aspects of the fourth modified spider silk fibroin and the like are as described above in the second modified spider silk fibroin and the third modified spider silk fibroin.

More specific examples of the fourth modified spider silk fibroin include (4-i) a modified spider silk fibroin having the amino acid sequence of SEQ ID NO: 7, SEQ ID NO: 8, or SEQ ID NO: 9 and (4-ii) a modified spider silk fibroin having an amino acid sequence having a sequence identity of 90% or more to the amino acid sequence of SEQ ID NO: 7, SEQ ID NO: 8, or SEQ ID NO: 9. Specific aspects of the modified spider silk fibroin having the amino acid sequence of SEQ ID NO: 7, SEQ ID NO: 8, or SEQ ID NO: 9 are as described above.

The domain sequence of the modified spider silk fibroin (the fifth modified spider silk fibroin) may have an amino acid sequence including a region locally having a high hydropathy index which corresponds to the amino acid sequence of the naturally derived spider silk fibroin except that at least one amino acid residue in an REP is substituted by an amino acid residue having a high hydropathy index and/or at least one amino acid residue having a high hydropathy index is inserted into an REP.

The region locally having a high hydropathy index preferably includes two to four consecutive amino acid residues.

The amino acid residue having a high hydropathy index is more preferably an amino acid residue selected from isoleucine (I), valine (V), leucine (L), phenylalanine (F), cysteine (C), methionine (M), and alanine (A).

The fifth modified fibroin may have the amino acid sequence of the naturally derived spider silk fibroin except that at least one amino acid residue in an REP is substituted by another amino acid residue having a high hydropathy index and/or at least one amino acid residue having a high hydropathy index is inserted into an REP and at least one amino acid residue is substituted, deleted, inserted, and/or added.

The fifth modified spider silk fibroin is obtained by, for example, substituting at least one hydrophilic amino acid residue (for example, an amino acid residue having a negative hydropathy index) in an REP in the cloned gene sequence of the naturally derived spider silk fibroin by a hydrophobic amino acid residue (for example, an amino acid residue having a positive hydropathy index) and/or by inserting at least one hydrophobic amino acid residue into an REP. Alternatively, the fifth modified spider silk fibroin is obtained by, for example, designing an amino acid sequence corresponding to the amino acid sequence of the naturally derived spider silk fibroin except that at least one hydrophilic amino acid residue in an REP is substituted by a hydrophobic amino acid residue and/or at least one hydrophobic amino acid residue is inserted into an REP and by chemically synthesizing a nucleic acid that encodes the designed amino acid sequence. In any case, the fifth modified spider silk fibroin may be modified to have the amino acid sequence of the naturally derived spider silk fibroin except that at least one hydrophilic amino acid residue in an REP is substituted by a hydrophobic amino acid residue and/or at least one hydrophobic amino acid residue is inserted into an REP and at least one amino acid residue is substituted, deleted, inserted, and/or added.

The fifth modified fibroin may have a domain sequence represented by Formula 1: [(A)ₙ motif-REP]ₘ and may have an amino acid sequence in which p/q is 6.2% or more where "p" represents the total number of amino acid residues included in a region where four consecutive amino acid residues have an average hydropathy index of 2.6 or more in all REPs included in the domain sequence excluding a range from the (A)ₙ motif closest to the C-terminus of the domain sequence to the C-terminus, and "q" represents the total number of amino acid residues included in the domain sequence excluding the range from the (A)ₙ motif closest to the C-terminus of the domain sequence to the C-terminus.

A known index (Hydropathy index: Kyte J, & Doolittle R (1982), "A simple method for displaying the hydropathic character of a protein", J. Mol. Biol., 157, pp. 105-132) is used as the hydropathy index of the amino acid residue. Specifically, the hydropathy index (hereinafter also referred to as "HI") of each amino acid is as shown in Table 1.

**[Table 1]**

| Amino Acid | HI | Amino Acid | HI |
|---|---|---|---|
| Isoleucine (Ile) | 4.5 | Tryptophan (Trp) | -0.9 |
| Valine (Val) | 4.2 | Tyrosine (Tyr) | -1.3 |
| Leucine (Leu) | 3.8 | Proline (Pro) | -1.6 |
| Phenylalanine (Phe) | 2.8 | Histidine (His) | -3.2 |
| Cysteine (Cys) | 2.5 | Asparagine (Asn) | -3.5 |
| Methionine (Met) | 1.9 | Aspartic Acid (Asp) | -3.5 |
| Alanine (Ala) | 1.8 | Glutamine (Gln) | -3.5 |
| Glycine (Gly) | -0.4 | Glutamic Acid (Glu) | -3.5 |
| Threonine (Thr) | -0.7 | Lysine (Lys) | -3.9 |
| Serine (Ser) | -0.8 | Arginine (Arg) | -4.5 |

A method for calculating p/q will be described in more detail. The calculation uses the sequence excluding the range from the (A)ₙ motif closest to the C-terminus of the domain sequence to the C-terminus from the domain sequence represented by Formula 1: [(A)ₙ motif-REP]ₘ (hereinafter also referred to as "sequence A"). First, in all REPs included in the sequence A, an average hydropathy index of four consecutive amino acid residues is calculated. An average hydropathy index is determined by dividing a sum of hydropathy indices of amino acid residues in four consecutive amino acid residues by 4 (the number of amino acid residues). An average hydropathy index is determined for all four consecutive amino acid residues (each amino acid residue is used for calculating an average one to four times). Then, a region where four consecutive amino acid residues have an average hydropathy index of 2.6 or more is determined. Even when a certain amino acid residue corresponds to a plurality of "four consecutive amino acid residues having an average hydropathy index of 2.6 or more", the region is regarded as including one amino acid residue. The total number of amino acid residues included in the region is "p". The total number of amino acid residues included in the sequence A is "q".

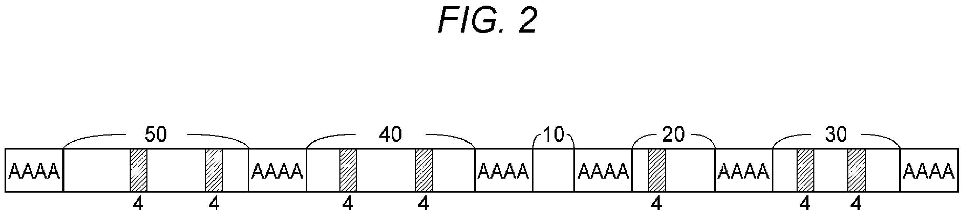

For example, in a case where "four consecutive amino acid residues having an average hydropathy index of 2.6 or more" are extracted from 20 places (no overlap), there are 20 sets of four consecutive amino acid residues (no overlap) in a region where four consecutive amino acid residues have an average hydropathy index of 2.6 or more, and thus, "p" is 20 × 4 = 80. Furthermore, for example, in a case where one amino acid residue overlaps within two sets of "four consecutive amino acid residues having an average hydropathy index of 2.6 or more", a region where four consecutive amino acid residues have an average hydropathy index of 2.6 or more is regarded as including seven amino acid residues (p = 2 × 4 - 1 = 7. The overlapping amino acid residue is deducted, being indicated by "-1"). For example, a domain sequence shown in Fig. 2 includes seven sets of "four consecutive amino acid residues having an average hydropathy index of 2.6 or more" with no overlap, and thus, "p" is 7 × 4 = 28. For example, in the domain sequence shown in Fig. 2, q is 4 + 50 + 4 + 40 + 4 + 10 + 4 + 20 + 4 + 30 = 170 (excluding the (A)ₙ motif at the end of the C-terminus). Next, "p" is divided by "q" to calculate p/q (%). In an example illustrated in Fig. 2, 28/170 = 16.47%.

In the fifth modified spider silk fibroin, p/q is preferably 6.2% or more, more preferably 7% or more, still more preferably 10% or more, still more preferably 20% or more, and still more preferably 30% or more. The upper limit of p/q is not particularly limited but may be, for example, 45% or less.

The fifth modified spider silk fibroin is obtained by modifying the cloned amino acid sequence of the naturally derived spider silk fibroin to have an amino acid sequence including a region locally having a high hydropathy index in such a manner that, for example, at least one hydrophilic amino acid residue (for example, an amino acid residue having a negative hydropathy index) in an REP is substituted by a hydrophobic amino acid residue (for example, an amino acid residue having a positive hydropathy index) and/or at least one hydrophobic amino acid residue is inserted into an REP so as to satisfy the condition of p/q. Alternatively, the fifth modified spider silk fibroin is obtained by, for example, designing an amino acid sequence satisfying the condition of p/q from the amino acid sequence of the naturally derived spider silk fibroin and by chemically synthesizing a nucleic acid that encodes the designed amino acid sequence. In any case, the fifth modified spider silk fibroin may be modified to have the amino acid sequence of the naturally derived spider silk fibroin except that at least one amino acid residue in an REP is substituted by another amino acid residue having a high hydropathy index and/or at least one amino acid residue having a high hydropathy index is inserted into an REP and at least one amino acid residue is substituted, deleted, inserted, and/or added.

The amino acid residue having a high hydropathy index is not particularly limited but is preferably isoleucine (I), valine (V), leucine (L), phenylalanine (F), cysteine (C), methionine (M), and alanine (A). Among these examples, valine (V), leucine (L), and isoleucine (I) are more preferable.

More specific examples of the fifth modified spider silk fibroin include (5-i) a modified spider silk fibroin having the amino acid sequence of SEQ ID NO: 19, SEQ ID NO: 20, or SEQ ID NO: 21 and (5-ii) a modified spider silk fibroin having an amino acid sequence having a sequence identity of 90% or more to the amino acid sequence of SEQ ID NO: 19, SEQ ID NO: 20, or SEQ ID NO: 21.

The modified spider silk fibroin (5-i) will be described. The amino acid sequence of SEQ ID NO: 22 is the same as the amino acid sequence of the naturally derived spider silk fibroin except that consecutive alanine residues in the (A)ₙ motifs are deleted in such a manner that the number of consecutive alanine residues becomes five. The amino acid sequence of SEQ ID NO: 19 is the same as the amino acid sequence of SEQ ID NO: 22 except that two sites of amino acid sequence consisting of three amino acid residues (VLI) are inserted into every other REP and amino acids at the C-terminus are partially deleted so as to be substantially equal to the amino acid sequence of SEQ ID NO: 22 in molecular weight. The amino acid sequence of SEQ ID NO: 23 is the same as the amino acid sequence of SEQ ID NO: 22 except that two alanine residues are inserted into the C-terminus of each (A)ₙ motif and glutamine (Q) residues are partially substituted by serine (S) residues and amino acids at the C-terminus are partially deleted so as to be substantially equal to the amino acid sequence of SEQ ID NO: 22 in molecular weight. The amino acid sequence of SEQ ID NO: 20 is the same as the amino acid sequence of SEQ ID NO: 23 except that one site of amino acid sequence consisting of three amino acid residues (VLI) is inserted into every other REP. The amino acid sequence of SEQ ID NO: 21 is the same as the amino acid sequence of SEQ ID NO: 23 except that two sites of amino acid sequence consisting of three amino acid residues (VLI) are inserted into every other REP.

The modified spider silk fibroin (5-i) may have the amino acid sequence of SEQ ID NO: 19, SEQ ID NO: 20, or SEQ ID NO: 21.

The modified spider silk fibroin (5-ii) has an amino acid sequence having a sequence identity of 90% or more to the amino acid sequence of SEQ ID NO: 19, SEQ ID NO: 20, or SEQ ID NO: 21. The modified spider silk fibroin (5-ii) is also a protein having a domain sequence represented by Formula 1: [(A)ₙ motif-REP]ₘ. The sequence identity is preferably 95% or more.

The modified spider silk fibroin (5-ii) preferably has a sequence identity of 90% or more to the amino acid sequence of SEQ ID NO: 19, SEQ ID NO: 20, or SEQ ID NO: 21 and has an amino acid sequence in which p/q is preferably 6.2% or more where "p" represents the total number of amino acid residues included in a region where four consecutive amino acid residues have an average hydropathy index of 2.6 or more in all REPs included in the domain sequence excluding the range from the (A)ₙ motif closest to the C-terminus of the domain sequence to the C-terminus, and "q" represents the total number of amino acid residues included in the domain sequence excluding the range from the (A)ₙ motif closest to the C-terminus of the domain sequence to the C-terminus.

The fifth modified spider silk fibroin may have a tag sequence at either or both of the N-terminus and the C-terminus.

More specific examples of the fifth modified spider silk fibroin including a tag sequence include (5-iii) a modified spider silk fibroin having the amino acid sequence of SEQ ID NO: 24, SEQ ID NO: 25, or SEQ ID NO: 26 and (5-iv) a modified spider silk fibroin having an amino acid sequence having a sequence identity of 90% or more to the amino acid sequence of SEQ ID NO: 24, SEQ ID NO: 25, or SEQ ID NO: 26.

The amino acid sequences of SEQ ID NO: 24, SEQ ID NO: 25, and SEQ ID NO: 26 are obtained by adding the amino acid sequence of SEQ ID NO: 12 (including a His tag sequence and a hinge sequence) to the N-termini of the amino acid sequences of SEQ ID NO: 19, SEQ ID NO: 20, and SEQ ID NO: 21, respectively.

The modified spider silk fibroin (5-iii) may have the amino acid sequence of SEQ ID NO: 24, SEQ ID NO: 25, or SEQ ID NO: 26.

The modified spider silk fibroin (5-iv) has an amino acid sequence having a sequence identity of 90% or more to the amino acid sequence of SEQ ID NO: 24, SEQ ID NO: 25, or SEQ ID NO: 26. The modified spider silk fibroin (5-iv) is also a protein having a domain sequence represented by Formula 1: [(A)ₙ motif-REP]ₘ. The sequence identity is preferably 95% or more.

The modified spider silk fibroin (5-iv) preferably has a sequence identity of 90% or more to the amino acid sequence of SEQ ID NO: 24, SEQ ID NO: 25, or SEQ ID NO: 26 and has an amino acid sequence in which p/q is 6.2% or more where "p" represents the total number of amino acid residues included in a region where four consecutive amino acid residues have an average hydropathy index of 2.6 or more in all REPs included in the domain sequence excluding the range from the (A)ₙ motif closest to the C-terminus of the domain sequence to the C-terminus, and "q" represents the total number of amino acid residues included in the domain sequence excluding the range from the (A)ₙ motif closest to the C-terminus of the domain sequence to the C-terminus.

The fifth modified spider silk fibroin may include a secretory signal for releasing a protein produced in a recombinant protein production system to the outside of a host. A sequence of the secretory signal is designed appropriately depending on the type of the host.

The domain sequence of the modified spider silk fibroin having a reduced glutamine residue content (sixth modified spider silk fibroin) has the amino acid sequence of the naturally derived spider silk fibroin except that the glutamine residue content is reduced.

In the sixth modified spider silk fibroin, at least one motif selected from GGX motif and GPGXX motif is preferably included in the amino acid sequence of an REP.

In a case where the sixth modified spider silk fibroin includes a GPGXX motif in an REP, the GPGXX motif content is typically 1% or more, may also be 5% or more, and is preferably 10% or more. The upper limit of the GPGXX motif content is not particularly limited and may be 50% or less or 30% or less.

Herein, the "GPGXX motif content" is a value calculated by the following method.

In a spider silk fibroin having a domain sequence represented by Formula 1: [(A)ₙ motif-REP]ₘ or Formula 2: [(A)ₙ motif-REP]ₘ-(A)ₙ motif, the GPGXX motif content is calculated as s/t where "s" represents the number obtained by tripling the total number of GPGXX motifs in all REPs included in the domain sequence excluding a range from the (A)ₙ motif closest to the C-terminus of the domain sequence to the C-terminus (that is, the total number of G and P in the GPGXX motifs), and "t" represents the total number of amino acid residues in all REPs included in the domain sequence excluding the range from the (A)ₙ motif closest to the C-terminus of the domain sequence to the C-terminus and further excluding the (A)ₙ motifs.

In calculating the GPGXX motif content, "the domain sequence excluding the range from the (A)ₙ motif closest to the C-terminus of the domain sequence to the C-terminus" is used to eliminate influence on calculation results of the GPGXX motif content when "m" is small (that is, when the domain sequence is short). This is because "the range from the (A)ₙ motif closest to the C-terminus of the domain sequence to the C-terminus" (sequence corresponding to an REP) may include a sequence that has low correlation with a specific sequence of a spider silk fibroin. In a case where the "GPGXX motif" is located at the C-terminus of an REP, even when "XX" is "AA", for example, it is regarded as the "GPGXX motif".

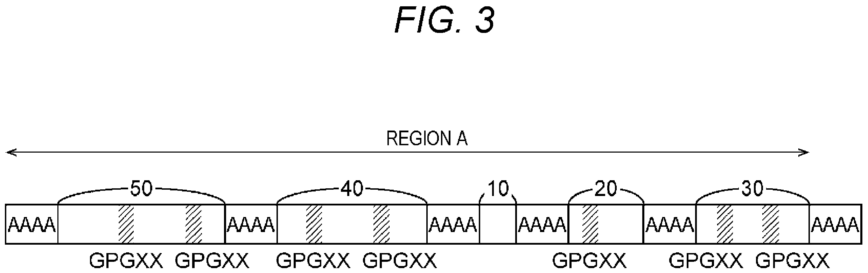

Fig. 3 is a schematic view illustrating a domain sequence of a spider silk fibroin. The calculation of the GPGXX motif content will be specifically described with reference to Fig. 3. First, in the domain sequence of the spider silk fibroin shown in Fig. 3 ("[(A)ₙ motif-REP]ₘ-(A)ₙ motif" type), all REPs are included in "the domain sequence excluding the range from the (A)ₙ motif closest to the C-terminus of the domain sequence to the C-terminus" (shown as "region A" in Fig. 3). The number of GPGXX motifs for calculating "s" is 7, and "s" is 7 × 3 = 21. Similarly, since all REPs are included in "the domain sequence excluding the range from the (A)ₙ motif closest to the C-terminus of the domain sequence to the C-terminus" (shown as "region A" in Fig. 3), the total number "t" of amino acid residues in all REPs after excluding the (A)ₙ motifs from the sequence is 50 + 40 + 10 + 20 + 30 = 150. Next, "s" is divided by "t" to calculate s/t (%). In the fibroin shown in Fig. 3, s/t is 21/150 = 14.0%.

In the sixth modified spider silk fibroin, the glutamine residue content is preferably 9% or less, more preferably 7% or less, still more preferably 4% or less, and particularly preferably 0%.

Herein, "the glutamine residue content" is a value calculated by the following method.

In a spider silk fibroin having a domain sequence represented by Formula 1: [(A)ₙ motif-REP]ₘ or Formula 2: [(A)ₙ motif-REP]ₘ-(A)ₙ motif, the glutamine residue content is calculated as u/t where "u" represents the total number of glutamine residues in all REPs included in the domain sequence excluding the range from the (A)ₙ motif closest to the C-terminus of the domain sequence to the C-terminus (corresponding to "region A" in Fig. 3), and "t" represents the total number of amino acid residues in all REPs included in the domain sequence excluding the range from the (A)ₙ motif closest to the C-terminus of the domain sequence to the C-terminus and further excluding the (A)ₙ motifs. In calculating the glutamine residue content, "the domain sequence excluding the range from the (A)ₙ motif closest to the C-terminus of the domain sequence to the C-terminus" is used for similar reasons to the above reasons.

The domain sequence of the sixth modified spider silk fibroin may have the amino acid sequence of the naturally derived spider silk fibroin except that at least one glutamine residue in an REP is deleted or substituted by another amino acid residue.

The "another amino acid residue" may be an amino acid residue other than the glutamine residue but is preferably an amino acid residue having a higher hydropathy index than that of the glutamine residue. The hydropathy index of each amino acid residue is shown in Table 1.

As shown in Table 1, an example of the amino acid residue having a higher hydropathy index than that of the glutamine residue includes one selected from isoleucine (I), valine (V), leucine (L), phenylalanine (F), cysteine (C), methionine (M), alanine (A), glycine (G), threonine (T), serine (S), tryptophan (W), tyrosine (Y), proline (P), and histidine (H). Among these examples, the amino acid residue is more preferably one selected from isoleucine (I), valine (V), leucine (L), phenylalanine (F), cysteine (C), methionine (M), and alanine (A), and still more preferably one selected from isoleucine (I), valine (V), leucine (L), and phenylalanine (F).

In the sixth modified spider silk fibroin, each REP preferably has a hydropathy index of -0.8 or more, more preferably -0.7 or more, still more preferably 0 or more, still more preferably 0.3 or more, and particularly preferably 0.4 or more. The upper limit of the hydropathy index of each REP is not particularly limited but may be 1.0 or less or 0.7 or less.

Herein, the "hydropathy index of each REP" is a value calculated by the following method.

In a spider silk fibroin having a domain sequence represented by Formula 1: [(A)ₙ motif-REP]ₘ or Formula 2: [(A)ₙ motif-REP]ₘ-(A)ₙ motif, the hydropathy index of each REP is calculated as v/t where "v" represents a sum of hydropathy indices of all amino acid residues in all REPs included in the domain sequence excluding the range from the (A)ₙ motif closest to the C-terminus of the domain sequence to the C-terminus (corresponding to "region A" in Fig. 3), and "t" represents the total number of amino acid residues in all REPs included in the domain sequence excluding the range from the (A)ₙ motif closest to the C-terminus of the domain sequence to the C-terminus and further excluding the (A)ₙ motifs. In calculating the hydropathy index of each REP, "the domain sequence excluding the range from the (A)ₙ motif closest to the C-terminus of the domain sequence to the C-terminus" is used for similar reasons to the above reasons.

The domain sequence of the sixth modified spider silk fibroin may be modified to have the amino acid sequence of the naturally derived spider silk fibroin except that at least one glutamine residue in an REP is deleted and/or at least one glutamine residue in an REP is substituted by another amino acid residue and at least one amino acid residue is substituted, deleted, inserted, and/or added.

The sixth modified spider silk fibroin is obtained by, for example, deleting at least one glutamine residue in an REP from the cloned gene sequence of the naturally derived spider silk fibroin and/or by substituting at least one glutamine residue in an REP by another amino acid residue. Alternatively, the sixth modified spider silk fibroin is obtained by, for example, designing an amino acid sequence corresponding to the amino acid sequence of the naturally derived spider silk fibroin except that at least one glutamine residue in an REP is deleted and/or at least one glutamine residue in an REP is substituted by another amino acid residue and by chemically synthesizing a nucleic acid that encodes the designed amino acid sequence.

More specific examples of the sixth modified spider silk fibroin include (6-i) a modified spider silk fibroin having the amino acid sequence of SEQ ID NO: 27, SEQ ID NO: 28, SEQ ID NO: 29, SEQ ID NO: 30, SEQ ID NO: 31, SEQ ID NO: 32, SEQ ID NO: 33, or SEQ ID NO: 43 and (6-ii) a modified spider silk fibroin having an amino acid sequence having a sequence identity of 90% or more to the amino acid sequence of SEQ ID NO: 27, SEQ ID NO: 28, SEQ ID NO: 29, SEQ ID NO: 30, SEQ ID NO: 31, SEQ ID NO: 32, SEQ ID NO: 33, or SEQ ID NO: 43.

The modified spider silk fibroin (6-i) will be described.

The amino acid sequence of SEQ ID NO: 7 (Met-PRT410) is obtained by modifying an amino acid for improving the productivity based on the nucleotide sequence and amino acid sequence of *Nephila clavipes* (GenBank Accession No.: P46804.1, GI: 1174415) which is a naturally derived fibroin. For example, an amino acid sequence having consecutive alanine residues in a (A)ₙ motif is modified so that the number of the consecutive alanine residues becomes five. On the other hand, a glutamine residue (Q) is not modified in Met-PRT410, whereby Met-PRT410 has the glutamine residue content approximately equal to that of a naturally derived fibroin.

The amino acid sequence of SEQ ID NO: 27 (M_PRT888) is obtained by substituting all QQs in Met-PRT410 (SEQ ID NO: 7) by VL.

The amino acid sequence of SEQ ID NO: 28 (M_PRT965) is obtained by substituting all QQs in Met-PRT410 (SEQ ID NO: 7) by TS and substituting the remaining Q by A.

The amino acid sequence of SEQ ID NO: 29 (M_PRT889) is obtained by substituting all QQs in Met-PRT410 (SEQ ID NO: 7) by VL and substituting the remaining Q by I.

The amino acid sequence of SEQ ID NO: 30 (M_PRT916) is obtained by substituting all QQs in Met-PRT410 (SEQ ID NO: 7) by VI and substituting the remaining Q by L.

The amino acid sequence of SEQ ID NO: 31 (M_PRT918) is obtained by substituting all QQs in Met-PRT410 (SEQ ID NO: 7) by VF and substituting the remaining Q by I.

The amino acid sequence of SEQ ID NO: 34 (M_PRT525) is the same as Met-PRT410 (SEQ ID NO: 7) except that two alanine residues are inserted into a region (A5) having consecutive alanine residues, two domain sequences on the C-terminus are deleted so as to be substantially equal to Met-PRT410 in molecular weight, and glutamine residues (Q) in 13 sites are substituted by a serine residue (S) or a proline residue (P).

The amino acid sequence of SEQ ID NO: 32 (M_PRT699) is obtained by substituting all QQs in M_PRT525 (SEQ ID NO: 34) by VL.

The amino acid sequence of SEQ ID NO: 33 (M_PRT698) is obtained by substituting all QQs in M_PRT525 (SEQ ID NO: 34) by VL and substituting the remaining Q by I.

The amino acid sequence of SEQ ID NO: 43 (Met-PRT966) is obtained by substituting all QQs in the amino acid sequence of SEQ ID NO: 9 (before the amino acid sequence of SEQ ID NO: 42 is added to the C-terminal) by VF and substituting the remaining Q by I.

In all the amino acid sequences of SEQ ID NO: 27, SEQ ID NO: 28, SEQ ID NO: 29, SEQ ID NO: 30, SEQ ID NO: 31, SEQ ID NO: 32, SEQ ID NO: 33, and SEQ ID NO: 43, the glutamine residue content is 9% or less (Table 2).

**[Table 2]**

| Modified Fibroin | | Glutamine Residue Content | GPGXX Motif Content | Hydropathy Index of REP |
|---|---|---|---|---|
| Met-PRT410 (SEQ ID NO: 7) | | 17.7% | 27.9% | -1.52 |
| | M_PRT888 (SEQ ID NO: 27) | 6.3% | 27.9% | -0.07 |
| | M_PRT965 (SEQ ID NO: 28) | 0.0% | 27.9% | -0.65 |
| | M_PRT889 (SEQ ID NO: 29) | 0.0% | 27.9% | 0.35 |
| | M_PRT916 (SEQ ID NO: 30) | 0.0% | 27.9% | 0.47 |
| | M_PRT918 (SEQ ID NO: 31) | 0.0% | 27.9% | 0.45 |
| | M_PRT525 (SEQ ID NO: 34) | 13.7% | 26.4% | -1.24 |
| | M_PRT699 (SEQ ID NO: 32) | 3.6% | 26.4% | -0.78 |
| | M_PRT698 (SEQ ID NO: 33) | 0.0% | 26.4% | -0.03 |
| Met-PRT966 (SEQ ID NO: 43) | | 0.0% | 28.0% | 0.35 |

The modified spider silk fibroin (6-i) may have the amino acid sequence of SEQ ID NO: 27, SEQ ID NO: 28, SEQ ID NO: 29, SEQ ID NO: 30, SEQ ID NO: 31, SEQ ID NO: 32, SEQ ID NO: 33, or SEQ ID NO: 43.

The modified spider silk fibroin (6-ii) has an amino acid sequence having a sequence identity of 90% or more to the amino acid sequence of SEQ ID NO: 27, SEQ ID NO: 28, SEQ ID NO: 29, SEQ ID NO: 30, SEQ ID NO: 31, SEQ ID NO: 32, SEQ ID NO: 33, or SEQ ID NO: 43. The modified spider silk fibroin (6-ii) is also a protein having a domain sequence represented by Formula 1: [(A)ₙ motif-REP]ₘ or Formula 2: [(A)ₙ motif-REP]ₘ-(A)ₙ motif. The sequence identity is preferably 95% or more.

The glutamine residue content of the modified spider silk fibroin (6-ii) is preferably 9% or less. Furthermore, the GPGXX motif content of the modified spider silk fibroin (6-ii) is preferably 10% or more.

The sixth modified spider silk fibroin may have a tag sequence at either or both of the N-terminus and the C-terminus. This enables isolation, immobilization, detection, and visualization of the modified spider silk fibroin.

More specific examples of the sixth modified spider silk fibroin including a tag sequence include (6-iii) a modified fibroin having the amino acid sequence of SEQ ID NO: 35, SEQ ID NO: 36, SEQ ID NO: 37, SEQ ID NO: 38, SEQ ID NO: 39, SEQ ID NO: 40, SEQ ID NO: 41, or SEQ ID NO: 44 and (6-iv) a modified spider silk fibroin having an amino acid sequence having a sequence identity of 90% or more to the amino acid sequence of SEQ ID NO: 35, SEQ ID NO: 36, SEQ ID NO: 37, SEQ ID NO: 38, SEQ ID NO: 39, SEQ ID NO: 40, SEQ ID NO: 41, or SEQ ID NO: 44.

The amino acid sequences of SEQ ID NO: 35, SEQ ID NO: 36, SEQ ID NO: 37, SEQ ID NO: 38, SEQ ID NO: 39, SEQ ID NO: 40, SEQ ID NO: 41, and SEQ ID NO: 44 are obtained by adding the amino acid sequence of SEQ ID NO: 12 (including a His tag sequence and a hinge sequence) to the N-termini of the amino acid sequences of SEQ ID NO: 27, SEQ ID NO: 28, SEQ ID NO: 29, SEQ ID NO: 30, SEQ ID NO: 31, SEQ ID NO: 32, SEQ ID NO: 33, and SEQ ID NO: 43, respectively. Only the tag sequence is added to each N-terminus, the amino acid sequences of SEQ ID NO: 35, SEQ ID NO: 36, SEQ ID NO: 37, SEQ ID NO: 38, SEQ ID NO: 39, SEQ ID NO: 40, SEQ ID NO: 41, and SEQ ID NO: 44 are not changed in glutamine residue content and all have the glutamine residue content of 9% or less (Table 3).

**[Table 3]**

| Modified Fibroin | Glutamine Residue Content | GPGXX Motif Content | Hydropathy Index of REP |
|---|---|---|---|
| PRT888 (SEQ ID NO: 35) | 6.3% | 27.9% | -0.07 |
| PRT965 (SEQ ID NO: 36) | 0.0% | 27.9% | -0.65 |
| PRT889 (SEQ ID NO: 37) | 0.0% | 27.9% | 0.35 |
| PRT916 (SEQ ID NO: 38) | 0.0% | 27.9% | 0.47 |
| PRT918 (SEQ ID NO: 39) | 0.0% | 27.9% | 0.45 |
| PRT699 (SEQ ID NO: 40) | 3.6% | 26.4% | -0.78 |
| PRT698 (SEQ ID NO: 41) | 0.0% | 26.4% | -0.03 |
| PRT966 (SEQ ID NO: 44) | 0.0% | 28.0% | 0.35 |

The modified spider silk fibroin (6-iii) may have the amino acid sequence of SEQ ID NO: 35, SEQ ID NO: 36, SEQ ID NO: 37, SEQ ID NO: 38, SEQ ID NO: 39, SEQ ID NO: 40, SEQ ID NO: 41, or SEQ ID NO: 44.

The modified spider silk fibroin (6-iv) has an amino acid sequence having a sequence identity of 90% or more to the amino acid sequence of SEQ ID NO: 35, SEQ ID NO: 36, SEQ ID NO: 37, SEQ ID NO: 38, SEQ ID NO: 39, SEQ ID NO: 40, SEQ ID NO: 41, or SEQ ID NO: 44. The modified spider silk fibroin (6-iv) is also a protein having a domain sequence represented by Formula 1: [(A)ₙ motif-REP]ₘ or Formula 2: [(A)ₙ motif-REP]ₘ-(A)ₙ motif. The sequence identity is preferably 95% or more.

The glutamine residue content of the modified spider silk fibroin (6-iv) is preferably 9% or less. Furthermore, the GPGXX motif content of the modified spider silk fibroin (6-iv) is preferably 10% or more.

The sixth modified spider silk fibroin may include a secretory signal for releasing a protein produced in a recombinant protein production system to the outside of a host. A sequence of the secretory signal is designed appropriately depending on the type of the host.

Furthermore, the modified spider silk fibroin may be a modified spider silk fibroin having at least two or more properties of the first modified spider silk fibroin, the second modified spider silk fibroin, the third modified spider silk fibroin, the fourth modified spider silk fibroin, the fifth modified spider silk fibroin, and the sixth modified spider silk fibroin.

The modified spider silk fibroin may be a hydrophilic modified spider silk fibroin or a hydrophobic modified spider silk fibroin. The "hydrophobic modified spider silk fibroin" herein has an average hydropathy index (HI) of 0 or less. The average HI is obtained by calculating a sum of hydropathy indices of all amino acid residues included in the modified spider silk fibroin and by dividing the sum by the total number of the amino acid residues. The hydropathy indices are shown in Table 1. Furthermore, the hydrophilic modified spider silk fibroin is a modified spider silk fibroin having an average HI less than 0.

An example of the hydrophobic modified spider silk fibroin includes the sixth modified fibroin. More specific examples of the hydrophobic modified spider silk fibroin include a modified spider silk fibroin having the amino acid sequence of SEQ ID NO: 27, SEQ ID NO: 28, SEQ ID NO: 29, SEQ ID NO: 30, SEQ ID NO: 31, SEQ ID NO: 32, SEQ ID NO: 33, or SEQ ID NO: 43 and a modified spider silk fibroin having the amino acid sequence of SEQ ID NO: 35, SEQ ID NO: 37, SEQ ID NO: 38, SEQ ID NO: 39, SEQ ID NO: 40, SEQ ID NO: 41, or SEQ ID NO: 44.

Examples of the hydrophilic modified spider silk fibroin include the first modified fibroin, second modified fibroin, third modified fibroin, fourth modified fibroin, and fifth modified fibroin. More specific examples of the hydrophilic spider silk protein include a modified spider silk fibroin including the amino acid sequence of SEQ ID NO: 4, a modified spider silk fibroin including the amino acid sequence of SEQ ID NO: 6, SEQ ID NO: 7, SEQ ID NO: 8, or SEQ ID NO: 9, a modified spider silk fibroin including the amino acid sequence of SEQ ID NO: 13, SEQ ID NO: 11, SEQ ID NO: 14, or SEQ ID NO: 15, a modified spider silk fibroin including the amino acid sequence of SEQ ID NO: 18, SEQ ID NO: 7, SEQ ID NO: 8, or SEQ ID NO: 9, a modified spider silk fibroin including the amino acid sequence of SEQ ID NO: 17, SEQ ID NO: 11, SEQ ID NO: 14, or SEQ ID NO: 15, and a modified spider silk fibroin including the amino acid sequence of SEQ ID NO: 19, SEQ ID NO: 20, or SEQ ID NO: 21.

One type of these modified spider silk fibroins is used independently, or two or more types thereof are used in combination.

A modified spider silk fibroin is produced by, for example, expressing a nucleic acid that encodes the modified spider silk fibroin, using a host transformed with an expression vector having a sequence of the nucleic acid and at least one regulatory sequence operably linked to the sequence of the nucleic acid.

A method for producing the nucleic acid that encodes the modified spider silk fibroin is not particularly limited. The nucleic acid is produced by, for example, amplification and cloning by a polymerase chain reaction (PCR) using a gene that encodes the modified spider silk fibroin or by chemical synthesis of the nucleic acid. A method for chemically synthesizing the nucleic acid is not particularly limited. For example, based on amino acid sequence information of a spider silk protein obtained from the NCBI web data base, the gene is chemically synthesized by PCR to link oligonucleotides automatically synthesized by AKTA oligopilot plus 10/100 (GE Healthcare, Japan). In this case, in order to facilitate purification and/or determination of the modified spider silk fibroin, the synthesis may be performed on the nucleic acid that encodes the modified spider silk fibroin including an amino acid sequence obtained by adding an amino acid sequence consisting of a start codon and a His10 tag to the N-terminus.

The regulatory sequence controls the expression of a recombinant protein in a host (for example, a promoter, an enhancer, a ribosome binding sequence, and a transcription termination sequence) and is appropriately selected depending on the type of the host. The promoter may be an inducible promoter that functions in a host cell and can induce the expression of a modified spider silk fibroin of interest. The inducible promoter controls transcription due to the presence of an inducer (expression inducer), the absence of a repressor molecule, and physical factors such as an increase or a decrease in temperature, osmotic pressure, or pH value.

The type of the expression vector is appropriately selected depending on the type of the host. Examples of the expression vector include plasmid vector, viral vector, cosmid vector, fosmid vector, and artificial chromosome vector. A preferable example of the expression vector includes one that enables autonomous replication in a host cell or enables incorporation into a chromosome of a host and contains a promoter at a position where the nucleic acid that encodes the modified spider silk fibroin is transcribed.

Both prokaryotes and eukaryotes such as yeasts, filamentous fungi, insect cells, animal cells, and plant cells are suitably used as a host.

In a case where a prokaryote such as a bacteria is a host, a preferable expression vector is one that can autonomously replicate in the prokaryote and contains a promoter, a ribosome binding sequence, the nucleic acid that encodes the modified spider silk fibroin, and a transcription termination sequence. The expression vector may contain a gene that controls the promoter.

Examples of the prokaryote include microorganisms belonging to the genera *Escherichia, Brevibacillus, Serratia, Bacillus, Microbacterium, Brevibacterium, Corynebacterium,* and *Pseudomonas.* An example of the microorganism belonging to the genus *Escherichia* includes *Echerichia coli.* An example of the microorganism belonging to the genus *Brevibacillus* includes *Brevibacillus agri.* An example of the microorganism belonging to the genus *Serratia* includes *Serratia liquefaciens.* An example of the microorganism belonging to the genus *Bacillus* includes *Bacillus subtilis.* An example of the microorganism belonging to the genus *Microbacterium* includes *Microbacterium ammoniaphilum.* An example of the microorganism belonging to the genus *Brevibacterium* includes *Brevibacterium divaricatum.* An example of the microorganism belonging to the genus *Corynebacterium* includes *Corynebacterium ammoniagenes.* An example of the microorganism belonging to the genus *Pseudomonas* includes *Pseudomonas putida.*

In a case where a prokaryote is a host, examples of a vector that introduces the nucleic acid that encodes the modified spider silk fibroin include pBTrp2 (available from Boehringer Mannheim GmbH), pGEX (available from Pharmacia Corporation), and pUC18, pBluescriptII, pSupex, pET22b, pCold, pUB110, and pNCO2 (JP 2002-238569 A).

Examples of the eukaryotic host include yeasts and filamentous fungi (such as molds). Examples of the yeasts include those belonging to the genera *Saccharomyces, Pichia,* and *Schizosaccharomyces.* Examples of the filamentous fungi include those belonging to the genera *Aspergillus, Penicillium,* and *Trichoderma.*

In a case where a eukaryote is a host, YEp13 (ATCC37115) or YEp24 (ATCC37051) is used as a vector that introduces the nucleic acid that encodes the modified spider silk fibroin. A method for introducing an expression vector into the host cell may employ any method as long as the method introduces DNA into the host cell. Examples of the method include a method using calcium ions [Proc. Natl. Acad. Sci. USA, 69, 2110 (1972)], electroporation method, spheroplast method, protoplast method, lithium acetate method, and competent method.

Examples of a method for expressing the nucleic acid in a host transformed with an expression vector include not only direct expression but also secretory production and fusion protein expression according to the method described in Molecular Cloning, 2nd edition.

The modified spider silk fibroin is produced by, for example, culturing a transformed host in a culture medium, producing and accumulating the modified spider silk fibroin in the culture medium, and collecting the modified spider silk fibroin from the culture medium. The transformed host is cultured in the culture medium according to a method commonly used for culturing a host.

In a case where the host is a prokaryote such as *Escherichia coli* or a eukaryote such as yeast, the culture medium may be either a natural medium or a synthetic medium as long as the medium contains a carbon source, a nitrogen source, an inorganic salt, or the like which can be assimilated by the host and facilitates efficient culturing of the host.

Any carbon source may be employed as long as the host assimilates the carbon source. Examples of the carbon source include carbohydrates such as glucose, fructose, sucrose, molasses containing these examples, starch, and starch hydrolysate; organic acids such as acetic acid and propionic acid; and alcohols such as ethanol and propanol.

Examples of the nitrogen source include ammonium salts of inorganic or organic acids such as ammonia, ammonium chloride, ammonium sulfate, ammonium acetate, and ammonium phosphate and also include other nitrogen-containing compounds, peptone, meat extract, yeast extract, corn steep liquor, casein hydrolysate, soybean cake, soybean cake hydrolysate, various fermenters, and digests thereof.

Examples of the inorganic salt include monopotassium phosphate, dipotassium phosphate, magnesium phosphate, magnesium sulfate, sodium chloride, ferrous sulfate, manganese sulfate, copper sulfate, and calcium carbonate.

Prokaryotes such as *Escherichia coli* or eukaryotes such as yeast are cultured under aerobic conditions by, for example, shaking or aeration and agitation in submerged culture. The culture temperature is, for example, from 15 to 40°C. The culture time is typically 16 hours to 7 days. The pH of the culture medium during the culturing is preferably maintained at 3.0 to 9.0. The pH of the culture medium is adjusted using, for example, an inorganic acid, an organic acid, an alkali solution, urea, calcium carbonate, and ammonia.

In addition, antibiotics such as ampicillin and tetracycline may be optionally added to the culture medium during the culturing. In culturing of a microorganism transformed with an expression vector using an inducible promoter as a promoter, an inducer may be optionally added to the medium. For example, in culturing of a microorganism transformed with an expression vector using a lac promoter, isopropyl-β-D-thiogalactopyranoside may be added to the medium, and in culturing of a microorganism transformed with an expression vector using a trp promoter, indole acrylic acid may be added to the medium.

The modified spider silk fibroin produced by the transformed host is isolated and purified by a method commonly used for protein isolation and purification. For example, in a case where the modified spider silk fibroin is expressed being dissolved in a cell, after completion of the culturing, the host cell is collected by centrifugation and suspended in an aqueous buffer, and then, disrupted with an ultrasonicator, a French press, a Manton-Gaulin homogenizer, a Dyno-Mill, or the like, thereby obtaining a cell-free extract. A purified preparation is obtained from supernatant obtained by centrifuging the cell-free extract according to one of the following methods commonly used for protein isolation and purification or according to a combination of the following methods, that is, solvent extraction, salting-out using ammonium sulfate or the like, desalting, precipitation using an organic solvent, anion exchange chromatography using a resin such as diethylaminoethyl (DEAE)-sepharose or DIAION HPA-75 (available from Mitsubishi Kasei Kogyo Kabushiki Kaisha), cation exchange chromatography using a resin such as S-sepharose FF (available from Pharmacia Corporation), hydrophobic chromatography using a resin such as butyl sepharose and phenyl sepharose, gel filtration using a molecular sieve, affinity chromatography, chromatofocusing, and electrophoresis such as isoelectric focusing phoresis.

The aforementioned chromatographies may preferably employ phenyl-TOYOPEARL (Tosoh Corporation), DEAE-TOYOPEARL (Tosoh Corporation), and Sephadex G-150 (Pharmacia Biotech Inc.).

In a case where the modified spider silk fibroin is expressed, forming an insoluble matter in the cell, the insoluble matter is collected in a similar manner. That is, the host cell is collected, disrupted, and centrifuged so as to collect the insoluble matter of the modified spider silk fibroin as a precipitated fraction. The collected insoluble matter of the modified spider silk fibroin is solubilized with a protein denaturing agent. After this manipulation, a purified preparation of the modified spider silk fibroin is yielded by isolation and purification in a similar manner.

In a case where the modified spider silk fibroin is secreted extracellularly, the modified spider silk fibroin is collected from culture supernatant. In other words, culture supernatant is obtained by centrifugation of a culture or by treating the culture with other techniques, and from the culture supernatant, a purified preparation is yielded by isolation and purification in a similar manner.

### [Muscle Tissue-regenerating Agent]

A muscle tissue-regenerating agent according to this embodiment contains a modified fibroin protein.

The muscle tissue-regenerating agent according to this embodiment is not particularly limited in form and may be formed into, for example, any one of a film, sheet, fiber, resin body, powder, and gel or formed by any combination of these forms. Alternatively, the muscle tissue-regenerating agent according to this embodiment may be formed into a film or a sheet or formed by a combination of these forms. A form including a film may be in the category of "film". The muscle tissue-regenerating agent according to this embodiment may contain the modified fibroin protein and a solvent such as an alcohol or dimethyl sulfoxide to the extent that the solvent does not harm a living organism. The solvent may be contained inside the muscle tissue-regenerating agent. The solvent may be dispersed and contained inside the muscle tissue-regenerating agent. The solvent may be uniformly dispersed inside the muscle tissue-regenerating agent.

Containing the modified fibroin protein and the solvent, the muscle tissue-regenerating agent according to the first embodiment significantly improves in elongation. The muscle tissue-regenerating agent according to the first embodiment improves in elongation while maintaining the stress, whereby the muscle tissue-regenerating agent also improves in toughness.

The muscle tissue-regenerating agent according to this embodiment is excellent in strength, elongation, and toughness and is preferably used for medical purposes. The muscle tissue-regenerating agent significantly improves in handleability during surgery or the like. In addition, the muscle tissue-regenerating agent exhibits resistance to repetitive expansion and contraction when being attached to an organ or the like.

The muscle tissue-regenerating agent according to this embodiment preferably has fracture-point displacement of 10% or more, 20% or more, 30% or more, 40% or more, 50% or more, 100% or more, or 200% or more. The larger the fracture-point displacement, the better the elongation (the higher the elongation).

The protein film according to this embodiment preferably has strain energy of 5 mJ or more, 10 mJ or more, 20 mJ or more, or 30 mJ or more. The higher the strain energy, the better the toughness. The muscle tissue-regenerating agent according to this embodiment may have ultimate stress of, for example, 5 MPa or more or 10 MPa or more. The higher the ultimate stress (MPa), the better the stress (the higher the stress).

The protein film preferably has toughness of 10 MJ/m3 or more, 20 MJ/m3 or more, 30 MJ/m3 or more, or 40 MJ/m3 or more. The protein film that is an unstretched film may have a toughness within the above range.

Fracture-point displacement, ultimate stress, strain energy, and toughness of the muscle tissue-regenerating agent are determined by tensile testing. Specifically, first, the muscle tissue-regenerating agent is cut into a dumbbell shape as a test piece, and a tensile test is performed at a temperature of 20°C and a relative humidity of 65% to measure fracture-point displacement. The tensile test is performed setting a gauge length (between shoulders) to 8 mm, a gauge width to 2 mm, lengths of both grip sections to 7 mm, and widths of both grip sections to 5 mm. The thickness of the 8-mm portion is measured at N = 1. The tensile test is performed with a tensile tester Instron 3345 and a load cell at 10 N and at a tension rate set to 10 mm/min. The measurement is performed at least three times for each standard, and an average of the measurements is used.

The thickness of the muscle tissue-regenerating agent may be appropriately set according to the use of the muscle tissue-regenerating agent. For example, from a perspective of easily suppressing variations in elongation, the muscle tissue-regenerating agent may have a thickness of 1 to 1000 µm, 10 to 300 µm, 10 to 100 µm, 30 to 100 µm, or 50 to 200 µm and preferably has a thickness of 30 to 70 µm. The thickness of the muscle tissue-regenerating agent is measured by a method to be described in the following Examples. The thickness of the muscle tissue-regenerating agent is adjusted, for example, by selecting a solvent used for a dope solution. For example, using water as the dope solvent tends to yield a muscle tissue-regenerating agent having a thickness of 30 to 100 µm, and using an organic solvent as the dope solvent tends to yield a protein film having a thickness of 50 to 200 µm.

The muscle tissue-regenerating agent may be subjected to methanol treatment or is not necessarily subjected to the methanol treatment. In elongation and toughness perspectives, it is preferable not to perform the methanol treatment. From a stress perspective, the methanol treatment may be performed. The methanol treatment is performed by bringing the protein film into contact with methanol. The methanol treatment may involve drying methanol after contact with methanol. For example, in the methanol treatment, the muscle tissue-regenerating agent is immersed in methanol at room temperature (from 20 to 30°C), and then, washed with ion-exchanged water. The washing time may be, for example, 1 to 10 minutes. Then, the drying may be performed at room temperature (from 20 to 30°C). The time for bringing the muscle tissue-regenerating agent into contact with methanol may be, for example, 1 to 10 minutes. An example of methanol includes a guaranteed reagent 99.5% methanol available from FUJIFILM Wako Pure Chemical Corporation.

The muscle tissue-regenerating agent according to this embodiment also has biodegradability because the agent contains the modified fibroin protein as a raw material. The muscle tissue-regenerating agent according to this embodiment may be, for example, biodegraded within 120 days or less, 100 days or less, 80 days or less, 70 days or less, 60 days or less, or 50 days or less in a mammalian living organism. From a perspective of suppressing the risk of unwanted foreign-body responses (such as thrombus, infection, thickening, and encapsulation) that may be caused by the muscle tissue-regenerating agent remaining in the body for a long time, and promoting an effective recovery, the muscle tissue-regenerating agent is preferably biodegraded within 80 days and more preferably biodegraded within 70 days, 60 days, or 50 days. The muscle tissue-regenerating agent for biodegradation within the above days may consist of the modified fibroin protein independently or may also contain components other than the modified fibroin protein. Examples of the components other than the modified fibroin protein include alcohols (as already described), water, organic solvents, and polymer plasticizers. However, from a perspective of maintaining the biodegradability of the modified fibroin protein, it is preferable to employ a biodegradable component that does not inhibit the biodegradation of the modified fibroin protein. Examples of such a component include an alcohol, water, and dimethyl sulfoxide. From a similar perspective, it is preferable that the muscle tissue-regenerating agent contains small amount of components other than the modified fibroin protein such as 50 mass% or less, 40 mass% or less, or 30 mass% or less with respect to the total mass% of the muscle tissue-regenerating agent.

Fibroin proteins including products degraded in vivo are biodegradable, non-toxic, and safe.

Due to the following properties, an artificially produced modified fibroin protein is superior to a fibroin protein obtained from a silkworm.

For example, the use of naturally derived fibroin proteins for medical purposes may cause allergies due to metals or the like contained in mulberries eaten by reared silkworms. Naturally derived fibroin proteins may change in higher-order structure, solubility in water, and resistance depending on the conditions of rearing and preserving silkworms, which leaves room for improvement in ensuring product stability. Furthermore, rearing animals before production of a silk film requires time and costs. On the other hand, industrially producing the modified fibroin protein enables quality control and ensures mass production in a short time. Accordingly, the industrially produced modified fibroin protein is particularly preferable as an embodiment.

Due to relatively low biodegradation rates, silk materials are not determined as biodegradable materials by U.S. Pharmacopeia and may require removal from the body. On the other hand, the modified fibroin protein has a high biodegradation rate and has no need for removal from the body. Accordingly, the modified fibroin protein shows promise for significant reduction of the risk of thrombus, infection, thickening, and encapsulation or the like after treatment.

In particular, a fibroin having an average hydropathy index (average HI) of 0 or less has high affinity for water. Therefore, a pharmaceutical agent produced using this fibroin as a component is expected to have further excellent biodegradability. For this reason, the modified fibroin protein preferably has an average HI of 0 or less, more preferably -0.2 or less, and still more preferably -0.5 or less.

An effective treatment is anticipated by, for example, bringing the muscle tissue-regenerating agent into contact with a site having a deficient myocyte in a human or a non-human animal (preferably a mammal).

Therefore, the muscle tissue-regenerating agent of this embodiment is used for a treatment of a condition in need of muscle tissue regeneration. The treatment of a condition in need of muscle tissue regeneration may involve bringing a muscle tissue-regenerating agent into contact with a site having a deficient myocyte in a human or a non-human animal. In addition, the muscle tissue-regenerating agent may be a therapeutic agent for a condition in need of muscle tissue regeneration. Examples of the condition in need of muscle tissue regeneration include a condition including a muscle tissue defect, specifically, hernia (including abdominal incisional hernia, diaphragmatic hernia, and inguinal hernia), site of amputation stump plasty, decubitus, and wound. The condition in need of muscle tissue regeneration may be one selected from the group consisting of abdominal incisional hernia, diaphragmatic hernia, inguinal hernia, site of amputation stump plasty, decubitus, and wound or may be a combination of these conditions.

A muscle tissue includes multinucleate cells of muscle fibers, and mononuclear satellite cells are present between the cells of the muscle fibers. In regard to features of muscle fibers, they do not undergo cell division but regenerate by the function of satellite cells or stem cells. Therefore, requirements are different between regeneration of muscle tissues and regeneration of skin tissues that undergo cell migration and cell division.

Regeneration of a skeletal muscle involves the following steps. 1) Satellite cells are typically in a stationary state and do not proliferate. Stimulation such as damage to a muscle tissue changes the satellite cells into myoblasts. 2) The myoblasts are differentiated into myocytes by cell division. A plurality of myocytes is fused to form multinucleate myotubes. 3) The myotubes form muscle fibers and fuse with the original muscle fibers to regenerate a skeletal muscle. Each step is unlikely to occur spontaneously. Regeneration of a muscle tissue is achieved by executing all of these steps.

An aspect of the present invention is to promote and achieve muscle tissue regeneration involving the above steps. The muscle tissue-regenerating agent according to this embodiment may be a prosthetic and regenerating agent for a muscle tissue defect. In addition, the muscle tissue-regenerating agent according to this embodiment may be an activator for a satellite cell in a muscle tissue. The muscle tissue-regenerating agent according to this embodiment may be an agent for differentiating a myoblast into a myocyte. In addition, the muscle tissue-regenerating agent according to this embodiment may be a myoblast adhesive. The muscle tissue-regenerating agent according to this embodiment may be a therapeutic agent for a condition in need of muscle tissue regeneration. For example, the condition is one selected from abdominal incisional hernia, diaphragmatic hernia, inguinal hernia, site of amputation stump plasty, decubitus, and wound.

The muscle tissue-regenerating agent preferably contains 30 mass% or more to 100 mass% or less of the modified fibroin protein with respect to the total mass of the muscle tissue-regenerating agent. The modified fibroin protein content may be 5 mass% or more and 100 mass% or less, 10 mass% or more and 100 mass% or less, 15 mass% or more and 100 mass% or less, or 30 mass% or more and 100 mass% or less with respect to the total mass of the muscle tissue-regenerating agent. The modified fibroin protein content in the muscle tissue-regenerating agent is calculated based on, for example, data obtained by amino acid composition analysis using an amino acid automatic analyzer after adding water with an acid to the regenerating agent.

The muscle tissue-regenerating agent according to this embodiment, for example, may be composed of a modified fibroin protein or may contain a modified fibroin protein and at least one component selected from an alcohol, water, an organic solvent, and a polymer plasticizer.

The alcohol is not limited in type, but preferable examples of the alcohol include those having 1 to 20 carbon atoms. Specific examples of the alcohol include methanol, ethanol, and propanol. A polyhydric alcohol is more preferable in that the alcohol has a high melting point and is hardly lost from components contained in the muscle tissue-regenerating agent at normal temperature and normal pressure.

The polyhydric alcohol represents an alcohol having two or more hydroxy groups in a molecule. The polyhydric alcohol may contain, for example, 2 to 10, 2 to 8, 2 to 6, 3 to 5, or 3 hydroxy groups.

The number of carbon atoms in the alcohol may be, for example, 20 or less, 15 or less, 10 or less, or 6 or less and may be 2 or more, or 3 or more.

Examples of the polyhydric alcohol include a lower alcohol, a saccharide, a sugar alcohol, and polyethylene glycol.

The lower alcohol represents a linear or branched alcohol having 2 to 5 carbon atoms. Examples of the lower alcohol include glycerin, propylene glycol, diethylene glycol, and ethylene glycol. The lower alcohol is preferably glycerin from a perspective of further improving the elongation of the muscle tissue-regenerating agent.

Examples of the saccharide include monosaccharide and disaccharide. An example of the monosaccharide includes glucose. Examples of the disaccharide include sucrose, lactose, trehalose, and maltose.

Examples of the sugar alcohol include sorbitol and mannitol.

The alcohol content may be 0.01 to 50 mass%, 0.1 to 40 mass%, 1 to 30 mass%, 0.01 to 1 mass%, or 0.01 to 5 mass% with respect to the total mass of the muscle tissue-regenerating agent. The alcohol content is measured by gel permeation chromatography (with, for example, Prominence 504H available from Shimadzu Corporation).

In a case where the muscle tissue-regenerating agent contains water, the moisture content of the muscle tissue-regenerating agent may be, for example, 1 to 70 mass% or 5 to 60 mass% and is preferably 5 to 40% so that the muscle tissue-regenerating agent further improves in elongation. The moisture content of the muscle tissue-regenerating agent is the water content with respect to the total mass of the muscle tissue-regenerating agent. The muscle tissue-regenerating agent may hold water inside. The moisture content of the muscle tissue-regenerating agent is measured by, for example, Karl Fischer moisture titrator MKH-700 (Kyoto Electronics Manufacturing Co., Ltd.) and heat drying moisture analyzer MX-50 (A & D Company, Limited).

Examples of the organic solvent include acetic acid, formic acid, HFIP, and aprotic polar solvents such as dimethyl sulfoxide (DMSO), N, N-dimethylformamide (DMF), and N, N-dimethylacetamide (DMAc). The organic solvent is desirably a volatile solvent having no toxicity to animals such as dimethyl sulfoxide. Dimethyl sulfoxide is more effective as the material in that DMSO has a high melting point and is hardly lost from components contained in the muscle tissue-regenerating agent at normal temperature and normal pressure. What is more, DMSO promotes dissolution of other useful biological substances. In a case where the muscle tissue-regenerating agent contains an organic solvent, the organic solvent content in the protein film may be 0.01 to 50 mass%, 0.1 to 40 mass%, 1 to 30 mass%, 0.01 to 1 mass%, or 0.01 to 5 mass%. The organic solvent content is measured by, for example, gel permeation chromatography (with, for example, Prominence 504H available from Shimadzu Corporation).

Assuming that the muscle tissue-regenerating agent is used for medical purposes, in consideration of influences on the patient's body, it is preferable to employ an alcohol, water, or dimethyl sulfoxide as a plasticizing liquid contained in the muscle tissue-regenerating agent. In addition, a fibroin protein composition containing an alcohol or dimethyl sulfoxide has a possibility of assisting the migration of phagocytes (such as neutrophils, monocytes, and macrophages) around necrosis of damaged myocytes and shows promise for activating behaviors of fibroblasts that produce extracellular matrix (ECM) components included in an intramuscular connective tissue, for activating satellite cells, and for proliferating these cells.

The liquid content of the muscle tissue-regenerating agent is not particularly limited, but the total amount of the liquid is preferably 0.01 to 50 mass%, more preferably 0.1 to 40 mass%, and still more preferably 1 to 30 mass% with respect to the total mass of the muscle tissue-regenerating agent so that the muscle tissue-regenerating agent maintains in strength and effectively improves in elongation.

The muscle tissue-regenerating agent according to this embodiment may contain unavoidable components such as impurities contained in components other than a protein. For example, in a case where the muscle tissue-regenerating agent contains a synthetic fibroin protein, the impurities are unreacted components. For example, in a case where the muscle tissue-regenerating agent contains a recombinant fibroin protein, the impurities are a trace amount of host cell-derived phospholipids or other substances in a fibroin protein composition produced using the host cell. Such host-derived substances (impurities) show promise in promoting inflammation of cells and activating behaviors of fibroblasts that produce extracellular matrix (ECM) components included in an intramuscular connective tissue. For this reason, a recombinant fibroin protein composition further containing a substance derived from a host used in producing the recombinant fibroin protein is useful as the muscle tissue-regenerating agent.

### <Protein Film>

The muscle tissue-regenerating agent according to this embodiment may be in the form of a film or may include a component in the form of a film. A film containing a modified fibroin protein (hereinafter also referred to as "protein film") may contain the modified fibroin protein and a solvent such as an alcohol and dimethyl sulfoxide to the extent that the solvent does not harm a living organism. The solvent may be contained inside the protein film. The solvent may be dispersedly contained inside the protein film. The solvent may be uniformly dispersed inside the protein film.

The protein film according to the first embodiment contains the modified fibroin protein and the solvent, which enhances the elongation significantly. The protein film according to the first embodiment improves in elongation while maintaining the stress, whereby the protein film also improves in toughness.

The protein film according to this embodiment is excellent in strength, elongation, and toughness and is preferably used for medical purposes. The muscle tissue-regenerating agent significantly improves in handleability during surgery or the like. In addition, the muscle tissue-regenerating agent exhibits resistance to repetitive expansion and contraction when being attached to an organ or the like.

The protein film according to this embodiment preferably has fracture-point displacement of 10% or more, 20% or more, 30% or more, 40% or more, 50% or more, 100% or more, or 200% or more. The larger the fracture-point displacement, the better the elongation (the higher the elongation).

The protein film according to this embodiment preferably has strain energy of 5 mJ or more, 10 mJ or more, 20 mJ or more, or 30 mJ or more. The higher the strain energy, the better the toughness. The protein film according to this embodiment may have ultimate stress of, for example, MPa or more or 10 MPa or more. The higher the ultimate stress (MPa), the better the stress (the higher the stress).

The protein film preferably has toughness of 10 MJ/m3 or more, 20 MJ/m3 or more, 30 MJ/m3 or more, or 40 MJ/m3 or more. The protein film that is an unstretched film may have toughness within the above range.

Fracture-point displacement, ultimate stress, strain energy, and toughness of the protein film are determined by tensile testing. Specifically, first, the protein film is cut into a dumbbell shape as a test piece, and a tensile test is performed at a temperature of 20°C and a relative humidity of 65% to measure fracture-point displacement. The tensile test is performed setting a gauge length (between shoulders) to 8 mm, a gauge width to 2 mm, lengths of both grip sections to 7 mm, and widths of both grip sections to 5 mm. The thickness of the 8-mm portion is measured at N = 1. The tensile test is performed with a tensile tester Instron 3345 and a load cell at 10 N and at a tension rate set to 10 mm/min. The measurement is performed at least three times for each standard, and an average of the measurements is used.

The thickness of the protein film may be appropriately set according to the use of the protein film. For example, from a perspective of easily suppressing variations in elongation, the protein film may have a thickness of 1 to 1000 µm, 10 to 300 µm, 10 to 100 µm, 30 to 100 µm, or 50 to 200 µm and preferably has a thickness of 30 to 70 µm. The thickness of the protein film is measured by a method to be described in the following Examples. The thickness of the protein film is adjusted, for example, by selecting a solvent used for a dope solution. For example, using water as the dope solvent tends to yield a protein film having a thickness of 30 to 100 µm, and using an organic solvent as the dope solvent tends to yield a protein film having a thickness of 50 to 200 µm.

The protein film may be an unstretched film or a stretched film. The stretched film may be a uniaxially or biaxially stretched film.

The protein film may be subjected to methanol treatment or is not necessarily subjected to the methanol treatment. In elongation and toughness perspectives, it is preferable not to perform the methanol treatment. From a stress perspective, the methanol treatment may be performed. The methanol treatment is performed by bringing the protein film into contact with methanol. The methanol treatment may involve drying methanol after contact with methanol. For example, in the methanol treatment, the protein film is immersed in methanol at room temperature (from 20 to 30°C), and then, washed with ion-exchanged water. The washing time may be, for example, 0.1 to 10 minutes. Then, the drying may be performed at room temperature (from 20 to 30°C). The time for bringing the protein film into contact with methanol may be, for example, 1 to 10 minutes. An example of methanol includes a guaranteed reagent 99.5% methanol available from FUJIFILM Wako Pure Chemical Corporation.

The modified fibroin protein content may be 50 to 90 mass%, 60 to 85 mass%, or 65 to 80 mass% with respect to the total mass of the protein film. The modified fibroin protein content of the protein film is calculated, for example, by measuring the water content and the alcohol content and by determining a difference between the total mass and the mass of water and the alcohol.

### <The polyhydric alcohol>

The polyhydric alcohol represents an alcohol having two or more hydroxy groups in a molecule. The polyhydric alcohol may contain, for example, 2 to 10, 2 to 8, 2 to 6, 3 to 5, or 3 hydroxy groups.

The number of carbon atoms in the polyhydric alcohol may be, for example, 12 or less, 10 or less, 8 or less, or 6 or less and may be 2 or more, or 3 or more.

Examples of the polyhydric alcohol include a lower alcohol, a saccharide, a sugar alcohol, and polyethylene glycol.

The lower alcohol represents a linear or branched alcohol having 2 to 5 carbon atoms. Examples of the lower alcohol include glycerin, propylene glycol, diethylene glycol, and ethylene glycol. The lower alcohol is preferably glycerin from a perspective of further improving the elongation of the protein film.

Examples of the saccharide include monosaccharide and disaccharide. An example of the monosaccharide includes glucose. Examples of the disaccharide include sucrose, lactose, trehalose, and maltose.

Examples of the sugar alcohol include sorbitol and mannitol.

The alcohol content may be 3 to 40 mass%, 5 to 35 mass%, 5 to 50 mass%, or 10 to 30 mass% with respect to the total mass of the protein film. The alcohol content is measured by gel permeation chromatography (with, for example, Prominence 504H available from Shimadzu Corporation).

### <Other Components>

The protein film may consist of a spider silk fibroin and glycerin or may also contain other components. Examples of other components include water, an organic solvent, and a polymer plasticizer.

In a case where the protein film contains water, the moisture content of the protein film may be, for example, 1 to 70 mass% or 5 to 60 mass% and is preferably 5 to 40% so that the protein film further improves in elongation. The moisture content of the protein film is the water content with respect to the total mass of the protein film. The protein film may hold water inside. The moisture content of the protein film is measured by, for example, Karl Fischer moisture titrator MKH-700 (Kyoto Electronics Manufacturing Co., Ltd.) and heat drying moisture analyzer MX-50 (A & D Company, Limited).

Examples of the organic solvent include acetic acid, formic acid, HFIP, and aprotic polar solvents such as dimethyl sulfoxide (DMSO), N, N-dimethylformamide (DMF), and N, N-dimethylacetamide (DMAc). The organic solvent is desirably a volatile solvent having no toxicity to animals such as dimethyl sulfoxide. In a case where the protein film contains an organic solvent, the organic solvent content of the protein film may be 2 to 50 mass% or 2 to 30 mass%. The organic solvent content is measured by, for example, gel permeation chromatography (with, for example, Prominence 504H available from Shimadzu Corporation).

The protein film according to this embodiment may contain unavoidable components such as impurities contained in a protein.

### <Production of Protein Film>

When the muscle tissue-regenerating agent according to this embodiment is or includes a protein film, the protein film is produced by casting a dope solution containing a spider silk fibroin and a solvent on a substrate surface, followed by drying and/or desolvating the dope solution. An alcohol may be added to the dope solution in advance. Even in a case where the dope solution contains an alcohol, the protein film of interest is obtained in a similar manner. With the dope solution containing an alcohol, film formation according to the aforementioned method makes it possible to obtain a protein film containing the alcohol dispersed therein. In a case where the dope solution does not contain an alcohol, a protein film formed according to the aforementioned method is immersed in the alcohol, thereby obtaining the protein film containing the alcohol dispersed therein.

The protein film according to an embodiment includes forming of a protein film using a dope solution containing a spider silk fibroin, an alcohol, and a solvent.

The dope solution contains the spider silk fibroin, the alcohol, and the solvent that dissolves these components. The solvent is not particularly limited as long as it dissolves a protein. Examples of the solvent include protic polar solvents such as hexafluoro-2-propanol (HFIP) and formic acid, and aprotic polar solvents such as dimethyl sulfoxide (DMSO), N, N-dimethylformamide (DMF), and N, N-dimethylacetamide (DMAc).

The spider silk fibroin content of the dope solution may be appropriately set according to the type and the like of the spider silk fibroin, alcohol, and solvent. For example, the spider silk fibroin content of the dope solution may be 1 to 30 mass%, 2 to 25 mass%, 3 to 20 mass%, 4 to 15 mass%, or 5 to 10 mass% with respect to the total mass of the dope solution.

The alcohol content of the dope solution may be 0.1 mass% or more, 0.3 mass% or more, 0.5 mass% or more, 1.0 mass% or more, or 3.0 mass% or more and may be 15 mass% or less, 10 mass% or less, 8 mass% or less, or 6 mass% or less.

A viscosity of the dope solution may be set appropriately. The dope solution is set to have a viscosity of, for example, 15 to 1000 cP (centipoise) at 35°C at 1000 revolutions per minute (RPM). The viscosity of the dope solution is measured with, for example, an "EMS viscometer" (trade name) available from Kyoto Electronics Manufacturing Co., Ltd.

In the forming, first, the dope solution is applied to a substrate surface to a predetermined thickness (for example, a thickness after drying and/or desolvation is 1 to 1000 pm) .

The substrate may be a resin substrate, a silicone rubber mold, a glass substrate, or a metal substrate. The substrate is preferably a resin substrate from a perspective that a resin substrate facilitates peeling of the casted film. The resin substrate may be, for example, a polyethylene terephthalate (PET) film, a fluororesin film containing polytetrafluoroethylene or the like, a polypropylene (PP) film, or release films of these materials having a surface on which a silicone compound is immobilized. It is more preferable that the substrate is a PET film or a release film in which a silicone compound is immobilized on a surface of a PET film. This is because those films are stable with respect to a DMSO solvent, enable stable casting of the dope solution, and facilitate peeling after the casting.

In the forming, next, the dope solution applied to the substrate is dried and/or desolvated. The drying and/or desolvation is performed by at least one technique selected from, for example, vacuum drying, hot air drying, and air drying. It is preferable to desorb the solvent to the extent possible. The desolvation may be performed after stretching the film.

The dried and/or desolvated unstretched film is stretched uniaxially or biaxially. The uniaxial or biaxial stretching may be performed in water. The biaxial stretching may be performed sequentially or simultaneously. The film may be stretched through multiple stages, that is, two stages or more. The stretching ratio is preferably 1.01 to 6 times and more preferably 1.05 to 4 times both in length and width. Within this range, it is easy to gain a balance between stress and strain. The stretching in water is preferably performed at a water temperature of 20 to 90°C. The stretched film is preferably fixed by dry heating at 50 to 200°C for 5 to 600 seconds. This heat fixation provides dimensional stability to the film at room temperature. Note that a uniaxially stretched film is uniaxially oriented, and a biaxially stretched film is biaxially oriented.

The dried and/or desolvated unstretched film or the stretched film obtained by stretching the unstretched film may be subjected to methanol treatment by bringing the film into contact with methanol. The methanol treatment enables a harder protein film. The methanol treatment may not be performed from a perspective of further improving the elongation and toughness. Conditions of the methanol treatment are as described above. The methanol treatment makes it possible to control toughness of the protein film containing the spider silk fibroin and the alcohol. Therefore, as an embodiment of this invention, there is provided a method for controlling toughness of a protein film containing a spider silk fibroin and an alcohol, the method involving methanol treatment on the protein film.

### <Method for Producing Compositions Other Than Film>

In this embodiment, a method for producing a regenerating agent is not particularly limited and may be a method involving the following steps as well as a film. In addition to the following steps, the method may involve adding of a solvent or controlling of crystallinity depending on the physical properties of compositions to be required.

### (Solution Producing)

In solution producing, a protein is dissolved in a solvent such as DMSO or HFIP to obtain a protein solution.

In dissolving, a purified protein or a protein in a host cell having an expressed protein (recombinant protein) may be used as the protein to be dissolved. The purified protein may be a protein purified from a host cell having an expressed protein. In a case where a protein in a host cell is dissolved as the protein of interest, the host cell is brought into contact with a solvent, thereby dissolving the protein of the host cell in the solvent. Any host cell may be used as long as the cell has the expressed protein of interest and may be, for example, an intact cell or a cell subjected to disruption or other treatments. Alternatively, the host cell may be a cell subjected to simple purification in advance.

A method for purifying a protein from a host cell having an expressed protein is not particularly limited and may employ, for example, the methods disclosed in JP 6077570 B2 and JP 6077569 B2.

The dissolving may be performed at room temperature or may be performed to dissolve the protein in the solvent while holding the temperature at various heating temperatures. A time for holding a heating temperature is not particularly limited but may be 10 minutes or longer. In consideration of industrial production, the time is preferably 10 to 120 minutes, more preferably 10 to 60 minutes, and still more preferably 10 to 30 minutes. A time for holding a heating temperature may be appropriately set under conditions that the protein is sufficiently dissolved and impurities (substances other than the protein of interest) are less dissolved.

An amount of the solvent to be added to dissolve the protein is not particularly limited as long as the protein is dissolved by the amount.

In a case where a purified protein is dissolved, an amount of the solvent to be added or a ratio (volume (mL)/weight (g)) of volume (mL) of the solvent may be 1 to 100 times, 1 to 50 times, 1 to 25 times, 1 to 10 times, or 1 to 5 times the weight (g) of the protein (dry powder containing the protein).

In a case where a protein in a host cell having an expressed protein is dissolved, an amount of the solvent to be added or a ratio (volume (mL)/weight (g)) of a volume (mL) of the solvent may be 1 to 100 times, 1 to 50 times, 1 to 25 times, 1 to 10 times, or 1 to 5 times the weight (g) of the host cell.

An insoluble matter may be optionally removed from the protein solution. That is, the method for producing a protein body of this embodiment may optionally involve removing of an insoluble matter from the protein solution after the dissolving. Examples of a method for removing an insoluble matter from the protein solution include generally-used methods such as centrifugation and filter filtration with a drum filter, a press filter, or the like. In the filter filtration, using a filter aid such as celite or diatomaceous earth and a pre-coating agent in combination makes it possible to remove the insoluble matter more efficiently from the protein solution.

The protein solution contains the protein and the solvent (solvent for dissolution) that dissolves the protein. The protein solution may contain impurities included together with the protein in the dissolving. The protein solution may be a solution for forming a protein body.

The protein content of the protein solution may be 1 mass% or more and 35 mass% or less, or 5 mass% or more and 50 mass% or less of the total amount of the protein solution.

### (Forming)

The forming is to form a protein body using a powder or solution containing a protein and a biodegradable material. The protein body is not particularly limited in form and may be formed into a sheet, a fiber, a porous body, a resin body, and a powder in addition to a film.

### (Fiber Forming)

In the protein solution, it is preferable to adjust a concentration and viscosity of the protein depending on the use of the protein body to be formed.

A method for adjusting a concentration of the protein in the protein solution is not particularly limited. For examples, the solvent may be evaporated by distillation to increase a concentration of the protein. Alternatively, a solution having a high concentration of the protein may be used in the dissolving. Alternatively, the solvent may be reduced in amount relative to the protein.

A viscosity suitable for spinning is generally 10 to 50,000 cP (centipoise). Viscosities is measured with, for example, "EMS viscometer" (trade name) available from Kyoto Electronics Manufacturing Co., Ltd. When a viscosity of the protein solution is not within a range of 10 to 10,000 cP, the viscosity of the protein solution may be adjusted to the level that allows spinning. The viscosity is adjusted by the aforementioned method or the like. The solvent may contain an appropriate inorganic salt selected from the examples above.

In a case where the protein body to be formed is a protein fiber, the protein content (protein concentration) in the protein solution may be optionally adjusted so that the protein body has a concentration and viscosity that allows spinning. A method for adjusting a concentration and viscosity of the protein is not particularly limited. In addition, an example of the spinning includes wet spinning. When the protein solution having an adjusted concentration and viscosity suitable for spinning is added to a coagulation liquid as a dope solution, the protein coagulates. Here, the protein solution is added to the coagulation liquid as a filamentous liquid so that the protein coagulates in filaments, leading to formation of a yarn (undrawn yarn). The undrawn yarn is formed, for example, according to the method disclosed in JP 5584932 B2.

Hereinafter, the wet spinning will be described as an example, but the spinning is not limited thereto and may be dry wet spinning.

### Wet Spinning - Drawing

### (a) Wet Spinning

Any coagulation liquid may be used as long as the liquid is a solution that is desolvated. Preferable examples of the coagulation liquid include lower alcohols having 1 to 5 carbon atoms such as methanol, ethanol, and 2-propanol or acetone. The coagulation liquid may also contain water. The coagulation liquid preferably has a temperature of 5 to 30°C from a perspective of spinning stability.

A method for applying the protein solution as a filamentous liquid is not particularly limited. For example, the protein solution is extruded (discharged) from a spinneret to the coagulation liquid in a desolvation bath. The coagulation of the protein yields an undrawn yarn. A speed at which the protein solution is extruded to the coagulation liquid is appropriately set according to a diameter of the spinneret, a viscosity of the protein solution, or the like. For example, using a syringe pump provided with a nozzle having a diameter of 0.1 to 0.6 mm, the extrusion speed is preferably 0.2 to 6.0 mL/h per hole and more preferably 1.4 to 4.0 mL/h per hole from a perspective of spinning stability. The desolvation bath (coagulation liquid bath) that contains the coagulation liquid is not particularly limited in length but may have a length of, for example, 200 to 500 mm. A drawing speed of the undrawn yarn formed by the coagulation of the protein may be, for example, 1 to 14 m/min, and a residence time may be, for example, 0.01 to 0.15 min. The drawing speed of the undrawn yarn may be 1 to 3 m/min from a perspective of efficient desolvation. The undrawn yarn formed by the coagulation of the protein may be drawn (pre-drawn) in the coagulation liquid. However, from a perspective of suppressing vaporization of a lower alcohol used in the coagulation liquid, it is preferable that the coagulation liquid is kept at a low temperature and that the undrawn yarn is taken out of the coagulation liquid in the undrawn state.

### (b) Drawing

The spinning method may also involve drawing of the undrawn yarn obtained by the aforementioned step. The drawing may be one-stage drawing or multi-stage drawing including two or more stages. The multi-stage drawing enables molecules to be oriented in multiple stages and increases a total draw ratio, which is suitable for producing a fiber having high toughness.

In a case where the protein-containing body is a film (protein film), the protein solution may be optionally adjusted to have a concentration and viscosity that allows film formation of the protein solution. A method for film forming a protein is not particularly limited. For example, the protein solution is applied to a flat solvent-resistant plate to a predetermined thickness to form a coating film, and then, the solvent is removed from the coating film, thereby obtaining a film having a predetermined thickness.

In a case where the protein-containing body is a porous body (protein porous body), the protein solution may be optionally adjusted to have a concentration and viscosity that allows porosification of the protein solution. A method for forming a protein porous body is not particularly limited. For example, an appropriate amount of a foaming agent is added to the protein solution adjusted to have a concentration and viscosity suitable for porosification, followed by removing a solvent, thereby obtaining a porous body. Alternatively, the method disclosed in JP 5796147 B2 may be employed.

In a case where the protein-containing body is a resin body, a method for forming a protein resin body is not particularly limited. For example, dried protein powder prepared in the powder preparing is introduced into a pressure molding machine, and then, pressurized and heated with a hand press machine or the like, which enables the dried protein power to reach a required temperature. Accordingly, a resin body is obtained. Alternatively, a protein resin body is formed according to the methods disclosed in Patent Literatures (JP 2017-539869 A, PCT/JP2016/076500).

In a case where the protein-containing body is a gel form (protein gel form), a method for forming a protein gel form is not particularly limited. For example, a gel form is obtained by solution producing in which a dried protein is dissolved into a dissolving solvent to obtain a polypeptide solution, and by substituting of the solution produced in the solution producing with a water-soluble solvent. Here, between the solution producing and the substituting of the dissolving solvent with the water-soluble solvent, the solution may be poured into a mold to form a predetermined shape. Alternatively, the solution may be cut into a predetermined shape after the substituting of the dissolving solvent with the water-soluble solvent. Alternatively, a protein gel form is formed according to the method disclosed in JP 05782580 B2.

### Examples

Hereinafter, the present invention will be described more specifically based on Examples. However, the invention is not limited to the following Examples.

### (PRT799)

### [Test Example 1: Production of Modified Spider Silk Fibroin]

### <(1-1) Production of Spider Silk Protein (Modified Spider Silk Fibroin: PRT799)>

### (Synthesis of Gene That Encodes Spider Silk Protein and Construction of Expression Vector)

Based on the nucleotide sequence and the amino acid sequence of the *Nephila clavipes-derived* fibroin (GenBank Accession No.: P 46804.1, GI: 1174415), designed was a modified fibroin having the amino acid sequence of SEQ ID NO: 15 (hereinafter referred to as "PRT799").

The amino acid sequence of SEQ ID NO: 15 is the same as the amino acid sequence of the *Nephila clavipes-derived* fibroin except that amino acid residues are substituted, inserted, and deleted to improve productivity and that the amino acid sequence of SEQ ID NO: 12 (tag sequence and hinge sequence) is added to the N-terminus.

Next, a nucleic acid that encodes PRT799 was synthesized. In the nucleic acid, an NdeI site was added to the 5'-end and an EcoRI site was added downstream of a stop codon. The nucleic acid was cloned into a cloning vector (pUC118). Thereafter, the nucleic acid was enzymatically cleaved by treatment with NdeI and EcoRI, and then, recombined into the protein expression vector pET-22b(+), thereby obtaining an expression vector.

The expression vector pET-22b(+) containing the nucleic acid that encodes PRT799 was used to transform *Escherichia coli* BLR (DE3). The transformed *Escherichia coli* was cultured for 15 hours in 2 mL of an LB culture medium containing ampicillin. The culture solution was added to 100 mL of a seed culture medium containing ampicillin (Table 4) so that OD600 reached 0.005. The culture solution was maintained at a temperature of 30°C and subjected to flask culture (for about 15 hours) until OD600 reached 5, thereby obtaining a seed culture solution.

**[Table 4]**

| Seed Culture Medium | |
|---|---|
| Reagent | Concentration (g/L) |
| Glucose | 5.0 |
| KH₂PO₄ | 4.0 |
| K₂HPO₄ | 9.3 |
| Yeast Extract | 6.0 |
| Ampicillin | 0.1 |

A 500 mL growing medium (Table 5) was added to a jar fermenter, and the seed culture solution was added to the jar fermenter so that OD600 reached 0.05. The culture solution was maintained at a temperature of 37°C, and the culture solution was cultured, being constantly controlled to have pH 6.9. Furthermore, the culture solution was maintained to have a 20% dissolved oxygen concentration of the saturated dissolved oxygen concentration.

**[Table 5]**

| Growing Medium | |
|---|---|
| Reagent | Concentration (g/L) |
| Glucose | 12.0 |
| KH₂PO₄ | 9.0 |
| MgSO₄·7H₂O | 2.4 |
| Yeast Extract | 15 |
| FeSO₄·7H₂O | 0.04 |
| MnSO₄·5H₂O | 0.04 |
| CaCl₂·2H₂O | 0.04 |
| ADEKA NOL (ADEKA, LG-295S) | 0.1 (mL/L) |

Immediately after glucose in the growing medium was completely consumed, a feed solution (455 g/1 L of glucose, 120 g/1 L of Yeast Extract) was added at a rate of 1 mL/min. The culture solution was maintained at a temperature of 37°C, and the culture solution was cultured, being constantly controlled to have pH 6.9. Furthermore, the culturing was performed for 20 hours while the dissolved oxygen concentration in the culture solution was maintained at 20% of the saturated dissolved oxygen concentration. Thereafter, 1 M isopropyl-β-thiogalactopyranoside (IPTG) was added to the culture solution to obtain a final concentration of 1 mM, thereby inducing the expression of PRT799. Twenty hours after the addition of IPTG, the culture solution was centrifuged to collect bacterial cells. SDS-PAGE was conducted using bacterial cells prepared from the culture solution before the addition of IPTG and from the culture solution after the addition of IPTG. The expression of PRT799 was determined by the appearance of a band having a size of PRT799 dependent on the addition of IPTG.

### (Purification of PRT799)

The bacterial cells collected two hours after the addition of IPTG were washed with 20 mM Tris-HCl buffer (pH 7.4). The washed bacterial cells were suspended in 20 mM Tris-HCl buffer (pH 7.4) containing about 1 mM PMSF, and the cells were disrupted with a high-pressure homogenizer (manufactured by GEA Niro Soavi). The disrupted cells were centrifuged to obtain a precipitate. The obtained precipitate was washed with 20 mM Tris-HCl buffer (pH 7.4) until the precipitate reached a high level of purity. The washed precipitate was suspended in 8 M guanidine buffer (8 M guanidine hydrochloride, 10 mM sodium dihydrogen phosphate, 20 mM NaCl, 1 mM Tris-HCl, pH 7.0) so that the precipitate reached a concentration of 100 mg/mL, and then, the precipitate was dissolved by being stirred with a stirrer at 60°C for 30 minutes. After the dissolving, the resultant was dialyzed with water using a dialysis tube (cellulose tube 36/32, available from Sanko Junyaku Co., Ltd.). A white aggregate protein (PRT799) obtained after the dialysis was collected by centrifugation, followed by removing moisture with a lyophilizer so as to collect lyophilized powder.

A purification level of PRT799 in the obtained lyophilized powder was determined by analyzing an image of polyacrylamide gel electrophoresis result of the powder using Totallab (Nonlinear Dynamics Ltd.). The analysis result showed that the purification level of PRT799 was about 85%.

### [Test Example 2: Production of Protein Film]

### <(2-1) Production of Protein Film>

The spider silk fibroin (PRT799) obtained in Test Example 1 (1-1) and glycerin were added to dimethyl sulfoxide so as to prepare a dope solution. (Mass percentages of the spider silk fibroin, dimethyl sulfoxide, and glycerin were 5.0%, 92.9%, and 2.1%, respectively). Using a stainless steel bottle, the dope solution was stirred and dissolved at 80°C for 30 minutes at 200 rpm, and then, cooled to remove dust and bubbles.

A protein film was produced from the dope solution by the following steps. That is, the dope solution prepared above was cast into a silicone square mold (size (width × depth × height): 2 inches × 2 inches × 2 inches), thereby preparing a wet film. Then, dimethyl sulfoxide was removed by evaporating the wet film at 60°C under negative pressure. After the drying, a protein film was peeled off the silicone mold. The obtained protein film had a thickness of about 40 to 50 µm. The thickness of the protein film was measured with a digital outside micrometer available from Niigata Seiki Co., Ltd.

The larger the fracture-point displacement, the better the elongation (the higher the elongation). The larger the ultimate stress (MPa), the better the stress (the larger the stress). The larger the strain energy, the better the toughness.

### [Example 1: In Vivo Test for Muscle Tissue Regeneration]

A triple anesthetic (medetomidine hydrochloride 0.375 mg/kg, midazolam 2 mg/kg, butorphanol tartrate 2.5 mg/kg) was applied to 10-week-old SD rats by intraperitoneal administration, and longitudinal skin incision was performed on the midline of abdomen of each rat. A subcutaneous tissue on the left side was subjected to blunt dissection so as to expose the left abdominal wall, followed by excision of the entire abdominal wall layer having a size of 2 × 3 cm so as to remove the abdominal wall completely. A 4 × 4 cm spider silk protein film PRT799 was fixed to the defect by 8 stiches with a 7-0 nylon. The skin was sutured with 4-0 vicryl, thereby completing the operation.

The rats were euthanized at postoperative weeks 6, 12, and 15, and then, subjected to pressure testing and histopathological evaluations. Pressure tests were performed by puncturing the abdominal cavities of the rats with a 25G butterfly needle attached with a 10 ml syringe and by injecting the abdominal cavities with air. The histopathological evaluations were performed by extracting a tissue site where the abdominal wall defect was reinforced and by performing hematoxylin/eosin staining after formalin fixation.

### (Histopathological Evaluation)

### Postoperative Week 6

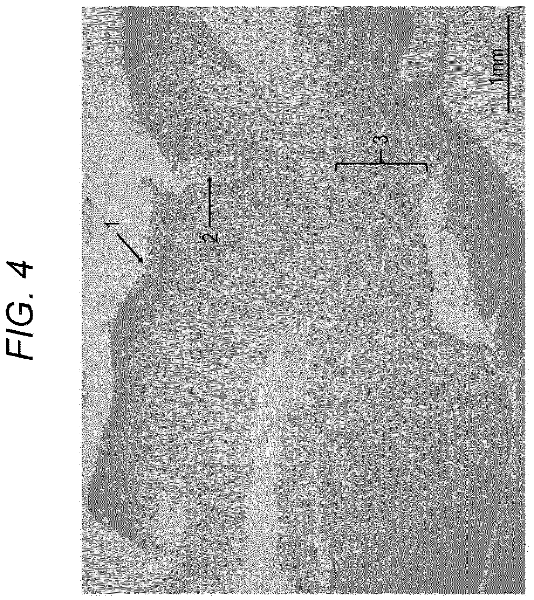

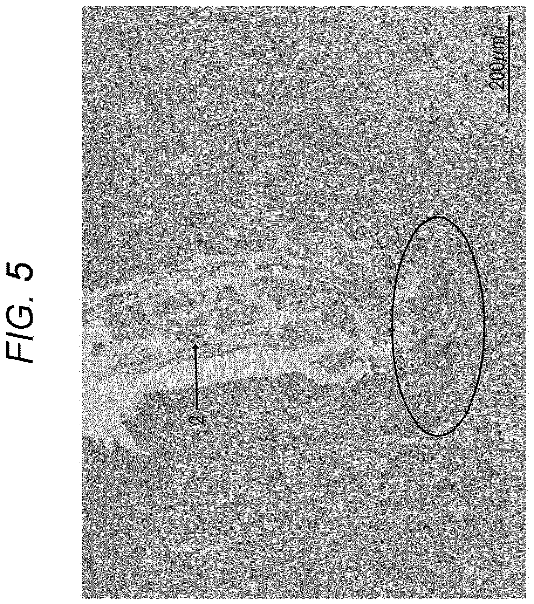

As shown in Figs. 4 and 5, a residual film 2 and cell swelling with high-grade inflammation accompanied by a foreign-body response were observed. Formation of a thin skeletal muscle fiber bundle was observed at the boundary with a known skeletal muscle.

### Postoperative Week 12

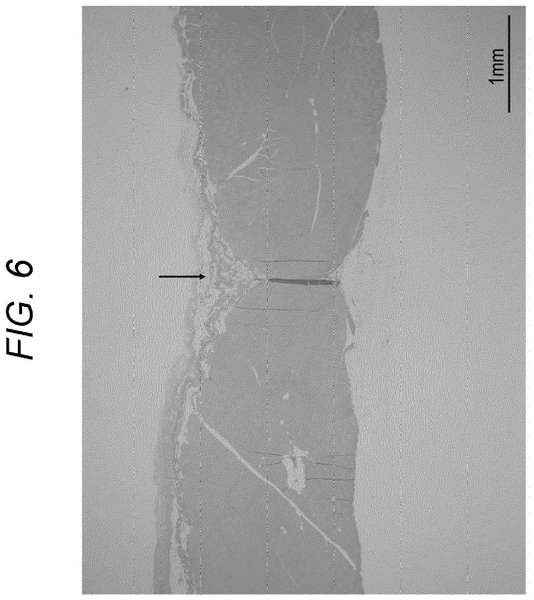

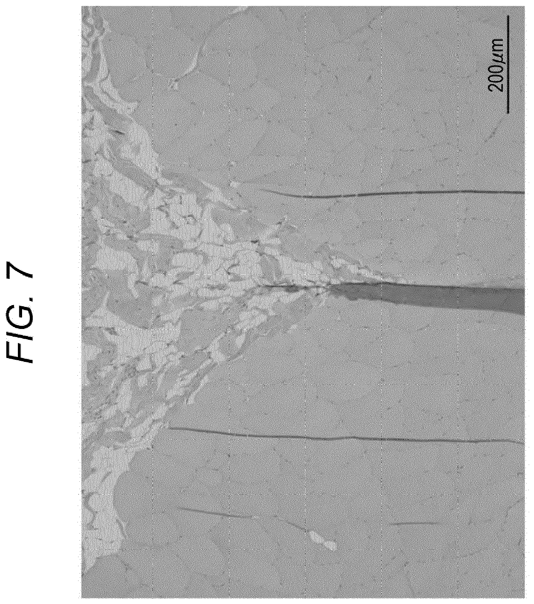

As shown in Figs. 6 and 7, the defect in the skeletal muscle was ill-defined, and regeneration of a muscle tissue was observed. No residual film 2 and no inflammatory sign were observed. The material disappeared within 12 weeks postoperatively, which demonstrated that the material had excellent biodegradability.

### Postoperative Week 15

Similarly to the results at the postoperative week 12, no residual film and no significant inflammation were observed. A skeletal muscle was observed, leading to such an inference that the tissue was repaired.

### (Pressure Test Results)

### Postoperative Week 6

The abdomen was expanded, but the reinforced site of the abdominal wall defect did not distend. Pathologically, the thin skeletal muscle fiber bundle was observed, but the residual film was also observed. Accordingly, it is inferred that the film had strength strong enough to withstand abdominal pressure at this period before regeneration of a muscle tissue.

### Postoperative Week 12

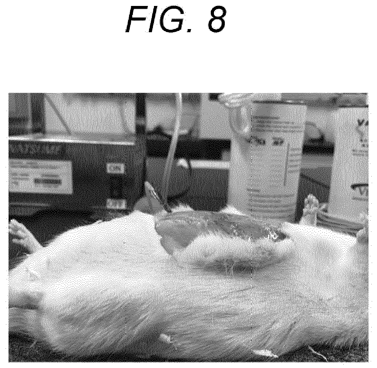

The abdomen was expanded, but the reinforced site of the abdominal wall defect did not distend (Fig. 8). Pathologically, the film had already disappeared, leading to such an inference that a muscle tissue capable of sufficiently withstanding abdominal pressure was regenerated.

### Postoperative Week 15

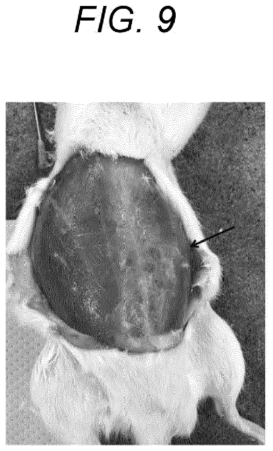

Similarly to the results at the postoperative week 12, there was no distension, leading to such an inference that a muscle tissue was regenerated (Fig. 9).

Muscle regeneration after complete loss of a muscle tissue was observed. Accordingly, it is inferred that the following steps were all executed to achieve muscle regeneration: (1) activation of satellite cells; (2) differentiation of myoblasts into myocytes; (3) formation of myotubes by fusion of the myocytes; and (4) formation of muscle fibers from the myotubes. Furthermore, it is inferred that cell adhesion occurred between the myoblasts and myocytes and the regenerating agent.

This invention shows that the fibroin protein has a plurality of properties required for practical muscle fiber regeneration such as muscle tissue regeneration ability, physical strength, bio-absorbability, and hypoallergenicity. Without such properties, prosthetic and regenerative effects for a muscle tissue defect cannot be obtained.

Enabling both prosthesis and regeneration of a muscle tissue defect, the fibroin protein is used for treatment of, for example, abdominal incisional hernia, diaphragmatic hernia, inguinal hernia, site of amputation stump plasty, decubitus, and wound.

Enabling muscle fiber regeneration, the fibroin protein shows promise for regeneration of tracheae including lungs and blood vessels.

### Reference Signs List

- 1: High-grade inflammation
- 2: Film
- 3: Thin skeletal muscle fiber bundle

## Claims

1. A muscle tissue-regenerating agent comprising a modified fibroin protein.

2. The muscle tissue-regenerating agent according to claim 1, wherein the muscle tissue-regenerating agent is a prosthetic and regenerating agent for a muscle tissue defect.

3. The muscle tissue-regenerating agent according to claim 1 or 2, wherein the muscle tissue-regenerating agent is an activator for a satellite cell in a muscle tissue.

4. The muscle tissue-regenerating agent according to any one of claims 1 to 3, wherein the muscle tissue-regenerating agent is an agent for differentiating a myoblast into a myocyte.

5. The muscle tissue-regenerating agent according to any one of claims 1 to 4, wherein the muscle tissue-regenerating agent is a myoblast adhesive.

6. The muscle tissue-regenerating agent according to any one of claims 1 to 5, wherein the modified fibroin protein is a recombinant fibroin protein, and the muscle tissue-regenerating agent further comprises a substance derived from a host used in production of the recombinant fibroin protein.

7. The muscle tissue-regenerating agent according to claim 6, wherein the host is a prokaryote.

8. The muscle tissue-regenerating agent according to any one of claims 1 to 7, wherein the muscle tissue-regenerating agent is biodegraded within 80 days in a mammalian living organism.

9. The muscle tissue-regenerating agent according to any one of claims 1 to 8, wherein the muscle tissue-regenerating agent further comprises one or both of an alcohol and dimethyl sulfoxide in amount of 0.01 to 50 mass%.

10. The muscle tissue-regenerating agent of any one of claims 1 to 9, wherein the modified fibroin protein is a modified spider silk fibroin protein.

11. The muscle tissue-regenerating agent according to any one of claims 1 to 10, wherein the modified fibroin protein has an average hydropathy index (average HI) of 0 or less.

12. The muscle tissue-regenerating agent according to any one of claims 1 to 11, wherein the muscle tissue-regenerating agent is a therapeutic agent for one selected from abdominal incisional hernia, diaphragmatic hernia, inguinal hernia, site of amputation stump plasty, decubitus, and wound.

13. The muscle tissue-regenerating agent according to any one of claims 1 to 12, wherein the muscle tissue-regenerating agent is formed into any one of a film, sheet, fiber, resin body, powder, and gel or formed by any combination of these forms.

14. The muscle tissue-regenerating agent of any one of claims 1 to 12, wherein the muscle tissue-regenerating agent is formed into a film or a sheet or formed by a combination of these forms.

15. A treatment of a condition in need of muscle tissue regeneration, the treatment comprising bringing the muscle tissue-regenerating agent according to any one of claims 1 to 14 into contact with a site having a deficient myocyte in a non-human animal.
